(19) Europäisches Patentamt
European Patent Office
Office européen des brevets

(11) Veröffentlichungsnummer: **0 329 012 A2**

(12) **EUROPÄISCHE PATENTANMELDUNG**

(21) Anmeldenummer: **89102209.7**

(22) Anmeldetag: **09.02.89**

(51) Int. Cl.[4]: **C07D 471/04 , A01N 43/90 , //(C07D471/04,221:00,221:00), (C07D471/04,239:00,221:00), (C07D471/04,241:00,221:00)**

(30) Priorität: **18.02.88 DE 3804990**

(43) Veröffentlichungstag der Anmeldung: **23.08.89 Patentblatt 89/34**

(84) Benannte Vertragsstaaten: **CH DE FR GB IT LI**

(71) Anmelder: **BASF Aktiengesellschaft**
**Carl-Bosch-Strasse 38**
**D-6700 Ludwigshafen(DE)**

(72) Erfinder: **Saupe, Thomas, Dr.**
**Kiesgrabenweg 23**
**D-6919 Bammental(DE)**
Erfinder: **Klebe, Gerhard, Dr.**
**Roemerstrasse 23**
**D-6900 Heidelberg(DE)**
Erfinder: **Schirmer, Ulrich, Dr.**
**Berghalde 79**
**D-6900 Heidelberg(DE)**
Erfinder: **Paul, Gerhard, Dr.**
**Berner Weg 34**
**D-6700 Ludwigshafen(DE)**
Erfinder: **Kober, Reiner, Dr.**
**Im Schlittweg 20**
**D-6701 Fussgoenheim(DE)**
Erfinder: **Wuerzer, Bruno, Dr.**
**Ruedigerstrasse 13**
**D-6701 Otterstadt(DE)**
Erfinder: **Berghaus, Rainer, Dr.**
**Dahlienstrasse 24**
**D-6704 Mutterstadt(DE)**
Erfinder: **Meyer, Norbert, Dr.**
**Dossenheimer Weg 22**
**D-6802 Ladenburg(DE)**
Erfinder: **Westphalen, Karl-Otto, Dr.**
**Mausbergweg 58**
**D-6720 Speyer(DE)**

(54) **Sulfonamide.**

(57) Sulfonamide der allgemeinen Formel I

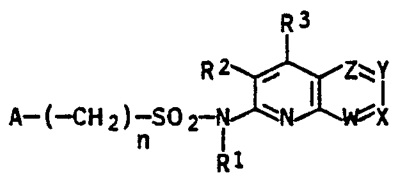

I,

in der A, n, $R^1$, $R^2$, $R^3$, W, X, Y und Z die in Beschreibung und Ansprüchen angegebene Bedeutung haben, deren Salze und N-Oxide sowie deren Verwendung als herbizide Mittel.

EP 0 329 012 A2

## Sulfonamide

Die vorliegende Erfindung betrifft Sulfonamide der allgemeinen Formel I

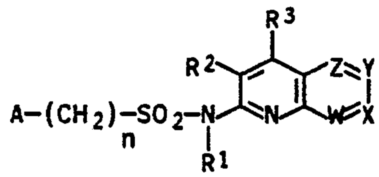

I

in der die Substituenten und Indices folgendes Bedeutung haben:

$R^1$ Wasserstoff, Cyano,
eine $C_1$-$C_8$-Alkylgruppe, welche durch einen der folgenden Reste substituiert sein kann: $C_1$-$C_2$-Alkoxy, $C_1$-$C_2$-Alkylthio, Aryl, Aryloxy, Arylthio, Heteroaryl, Heteroaryloxy oder Heteroarylthio,
eine $C_2$-$C_5$-Alkenylgruppe,
eine $C_2$-$C_4$-Alkinylgruppe,
ein Rest $COR^4$, worin $R^4$ $C_1$-$C_4$-Alkyl, $C_1$-$C_4$-Alkoxy, $C_1$-$C_4$-Alkylthio, Aryl, Aryloxy, Arylthio, Aryl-$C_1$-$C_4$-alkoxy, Heteroaryl, Heteroaryloxy, Heteroarylthio oder Heteroaryl-$C_1$-$C_4$-alkoxy bedeutet,
ein Rest $CONR^5R^6$, worin $R^5$ und $R^6$ unabhängig voneiander folgende Bedeutung haben: Wasserstoff, $C_1$-$C_4$-Alkyl, $C_3$-$C_6$-Cycloalkyl, $C_2$-$C_5$-Alkenyl, Aryl, Heteroaryl, Aryl-$C_1$-$C_4$-alkyl, Heteroaryl-$C_1$-$C_4$-alkyl, $C_1$-$C_4$-Alkylcarbonyl oder gemeinsam eine $C_2$-$C_6$-Alkylenkette,
oder ein Rest $SO_mR^4$, worin m den Wert 1 oder 2 hat und $R^4$ die vorstehend gegebene Bedeutung besitzt;

$R^2$, $R^3$ unabhängig voneinander Nitro, Hydroxy, Carboxy, Mercapto, Halogen,
$C_1$-$C_4$-Alkyl, welches einfach durch Hydroxy, Mercapto, Amino, Aryloxy oder Heteroaryloxy und/oder ein- bis dreifach durch Halogen substituiert sein kann,
$C_3$-$C_6$-Cycloalkyl, welches ein- bis dreifach durch Halogen, Hydroxy, Mercapto und/oder $C_1$-$C_4$-Alkyl substituiert sein kann,
$C_3$-$C_6$-Cycloalkoxy,
$C_3$-$C_6$-Cycloalkylthio,
$C_2$-$C_5$-Halogenalkenyl,
$C_2$-$C_4$-Halogenalkinyl,
$C_1$-$C_4$-Alkoxy, welches ein- bis dreifach durch Halogen und/oder einfach durch Aryl oder Heteroaryl substituiert sein kann,
$C_1$-$C_4$-Alkylthio, welches ein- bis dreifach durch Halogen und/oder einfach durch Aryl oder Heteroaryl substituiert sein kann,
$C_2$-$C_5$-Alkenyloxy,
$C_2$-$C_4$-Alkinyloxy,
ein Rest $-NR^5R^6$ worin $R^5$ und $R^6$ die vorstehend gegebene Bedeutung besitzen oder
eine der unter $R^1$ genannten Gruppen,

W, X, Y, Z unabhängig voneinander Stickstoff oder
eine Gruppe

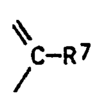

worin $R^7$ Hydrazino oder eine der unter $R^2$ genannten Gruppen bedeutet,
wobei X, Y, Z und W nicht gleichzeitig Stickstoff bedeuten,

n den Wert 0 oder 1 und

A eine Aryl- oder Heteroarylgruppe, wobei diese Gruppen ein bis fünf Halogenatome und/oder ein bis drei der folgenden Substituenten tragen können:
$-SO_2R^8$, worin $R^8$ Hydroxy, $C_1$-$C_4$-Alkoxy, Aryl-$C_1$-$C_4$-alkoxy, Aryloxy, Heteroaryloxy, Heteroaryl-$C_1$-$C_4$-alkoxy oder $-NR^5R^6$ bedeutet, wobei $R^5$ und $R^6$ die vorstehend gegebene Bedeutung besitzen und/oder die unter $R^2$ genannten Gruppen

sowie deren Salze und N-Oxide, ausgenommen die Verbindungen der Formel I, in der A die Bedeutung 4-Aminophenyl hat, n = 0 ist, $R^1$, $R^2$ und $R^3$ die Bedeutung Wasserstoff, W die Bedeutung Stickstoff, X, Y und Z die Bedeutung Kohlenstoff hat und $R^4$ an X und Z Methyl und im übrigen Wasserstoff ist, sowie deren 4-Aminophenyl-Umsetzungsprodukte.

Weiterhin betrifft die Erfindung ein Verfahren zur Herstellung dieser Verbindungen sowie ihre Verwendung zur Bekämpfung unerwünschten Pflanzenwuchses und ihre Verwendung zur Beeinflussung des Pflanzenwachstums.

In der EP-A-7 687 und später erschienenen Druckschriften werden Sulfonylharnstoffe beschrieben, die eine herbizide Wirkung haben können. Sie befriedigen jedoch nicht alle Anforderungen, z.B. nach Selektivität und spezifischer Wirkung.

Der Erfindung lag daher die Aufgabe zugrunde, Substanzen mit befriedigenden Eigenschaften zu finden und zu synthetisieren.

Entsprechend dieser Aufgabe wurden die eingangs definierten Sulfonamide I gefunden, die eine vielseitige Wirkung als Herbizide und Wachstumsregulatoren haben. Die Erfindung umfaßt auch einfache Umsetzungsprodukte dieser Sulfonamide wie Salze oder N-Oxide.

Zur Erfindung gehören auch Verfahren zur Herstellung dieser Verbindungen (I), Herbizide und Mittel zur Beeinflussung des Pflanzenwachstums, die die neuen Verbindungen als Wirkstoffe enthalten, sowie ein Verfahren zur Beeinflussung und Bekämpfung von Pflanzenwuchs mit diesen Verbindungen.

Die Synthese von Verbindungen der allgemeinen Formel I kann in konvergenter Weise, entsprechend dem Schema 1 durchgeführt werden, indem man ein heterocyclisches Amin der Formel II, in der W, X, Y, Z, $R^1$, $R^2$, $R^3$ die oben genannte Bedeutung haben, mit ungefähr stöchimetrischen Mengen eines Sulfochlorids der Formel III, in der A und n die oben genannte Bedeutung haben, unter basischen Bedingungen gegebenenfalls in einem organischen Lösungsmittel bei einer für organische Reaktionen üblichen Temperatur umsetzt.

Schema 1

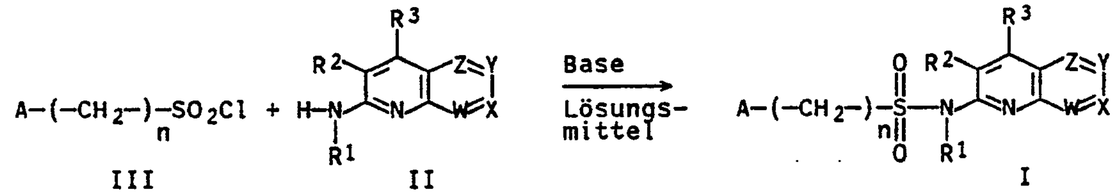


Zweckmäßigerweise verwendet man für die dem Schema 1 entsprechenden Umsetzungen übliche Lösungs- oder Verdünnungsmittel. Als solche Lösungsmittel können Halogenkohlenwasserstoffe, insbesondere Chlorkohlenwasserstoffe dienen; z.B. sind Tetrachlorethan, Methylenchlorid, Chloroform, Dichlorethan, Chlorbenzol geeignet. Ether, z.B. Diethylether, Tetrahydrofuran, Dioxan; dipolare aprotische Lösungsmittel, insbesondere Acetonitril, Dimethylformamid, Dimethylacetamid, Dimethylsulfoxid, N-Methylpyrrolidon; Aromaten, z.B. Benzol, Toluol, Pyridin, Chinolin; Ketone, z.B. Aceton, Methylethylketon; Alkohole, z.B. Methanol, Ethanol, iso-Propanol, t-Butanol, und entsprechende Gemische können ebenfalls verwendet werden.

Die dem Schema 1 entsprechenden Umsetzungen können bei Temperaturen von Raumtemperatur bis zur Rückflußtemperatur des jeweiligen Lösungsmittels bzw. -gemisches durchgeführt werden.

Als katalytisch wirksame Basen können für die dem Schema 1 entsprechenden Umsetzungen aromatische Stickstoffbasen, wie z.B. Pyridin, 4-Dimethylaminopyridin oder Chinolin, tertiäre aliphatische Amine, wie z.B. Triethylamin oder N-Methylmorpholin, bi- und tricyclische Amine, wie z.B. Diazabicycloundecen (DBU) oder Diazabicyclooctan (DABCO) sowie die Hydroxide, Hydride, Alkoxide, Carbonate und Hydrogencarbonate der Alkali- und Erdalkalimetalle, insbesondere NaOH, KOH, NaH, KH, $CaH_2$, LiH, NaOMe, NaOEt, KOtBu, $Na_2CO_3$, $K_2CO_3$, $NaHCO_3$, $KHCO_3$ verwendet werden. Mitunter ist es auch nützlich, Kombinationen der oben angeführten Basen zu verwenden.

Die molaren Verhältnisse, in denen die benötigten Ausgangsverbindungen im Sinne des Reaktionsschemas 1 miteinander umgesetzt werden, betragen im allgemeinen 1:1 bis 1:3 für das Verhältnis von heterocyclischen Amin II zu Sulfonylchlorid III und 1:1 bis 1:5 für das Verhältnis von heterocyclischen Amin II zu katalytisch wirksamer Base. Die Sulfonylchloride III sowie die reaktionsbeschleunigend wirkenden Basen können aber auch jeweils in weniger als stöchiometrischer Menge eingesetzt werden.

Die Reaktionskonzentration für die dem Schema 1 entsprechenden Umsetzungen liegt im allgemeinen im Bereich von 0,1 bis 5 molar, bevorzugt im Bereich von 0,2 bis 2 molar.

Im bestimmten Fällen kann die Verwendung von Pyridin als Lösungsmittel für die dem Schema 1

entsprechenden Umsetzungen besonders nützlich und bequem sein, da Pyridin sowohl als Lösungsmittel als auch als reaktionsbeschleunigend wirkende Base fungieren kann.

Die Sulfochloride der allgemeinen Formel III, die für die dem Schema 1 entsprechenden Umsetzungen benötigt werden, sind in vielen Fällen handelsüblich. Neue Sulfochloride der Formel III lassen sich im allgemeinen nach bekannten Methoden herstellen. Zu solchen allgemein bekannten Methoden zur Herstellung von Sulfochloriden gehören die Sulfochlorierung von geeignet substituierten Aromaten, wie dies z.B. von H.T. Clarke et al. in Org. Synth. Coll. Vol. I. 2. Aufl., 1941, auf S. 85 ff beschrieben wird, die Diazotierung von geeignet substituierten Anilinen oder heterocyclischen Aminen und nachfolgender Reaktion des resultierenden Diazoniumsalzes mit $SO_2$ in Gegenwart von Kupfer(II)chlorid, wie dies z.B. von R.V. Hoffmann in Org. Synth., Vol. 60 auf Seite 121 ff beschrieben wird, oder auch die Behandlung von aromatischen Alkylthio- bzw. Benzylthiogruppen mit $Cl_2$ in wäßrig-saurem Medium.

Die heterocyclischen Amine der allgemeinen Formel II, die für die dem Schema 1 entsprechenden Umsetzungen benötigt werden, können nach bekannten Methoden hergestellt werden, wie sie z.B. von P.-A. Lowe in Comprehensive Heterocyclic Chem. (The Structure, Reactions, Synthesis and Uses of Heterocyclic Compounds), Vol. 2, 1st ed. (1984), Kap. 2.11, von E. Lunt und C.G. Newton in Comprehensive Heterocyclic Chem., Vol. 3, 1st ed. (1984), Kap. 2.15 und von G.W.H. Cheeseman und R.F. Cookson in The Chemistry of Heterocyclic Compounds, Vol. 35, 1979, Kap. XXVIII angegeben sind.

Heterocyclische Amine der allgemeinen Formel II, die nach bekannten Methoden der oben angeführten und dort zitierten Quellen hergestellt werden können und deren heterocyclisches Gerüst z.B. durch eine oder mehrere OH-Gruppen substituiert ist (nachstehend als IIa bezeichnet), können weitere, dem Schema 2 entsprechende Umsetzungen eingehen und so in neue heterocyclische Amine der Formel IIb überführt werden. Z.B. lassen sich solche heteroaromatischen OH-Gruppen mit $POCl_3$ gegen Cl austauschen; diese Reaktion wird im allgemeinen in reinem $POCl_3$ bei Rückflußtemperatur durchgeführt. Das so in Verbindungen der Formel IIb eingeführte heteroaromatische Cl läßt sich seinerseits gegen Nukleophile (Nu) wie Alkoholate, Thiolate, Amine, Amide, Grignard-Verbindungen etc. austauschen (IIc).

Schema 2

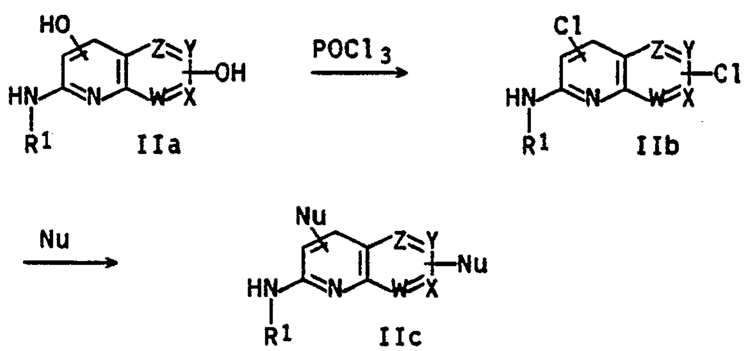


Sulfonamide der vorliegenden Erfindung, die der allgemeinen Formel I gemäß Anspruch 1 unter Ausnahme der Bedeutung von $R^1$ = H entsprechen, können z.B. ausgehend von Sulfonamiden der allgemeinen Formel I gemäß Anspruch 1 mit der Bedeutung von $R^1$ = H hergestellt werden, indem man entsprechend dem Schema 3 eine Verbindung der Formel I, in der $R^1$ Wasserstoff bedeutet, mit ungefähr stöchiometrischen Mengen eines Elektrophils der Formel IV, in der $R^1$ nicht Wasserstoff ist, und B eine geeignete Abgangsgruppe wie z.B. Halogen, insbesondere Cl, bedeutet, unter basischen Bedingungen gegebenenfalls in einem Lösungsmittel bei einer für organische Reaktionen üblichen Temperatur umsetzt.

Schema 3

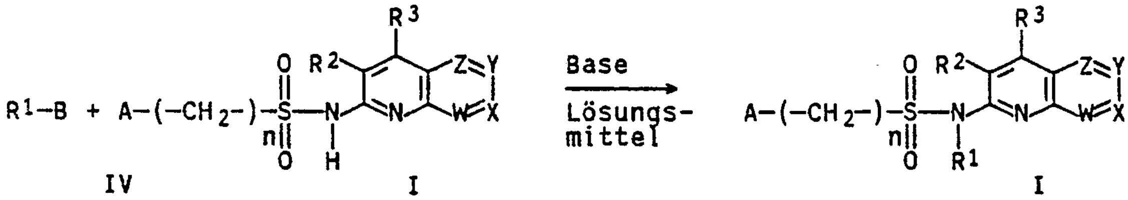


IV I I

Zweckmäßigerweise verwendet man für die dem Schema 3 entsprechenden Umsetzungen unter den jeweiligen Reaktionsbedingungen inerte Lösungs- oder Verdünnungsmittel. Als solche können prinzipell die gleichen Lösungsmittel und entsprechende Gemische verwendet werden, wie sie bereits bei den dem Schema 1 entsprechenden Umsetzungen aufgeführt worden sind.

Die dem Schema 3 entsprechenden Umsetzungen können bei Temperaturen von Raumtemperatur bis zur Rückflußtemperatur des jeweiligen Lösungsmittels bzw. -gemisches durchgeführt werden.

Als reaktionsbeschleunigend wirkende Basen können für die dem Schema 3 entsprechenden Umsetzungen prinzipiell die gleichen Basen verwendet werden, wie sie bereits bei den dem Schema 1 entsprechenden Umsetzungen aufgeführt worden sind. Mitunter kann es auch nützlich sein, bestimmte Kombinationen dieser bereits aufgeführten Basen zu verwenden.

In bestimmten Fällen kann die Verwendung von Pyridin als Lösungsmittel für die dem Schema 3 entsprechenden Umsetzungen besonders nützlich und bequem sein, da Pyridin sowohl als Lösungsmittel als auch als reaktionsbeschleunigend wirkende Base fungieren kann.

Die molaren Verhältnisse, in denen die benötigten Ausgangsverbindungen im Sinne des Reaktionsschemas 3 miteinander umgesetzt werden, betragen im allgemeinen 1:1 bis 1:3 für das Verhältnis von Verbindung I mit der Bedeutung $R^1 = H$ zu Elektrophil IV mit der Bedeutung $R^1 \neq H$ und 1:1 bis 1:5 für das Verhältnis von Verbindung I mit der Bedeutung $R^1 = H$ zu katalytisch wirksamer Base.

Die Reaktionskonzentration für die dem Schema 3 entsprechenden Umsetzungen liegt im allgemeinen im Bereich von 0,1 bis 5 molar, bevorzugt im Bereich von 0, 2 bis 2 molar.

Als geeignete elektrophile Reagenzien der allgemeinen Formel IV, die für die dem Schema 3 entsprechenden Umsetzungen benötigt werden, kommen Alkylhalogenide, Dialkylsulfate, Arylalkylhalogenide wie z.B. Benzyl chlorid, Acylchloride, aliphatische, aromatische und araliphatische Chlorameisensäureester, Chlorameisensäureamide (Carbamoylchloride), Alkylsulfonylchloride, Arylsulfonylchloride, Aralkylsulfonylchloride sowie aliphatische, aromatische und araliphatische Chlorthioameisensäureester in Betracht. Solche elektrophilen Reagenzien sind im allgemeinen kommerziell erhältlich bzw. können in einfacher und allgemein bekannter Art und Weise aus leicht erhältlichen Vorprodukten leicht hergestellt werden.

Die für die dem Schema 3 entsprechenden Umsetzungen benötigten Sulfonamide der allgemeinen Formel I gemäß Anspruch 1 mit der Bedeutung von $R^1 = H$ sind nach dem bereits geschilderten allgemeinen Verfahren erhältlich, das den dem Schema 1 gemäßen Umsetzungen entspricht.

N-Oxide (V) der Sulfonamide der allgemeinen Formel I können entsprechend dem Schema 4 ausgehend von den entsprechenden nicht-oxidierten Sulfonamiden hergestellt werden, indem man diese mit einem üblichen Oxidationsmittel umsetzt. Wenn W, X, Y oder Z Stickstoff ist, können natürlich auch diese N-Atome N-Oxide bilden.

Schema 4

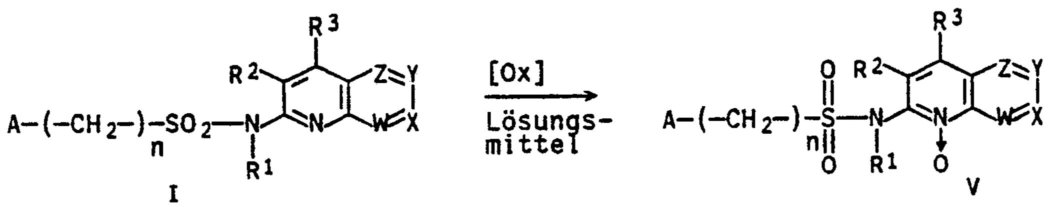


I V

Zweckmäßigerweise verwendet man auch für die dem Schema 4 entsprechenden Umsetzungen unter den jeweiligen Reaktionsbedingungen inerte Lösungs- oder Verdünnungsmittel. Als solche können Wasser, Essigsäure und prinzipiell die gleichen organischen Lösungsmittel sowie entsprechende Gemische verwendet werden, wie sie bereits bei den dem Schema 1 entsprechenden Umsetzungen aufgeführt worden sind.

Die dem Schema 4 entsprechenden Umsetzungen können bei Temperaturen von Raumtemperatur bis zur Rückflußtemperatur des jeweiligen Lösungsmittels bzw. -gemisches durchgeführt werden.

Im allgemeinen wird die dem Schema 4 entsprechende Ausgangsverbindung der allgemeinen Formel I gemäß Anspruch 1 mit einem bis fünf Äquivalenten an Oxidationsmittel Ox umgesetzt.

Die Reaktionskonzentration für die dem Schema 4 entsprechenden Umsetzungen liegt im allgemeinen im Bereich von 0,1 bis 5 molar, bevorzugt im Bereich von 0,2 bis 2 molar.

Als Oxidationsmittel Ox können für die dem Schema 4 entsprechenden Umsetzungen $H_2O_2$, organische Peroxysäuren wie z.B. Peressigsäure, Perbenzoesäure und substituierte Perbenzoesäuren, organische Peroxide wie z.B. tert.-Butylhydroperoxid und anorganische Peroxide wie z.B. $NaWO_4$ eingesetzt werden. Solche Oxidationsmittel Ox sind im allgemeinen handelsüblich.

Im Hinblick auf die bestimmungsgemäße Verwendung der Verbindungen I kommen als Initiatoren bevorzugt folgende Reste in Betracht:

$R^1$ Wasserstoff, Cyano

$C_1$-$C_8$-Alkyl wie Methyl, Ethyl, n-Propyl, iso-Propyl, n-Butyl, iso-Butyl, tert.-Butyl, geradkettiges oder verzweigtkettiges $C_5$- bis $C_8$-Alkyl, insbesondere Methyl, Ethyl, Propyl und iso-Propyl, wobei diese Gruppe durch einen der folgenden Reste substituiert sein kann:

Alkoxy wie Methoxy und Ethoxy, Alkylthio wie Methylthio und Ethylthio, Aryl- bzw. Heteroaryl wie Phenyl, Naphthyl, sechsgliedrige Heterocyclen mit einem oder mehreren Heteroatomen wie Pyridyl, Pyridazinyl, Pyrimidinyl, Pyrazinyl, 1.3.5-Triazinyl, 1.2.4-Triazinyl, 1.2.3-Triazinyl, Tetrazinyl, fünfgliedrige Heterocyclen mit einem oder mehreren Heteroatomen wie Pyrryl, Furyl, Thienyl, Pyrazolyl, Imidazolyl, Oxazolyl, Isoxazolyl, Thiazolyl, Isothiazolyl, 1.2.3-Triazolyl, 1.2.4-Triazolyl, Tetrazolyl, Thiadiazolyl, annellierte und benzokondensierte Heteroaromaten, wie Indolyl, Isoindolyl, Thionaphthyl, Chinolyl, Isochinolyl, Cinnolyl, Phthalazinyl, Chinazolyl, Chinoxalyl, Indazolyl, Naphthyridinyl, Benzthiazolyl, Benzimidazolyl, Benzofuryl, Benzoxazolyl und Benztriazolyl, oder einen entsprechenden Aryloxy- oder -thiorest bzw. Hetaryloxy- oder -thiorest,

$C_2$-$C_5$-Alkenyl, insbesondere Vinyl, Allyl, 1-Propenyl und Butenyl, $C_2$-$C_4$-Alkinyl, insbesondere Ethinyl, Propinyl und Butinyl, ein Rest -$COR^4$ wobei

$R^4$ $C_1$-$C_4$-Alkyl wie oben genannt, insbesondere Methyl, Ethyl und iso-Propyl,

$C_1$-$C_4$-Alkoxy, insbesondere Methoxy, Ethoxy, Propoxy und Butoxy, welches durch einen der vorstehend genannten Aryl- oder Heteroarylreste substituiert sein kann, $C_1$-$C_4$-Alkylthio, insbesondere Methylthio, Ethylthio, Propylthio und Butylthio oder Aryl, Heteroaryl, Aryloxy, Heteroaryloxy, Arylthio und Heteroarylthio wie vorstehend genannt

bedeutet,

ein Rest -$CONR^5R^6$ wobei

$R^5$, $R^6$ unabhängig voneinander Wasserstoff, $C_1$-$C_4$-Alkyl wie unter $R^4$ genannt, welches durch einen der vorstehend genannten Aryl- oder Heteroarylreste substituiert sein kann,

$C_3$-$C_6$-Cycloalkyl, wie Cyclopropyl, Cyclobutyl, Cyclopentyl und Cyclohexyl,

$C_2$-$C_5$-Alkenyl, Aryl und Heteroaryl wie vorstehend genannt, $C_1$-$C_4$-Alkylcarbonyl wie Methylcarbonyl, Ethylcarbonyl, Propylcarbonyl, iso-Propylcarbonyl, Butylcarbonyl, iso-Butylcarbonyl, sek.-Butylcarbonyl und tert.-Butylcarbonyl, insbesondere Methylcarbonyl, Ethylcarbonyl und Propylcarbonyl,

oder gemeinsam eine $C_2$-$C_6$-Alkylenkette wie Ethylen, Propylen, Butylen, Pentylen und Hexylen,

bedeutet, oder

ein Rest -$SO_mR^4$ worin

m den Wert 1 oder 2 hat und

$R^4$ die vorstehend gegebene Bedeutung besitzt,

$R^2$, $R^3$ unabhängig voneinander Nitro, Hydroxy, Carboxy, Mercapto, Halogen, insbesondere Fluor, Chlor und Brom,

$C_1$-$C_4$-Akyl wie unter $R^1$ genannt, insbesondere Methyl, Ethyl und iso-Propyl, welches ein- bis dreifach durch Halogenatome wie Fluor und Chlor und/oder einfach durch Hydroxy, Mercapto, Amino, Aryloxy oder Heteroaryloxy wie vorstehend genannt substituiert sein kann, $C_3$-$C_6$-Cycloalkyl wie unter $R^5$ genannt, welches ein- bis dreifach durch Halogen wie Fluor und Chlor, Hydroxy, Mercapto und/oder die oben genannten $C_1$-$C_4$-Alkylgruppen substituiert sein kann,

$C_3$-$C_5$-Cycloalkoxy, insbesondere Cyclopropyloxy, Cyclopentyloxy und Cyclohexyloxy bzw. die entsprechenden Cycloalkylthioreste, $C_2$-$C_5$-Halogenalkenyl, insbesondere durch Fluor oder Chlor, ein- bis dreifach substituiertes Allyl, But-2-enyl und But-3-enyl,

$C_2$-$C_4$-Halogenalkinyl, insbesondere durch Fluor oder Chlor, ein- bis dreifach substituiertes Prop-2-inyl, But-2-inyl und But-3-inyl,

$C_1$-$C_4$-Alkoxy wie unter $R^4$ genannt, insbesondere Methoxy, Ethoxy und iso-Propyloxy, wobei dieser Rest ein- bis dreifach durch Halogen wie Fluor und Chlor und/oder einfach durch Aryl oder Heteroaryl wie unter $R^1$ genannt substituiert sein kann,

$C_2$-$C_5$-Alkenyloxy, insbesondere Allyloxy, But-2-enyloxy und But-3-enyloxy,

$C_2$-$C_4$-Alkinyloxy, insbesondere Prop-2-inyloxy, But-2-inyloxy und But-3-inyloxy,

ein Rest -$NR^5R^6$ worin $R^5$ und $R^6$ die vorstehend gegebene Bedeutung besitzen oder

eine der unter $R^1$ genannten Gruppen,

W, X, Y und Z unabhängig voneinander Stickstoff oder eine Gruppe

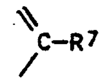

worin

$R^7$ Hydrazino oder eine der unter $R^2$ genannten Gruppen bedeutet, wobei W, X, Y und Z nicht gleichzeitig Stickstoff bedeuten,

n den Wert 0 oder 1 und

A eine der unter $R^1$ genannten Aryl- bzw. Heteroarylgruppen, wobei diese Gruppen ein bis fünf Halogenatome wie Fluor, Chlor und Brom und/oder ein bis drei der folgenden Substituenten tragen können:

- $SO_2R^8$, worin

$R^8$ Hydroxy, $C_1$-$C_4$-Alkoxy wie unter $R^4$ genannt, welches durch einen der vorstehend genannten Aryl- oder Heteroarylreste substituiert sein kann, ein Aryloxy- oder Heteroaryloxyrest wie vorstehend genannt oder eine Gruppe -$NR^5R^6$ worin $R^5$ und $R^6$ die vorstehend gegebene Bedeutung besitzen,

bedeutet, oder

die unter $R^2$ genannten Gruppen.

Ausgenommen sind die Verbindungen der Formel I, in der A die Bedeutung 4-Aminophenyl hat, n = 0 ist, $R^1$, $R^2$ und $R^3$ die Bedeutung Wasserstoff, W die Bedeutung Stickstoff, X, Y und Z die Bedeutung Kohlenstoff hat und $R^4$ an X und Z Methyl und im übrigen Wasserstoff ist, sowie deren 4-Aminophenyl-Umsetzungsprodukte.

Besonders bevorzugte Verbindungen sind Sulfonamide der allgemeinen Formel Ia

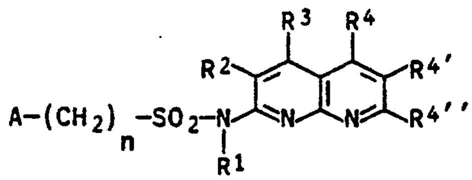

Ia

in der die Substituenten und Indices folgende Bedeutung haben:

$R^1$ Wasserstoff, eine $C_1$-$C_4$-Alkylcarbonylgruppe, welche im Alkylteil ein bis drei der folgenden Reste tragen kann: Halogenatome, $C_1$-$C_4$-Alkoxygruppen und/oder $C_1$-$C_4$-Alyklthiogruppen,

oder eine $C_1$-$C_4$-Alkylgruppe, welche ein bis drei der folgenden Reste tragen kann: Halogenatome, $C_1$-$C_4$-Alkoxygruppen und/oder $C_1$-$C_4$-Alkylthiogruppen,

$R^2$ Wasserstoff, eine Cyanogruppe oder ein Halogenatom,

$R^3$ eine $C_1$-$C_4$-Alkylgruppe oder ein Halogenatom,

$R^4$ $R^{4'}$ und $R^{4''}$ unabhängig voneinander Wasserstoff, eine Methylgruppe, eine Trifluormethylgruppe, eine Methoxygruppe, eine Dimethylaminogruppe, eine Methylthiogruppe, ein Phenyl- oder Phenoxyrest, eine Hydrazinogruppe oder ein Halogenatom wie Fluor, Chlor und Brom,

n 0 oder 1 und

A ein Phenylrest, ein Naphthylrest, ein Thienylrest oder ein Chinolinylrest, wobei diese aromatischen Reste ein bis drei der folgenden Gruppen tragen können: Halogen, $C_1$-$C_8$-Alkyl, $C_1$-$C_4$-Alkoxy, $C_1$-$C_2$-Alkoxy-$C_1$-$C_4$-Alkyl oder $C_1$-$C_4$-Alkoxycarbonyl,

sowie deren Salze und N-Oxide.

Des weiteren sind die Verbindungen Ib bevorzugt,

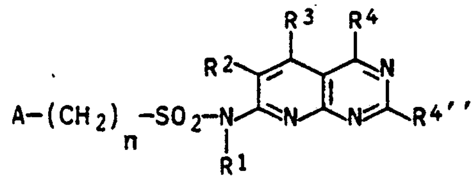

Ib

in der die Substituenten und Indices folgende Bedeutung haben:

$R^1$ Wasserstoff oder eine Methylgruppe,

$R^2$ Wasserstoff, eine Cyanogruppe, eine Methoxycarbonylgruppe, eine Methylsulfonylgruppe, ein Phenylrest oder ein Halogenatom wie Chlor oder Brom,

$R^3$ insbesondere Wasserstoff,

$R^4$ und $R^{4''}$ die unter Ia hierfür gegebene Bedeutung und
A ein Phenylrest oder ein Thienylrest, wobei diese aromatischen Reste insbesondere ein oder zwei der folgenden Reste tragen können:
Halogenatom wie Fluor, Chlor und Brom, Methyl, Methoxy, Methoxycarbonyl und/oder Cyclopentyl,
sowie deren Salze und N-Oxide.

Bevorzugte Verbindungen sind auch Sulfonamide der allgemeinen Formel Ic

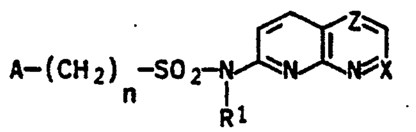

Ic,

in der A, X, Z, n und $R^1$ die vorstehend angegebene Bedeutung haben, sowie deren Salze und N-Oxide.

Weiterhin bevorzugte Verbindungen sind Sulfonamide der allgemeinen Formel Id

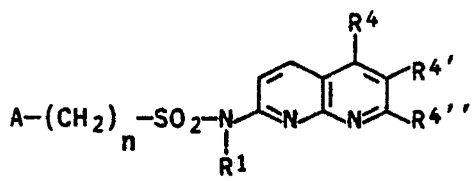

Id

in der A, n, $R^1$ und $R^4$, $R^{4'}$ und $R^{4''}$ die vorstehend angegebene Bedeutung haben, sowie deren Salze und N-Oxide.

Beispiele für sehr aktive Verbindungen der Formeln Ia, Ib, Ic und Id sind in den nachstehenden Tabellen I, II, III und IV aufgeführt.

Tabelle I

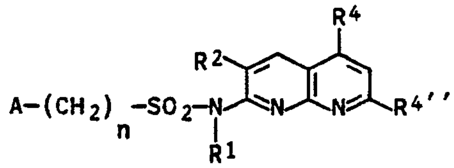

Ia

| A | n | $R^1$ | $R^2$ | $R^4$ | $R^{4''}$ |
|---|---|---|---|---|---|
| 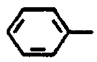 | 0 | H | H | $CH_3$ | $CH_3$ |
| 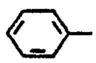 | 1 | H | H | $CH_3$ | $CH_3$ |
| 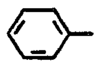 | 0 | $CH_3$ | H | $CH_3$ | $CH_3$ |
| 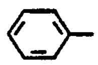 | 1 | $CH_3$ | H | $CH_3$ | $CH_3$ |
| 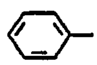 | 0 | $COCH_3$ | H | $CH_3$ | $CH_3$ |
| 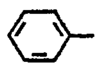 | 1 | $COCH_3$ | H | $CH_3$ | $CH_3$ |
| 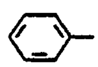 | 0 | H | Cl | $CH_3$ | $CH_3$ |
| 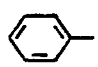 | 1 | H | Cl | $CH_3$ | $CH_3$ |
| 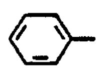 | 0 | $CH_3$ | Cl | $CH_3$ | $CH_3$ |
| 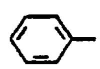 | 1 | $CH_3$ | Cl | $CH_3$ | $CH_3$ |
| 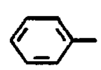 | 0 | $COCH_3$ | Cl | $CH_3$ | $CH_3$ |
| 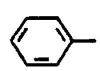 | 1 | $COCH_3$ | Cl | $CH_3$ | $CH_3$ |
| 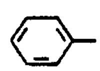 | 0 | H | CN | $CH_3$ | $CH_3$ |
| 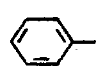 | 1 | H | CN | $CH_3$ | $CH_3$ |
| 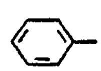 | 0 | $CH_3$ | CN | $CH_3$ | $CH_3$ |
| 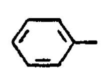 | 1 | $CH_3$ | CN | $CH_3$ | $CH_3$ |

Tabelle I - Forts.

| A | n | $R^1$ | $R^2$ | $R^4$ | $R^{4''}$ |
|---|---|---|---|---|---|
| 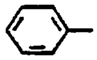 | 0 | $COCH_3$ | CN | $CH_3$ | $CH_3$ |
| 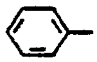 | 1 | $COCH_3$ | CN | $CH_3$ | $CH_3$ |
| 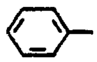 | 0 | H | H | $CH_3$ | Cl |
| 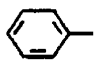 | 1 | H | H | $CH_3$ | Cl |
| 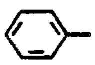 | 0 | $CH_3$ | H | $CH_3$ | Cl |
| 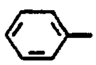 | 1 | $CH_3$ | H | $CH_3$ | Cl |
| 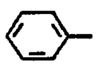 | 0 | $COCH_3$ | H | $CH_3$ | Cl |
| 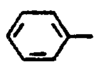 | 1 | $COCH_3$ | H | $CH_3$ | Cl |
| 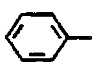 | 0 | H | Cl | $CH_3$ | Cl |
| 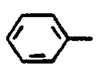 | 1 | H | Cl | $CH_3$ | Cl |
| 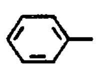 | 0 | $CH_3$ | Cl | $CH_3$ | Cl |
| 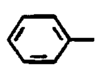 | 1 | $CH_3$ | Cl | $CH_3$ | Cl |
| 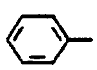 | 0 | $COCH_3$ | Cl | $CH_3$ | Cl |
| 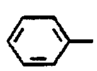 | 1 | $COCH_3$ | Cl | $CH_3$ | Cl |
| 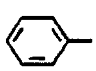 | 0 | H | CN | $CH_3$ | Cl |
| 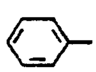 | 1 | H | CN | $CH_3$ | Cl |
| 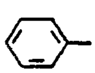 | 0 | $CH_3$ | CN | $CH_3$ | Cl |
| 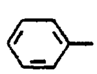 | 1 | $CH_3$ | CN | $CH_3$ | Cl |

Tabelle I - Forts.

| A | n | $R^1$ | $R^2$ | $R^4$ | $R^{4''}$ |
|---|---|---|---|---|---|
| 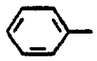 | 0 | $COCH_3$ | CN | $CH_3$ | Cl |
| 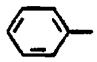 | 1 | $COCH_3$ | CN | $CH_3$ | Cl |
| 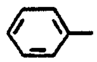 | 0 | H | H | $CH_3$ | $CF_3$ |
| 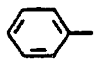 | 1 | H | H | $CH_3$ | $CF_3$ |
| 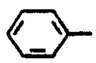 | 0 | $CH_3$ | H | $CH_3$ | $CF_3$ |
| 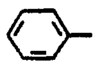 | 1 | $CH_3$ | H | $CH_3$ | $CF_3$ |
| 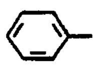 | 0 | $COCH_3$ | H | $CH_3$ | $CF_3$ |
| 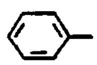 | 1 | $COCH_3$ | H | $CH_3$ | $CF_3$ |
| 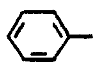 | 0 | H | Cl | $CH_3$ | $CF_3$ |
| 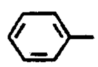 | 1 | H | Cl | $CH_3$ | $CF_3$ |
| 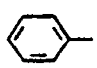 | 0 | $CH_3$ | Cl | $CH_3$ | $CF_3$ |
| 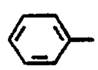 | 1 | $CH_3$ | Cl | $CH_3$ | $CF_3$ |
| 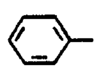 | 0 | $COCH_3$ | Cl | $CH_3$ | $CF_3$ |
| 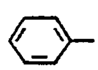 | 1 | $COCH_3$ | Cl | $CH_3$ | $CF_3$ |
| 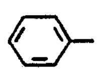 | 0 | H | CN | $CH_3$ | $CF_3$ |
| 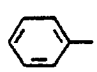 | 1 | H | CN | $CH_3$ | $CF_3$ |
| 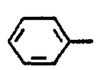 | 0 | $CH_3$ | CN | $CH_3$ | $CF_3$ |
| 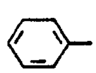 | 1 | $CH_3$ | CN | $CH_3$ | $CF_3$ |

Tabelle I - Forts.

| A | n | $R^1$ | $R^2$ | $R^4$ | $R^{4''}$ |
|---|---|---|---|---|---|
| 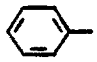 | 0 | $COCH_3$ | CN | $CH_3$ | $CF_3$ |
| 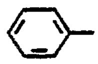 | 1 | $COCH_3$ | CN | $CH_3$ | $CF_3$ |
| 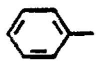 | 0 | H | H | $CH_3$ | $OCH_3$ |
| 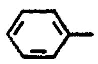 | 1 | H | H | $CH_3$ | $OCH_3$ |
| 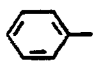 | 0 | $CH_3$ | H | $CH_3$ | $OCH_3$ |
| 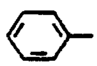 | 1 | $CH_3$ | H | $CH_3$ | $OCH_3$ |
| 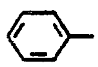 | 0 | $COCH_3$ | H | $CH_3$ | $OCH_3$ |
| 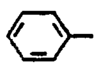 | 1 | $COCH_3$ | H | $CH_3$ | $OCH_3$ |
| 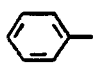 | 0 | H | Cl | $CH_3$ | $OCH_3$ |
| 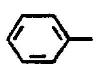 | 1 | H | Cl | $CH_3$ | $OCH_3$ |
| 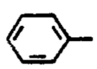 | 0 | $CH_3$ | Cl | $CH_3$ | $OCH_3$ |
| 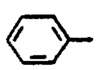 | 1 | $CH_3$ | Cl | $CH_3$ | $OCH_3$ |
| 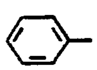 | 0 | $COCH_3$ | Cl | $CH_3$ | $OCH_3$ |
| 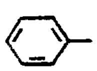 | 1 | $COCH_3$ | Cl | $CH_3$ | $OCH_3$ |
| 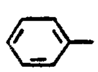 | 0 | H | CN | $CH_3$ | $OCH_3$ |
| 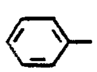 | 1 | H | CN | $CH_3$ | $OCH_3$ |
| 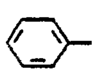 | 0 | $CH_3$ | CN | $CH_3$ | $OCH_3$ |
| 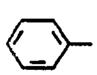 | 1 | $CH_3$ | CN | $CH_3$ | $OCH_3$ |

Tabelle I - Forts.

| A | n | $R^1$ | $R^2$ | $R^4$ | $R^{4''}$ |
|---|---|---|---|---|---|
| 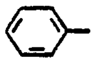 | 0 | $COCH_3$ | CN | $CH_3$ | $OCH_3$ |
| 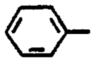 | 1 | $COCH_3$ | CN | $CH_3$ | $OCH_3$ |
| 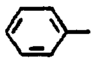 | 0 | H | H | $CF_3$ | $CH_3$ |
| 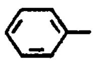 | 1 | H | H | $CF_3$ | $CH_3$ |
| 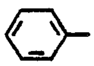 | 0 | $CH_3$ | H | $CF_3$ | $CH_3$ |
| 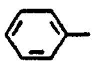 | 1 | $CH_3$ | H | $CF_3$ | $CH_3$ |
| 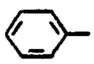 | 0 | $OCH_3$ | H | $CF_3$ | $CH_3$ |
| 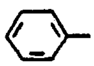 | 1 | $OCH_3$ | H | $CF_3$ | $CH_3$ |
| 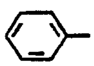 | 0 | H | Cl | $CF_3$ | $CH_3$ |
| 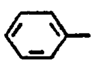 | 1 | H | Cl | $CF_3$ | $CH_3$ |
| 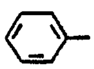 | 0 | $CH_3$ | Cl | $CF_3$ | $CH_3$ |
| 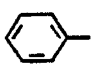 | 1 | $CH_3$ | Cl | $CF_3$ | $CH_3$ |
| 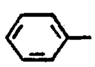 | 0 | $COCH_3$ | Cl | $CF_3$ | $CH_3$ |
| 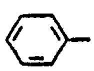 | 1 | $COCH_3$ | Cl | $CF_3$ | $CH_3$ |
| 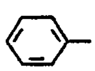 | 0 | H | CN | $CF_3$ | $CH_3$ |
| 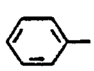 | 1 | H | CN | $CF_3$ | $CH_3$ |
| 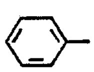 | 0 | $CH_3$ | CN | $CF_3$ | $CH_3$ |
| 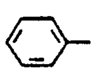 | 1 | $CH_3$ | CN | $CF_3$ | $CH_3$ |

Tabelle I - Forts.

| A | n | $R^1$ | $R^2$ | $R^4$ | $R^{4''}$ |
|---|---|---|---|---|---|
| 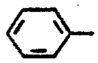 | 0 | $COCH_3$ | CN | $CF_3$ | $CH_3$ |
| 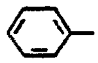 | 1 | $COCH_3$ | CN | $CF_3$ | $CH_3$ |
| 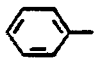 | 0 | H | H | $CF_3$ | $CF_3$ |
| 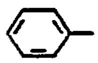 | 1 | H | H | $CF_3$ | $CF_3$ |
| 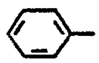 | 0 | $CH_3$ | H | $CF_3$ | $CF_3$ |
| 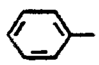 | 1 | $CH_3$ | H | $CF_3$ | $CF_3$ |
| 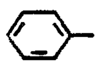 | 0 | $COCH_3$ | H | $CF_3$ | $CF_3$ |
| 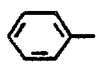 | 1 | $COCH_3$ | H | $CF_3$ | $CF_3$ |
| 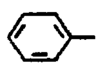 | 0 | H | Cl | $CF_3$ | $CF_3$ |
| 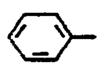 | 1 | H | Cl | $CF_3$ | $CF_3$ |
| 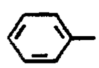 | 0 | $CH_3$ | Cl | $CF_3$ | $CF_3$ |
| 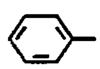 | 1 | $CH_3$ | Cl | $CF_3$ | $CF_3$ |
| 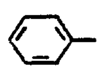 | 0 | $COCH_3$ | Cl | $CF_3$ | $CF_3$ |
| 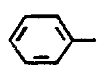 | 1 | $COCH_3$ | Cl | $CF_3$ | $CF_3$ |
| 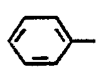 | 0 | H | CN | $CF_3$ | $CF_3$ |
| 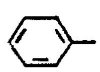 | 1 | H | CN | $CF_3$ | $CF_3$ |
| 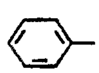 | 0 | $CH_3$ | CN | $CF_3$ | $CF_3$ |
| 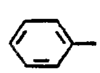 | 1 | $CH_3$ | CN | $CF_3$ | $CF_3$ |

Tabelle I - Forts.

| A | n | $R^1$ | $R^2$ | $R^4$ | $R^{4''}$ |
|---|---|---|---|---|---|
| 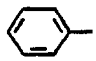 | 0 | $COCH_3$ | CN | $CF_3$ | $CF_3$ |
| 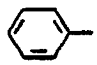 | 0 | $COCH_3$ | CN | $CF_3$ | $CF_3$ |
| 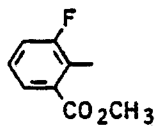 | 0 | H | H | $CH_3$ | $CH_3$ |
| 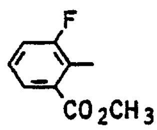 | 1 | H | H | $CH_3$ | $CH_3$ |
| 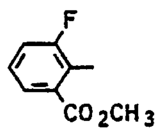 | 0 | $CH_3$ | H | $CH_3$ | $CH_3$ |
| 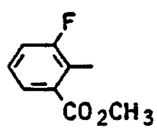 | 1 | $CH_3$ | H | $CH_3$ | $CH_3$ |
| 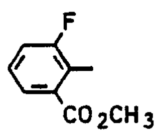 | 0 | $COCH_3$ | H | $CH_3$ | $CH_3$ |
| 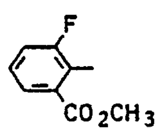 | 1 | $COCH_3$ | H | $CH_3$ | $CH_3$ |
| 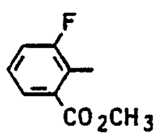 | 0 | H | Cl | $CH_3$ | $CH_3$ |
| 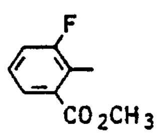 | 1 | H | Cl | $CH_3$ | $CH_3$ |
| 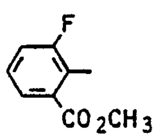 | 0 | $CH_3$ | Cl | $CH_3$ | $CH_3$ |
| 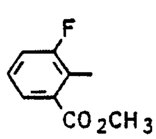 | 1 | $CH_3$ | Cl | $CH_3$ | $CH_3$ |

Tabelle I - Forts.

| A | n | $R^1$ | $R^2$ | $R^4$ | $R^{4''}$ |
|---|---|---|---|---|---|
| 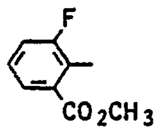 | 0 | $COCH_3$ | Cl | $CH_3$ | $CH_3$ |
| 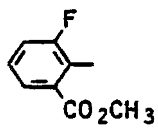 | 1 | $COCH_3$ | Cl | $CH_3$ | $CH_3$ |
| 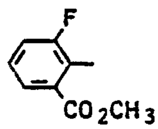 | 0 | H | CN | $CH_3$ | $CH_3$ |
| 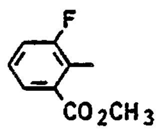 | 1 | H | CN | $CH_3$ | $CH_3$ |
| 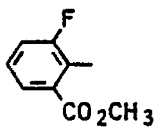 | 0 | $CH_3$ | CN | $CH_3$ | $CH_3$ |
| 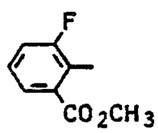 | 1 | $CH_3$ | CN | $CH_3$ | $CH_3$ |
| 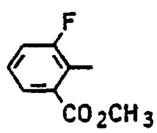 | 0 | $COCH_3$ | CN | $CH_3$ | $CH_3$ |
| 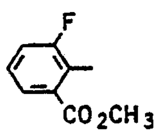 | 1 | $COCH_3$ | CN | $CH_3$ | $CH_3$ |
| 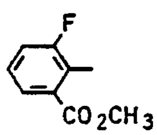 | 0 | H | H | $CH_3$ | Cl |
| 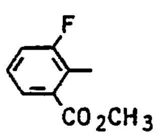 | 1 | H | H | $CH_3$ | Cl |
| 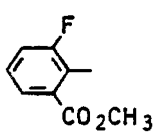 | 0 | $CH_3$ | H | $CH_3$ | Cl |
| 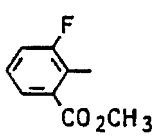 | 1 | $CH_3$ | H | $CH_3$ | Cl |

Tabelle I - Forts.

| A | n | $R^1$ | $R^2$ | $R^4$ | $R^{4''}$ |
|---|---|---|---|---|---|
| 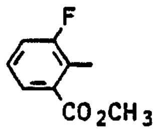 | 0 | $COCH_3$ | H | $CH_3$ | Cl |
| 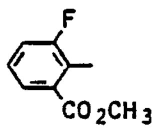 | 1 | $COCH_3$ | H | $CH_3$ | Cl |
| 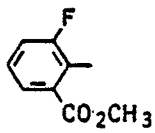 | 0 | H | Cl | $CH_3$ | Cl |
| 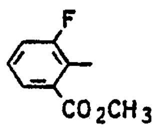 | 1 | H | Cl | $CH_3$ | Cl |
| 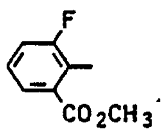 | 0 | $CH_3$ | Cl | $CH_3$ | Cl |
| 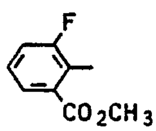 | 1 | $CH_3$ | Cl | $CH_3$ | Cl |
| 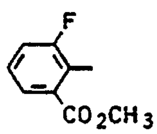 | 0 | $COCH_3$ | Cl | $CH_3$ | Cl |
| 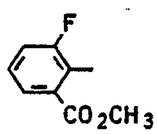 | 1 | $COCH_3$ | Cl | $CH_3$ | Cl |
| 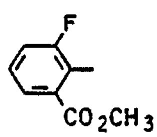 | 0 | H | CN | $CH_3$ | Cl |
| 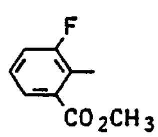 | 1 | H | CN | $CH_3$ | Cl |
| 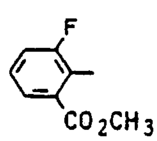 | 0 | $CH_3$ | CN | $CH_3$ | Cl |
| 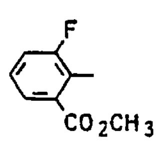 | 1 | $CH_3$ | CN | $CH_3$ | Cl |

Tabelle I - Forts.

| A | n | $R^1$ | $R^2$ | $R^4$ | $R^{4''}$ |
|---|---|---|---|---|---|
| 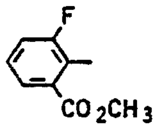 | 0 | $COCH_3$ | CN | $CH_3$ | Cl |
| 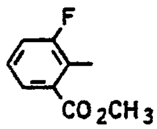 | 1 | $COCH_3$ | CN | $CH_3$ | Cl |
| 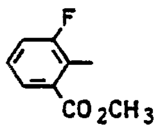 | 0 | H | H | $CH_3$ | $CF_3$ |
| 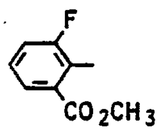 | 1 | H | H | $CH_3$ | $CF_3$ |
| 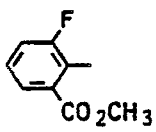 | 0 | $CH_3$ | H | $CH_3$ | $CF_3$ |
| 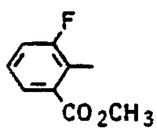 | 1 | $CH_3$ | H | $CH_3$ | $CF_3$ |
| 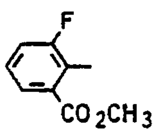 | 0 | $COCH_3$ | H | $CH_3$ | $CF_3$ |
| 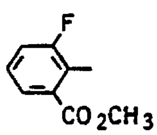 | 1 | $COCH_3$ | H | $CH_3$ | $CF_3$ |
| 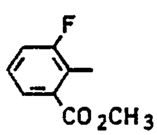 | 0 | H | Cl | $CH_3$ | $CF_3$ |
| 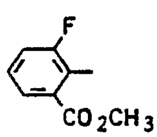 | 1 | H | Cl | $CH_3$ | $CF_3$ |
| 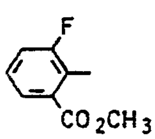 | 0 | $CH_3$ | Cl | $CH_3$ | $CF_3$ |
| 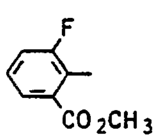 | 1 | $CH_3$ | Cl | $CH_3$ | $CF_3$ |

Tabelle I - Forts.

| A | n | $R^1$ | $R^2$ | $R^4$ | $R^{4''}$ |
|---|---|---|---|---|---|
| 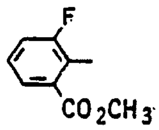 | 0 | $COCH_3$ | Cl | $CH_3$ | $CF_3$ |
| 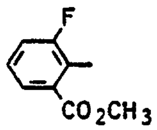 | 1 | $COCH_3$ | Cl | $CH_3$ | $CF_3$ |
| 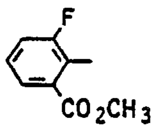 | 0 | H | CN | $CH_3$ | $CF_3$ |
| 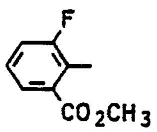 | 1 | H | CN | $CH_3$ | $CF_3$ |
| 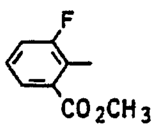 | 0 | $CH_3$ | CN | $CH_3$ | $CF_3$ |
| 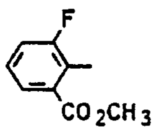 | 1 | $CH_3$ | CN | $CH_3$ | $CF_3$ |
| 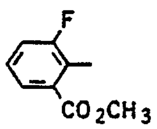 | 0 | $COCH_3$ | CN | $CH_3$ | $CF_3$ |
| 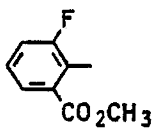 | 1 | $COCH_3$ | CN | $CH_3$ | $CF_3$ |
| 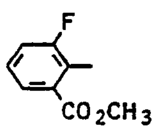 | 0 | H | H | $CH_3$ | $OCH_3$ |
| 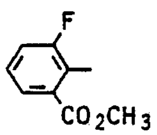 | 1 | H | H | $CH_3$ | $OCH_3$ |
| 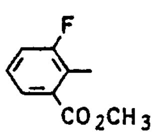 | 0 | $CH_3$ | H | $CH_3$ | $OCH_3$ |
| 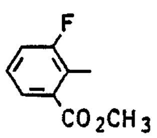 | 1 | $CH_3$ | H | $CH_3$ | $OCH_3$ |

Tabelle I - Forts.

| A | n | $R^1$ | $R^2$ | $R^4$ | $R^{4''}$ |
|---|---|---|---|---|---|
| 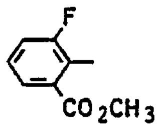 | 0 | $COCH_3$ | H | $CH_3$ | $OCH_3$ |
| 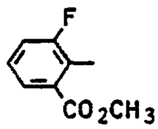 | 1 | $COCH_3$ | H | $CH_3$ | $OCH_3$ |
| 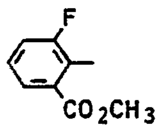 | 0 | H | Cl | $CH_3$ | $OCH_3$ |
| 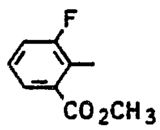 | 1 | H | Cl | $CH_3$ | $OCH_3$ |
| 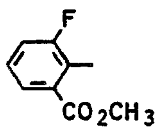 | 0 | $CH_3$ | Cl | $CH_3$ | $OCH_3$ |
| 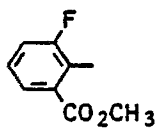 | 1 | $CH_3$ | Cl | $CH_3$ | $OCH_3$ |
| 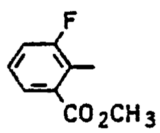 | 0 | $COCH_3$ | Cl | $CH_3$ | $OCH_3$ |
| 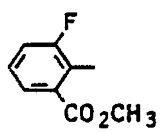 | 1 | $COCH_3$ | Cl | $CH_3$ | $OCH_3$ |
| 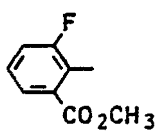 | 0 | H | CN | $CH_3$ | $OCH_3$ |
| 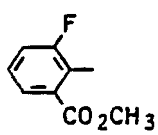 | 1 | H | CN | $CH_3$ | $OCH_3$ |
| 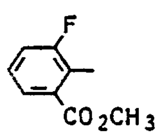 | 0 | $CH_3$ | CN | $CH_3$ | $OCH_3$ |
| 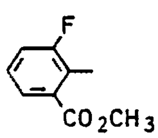 | 1 | $CH_3$ | CN | $CH_3$ | $OCH_3$ |

Tabelle I - Forts.

| A | n | $R^1$ | $R^2$ | $R^4$ | $R^{4''}$ |
|---|---|---|---|---|---|
| 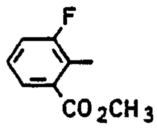 | 0 | $COCH_3$ | CN | $CH_3$ | $OCH_3$ |
| 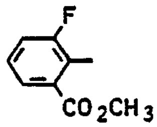 | 1 | $COCH_3$ | CN | $CH_3$ | $OCH_3$ |
| 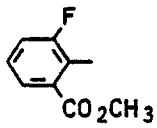 | 0 | H | H | $CF_3$ | $CH_3$ |
| 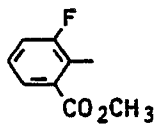 | 1 | H | H | $CF_3$ | $CH_3$ |
| 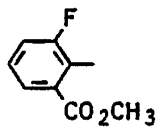 | 0 | $CH_3$ | H | $CF_3$ | $CH_3$ |
| 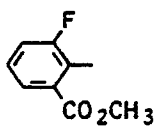 | 1 | $CH_3$ | H | $CF_3$ | $CH_3$ |
| 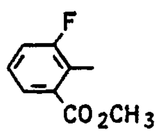 | 0 | $COCH_3$ | H | $CF_3$ | $CH_3$ |
| 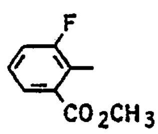 | 1 | $COCH_3$ | H | $CF_3$ | $CH_3$ |
| 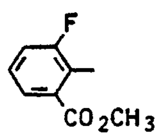 | 0 | H | Cl | $CF_3$ | $CH_3$ |
| 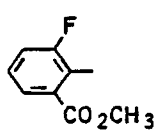 | 1 | H | Cl | $CF_3$ | $CH_3$ |
| 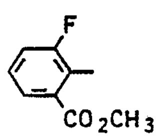 | 0 | $CH_3$ | Cl | $CF_3$ | $CH_3$ |
| 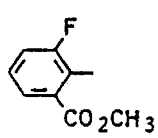 | 1 | $CH_3$ | Cl | $CF_3$ | $CH_3$ |

Tabelle I - Forts.

| A | n | $R^1$ | $R^2$ | $R^4$ | $R^{4''}$ |
|---|---|---|---|---|---|
| 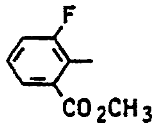 | 0 | $COCH_3$ | Cl | $CF_3$ | $CH_3$ |
| 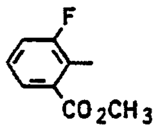 | 1 | $COCH_3$ | Cl | $CF_3$ | $CH_3$ |
| 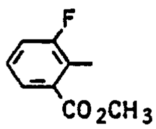 | 0 | H | CN | $CF_3$ | $CH_3$ |
| 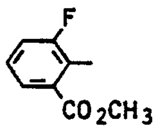 | 1 | H | CN | $CF_3$ | $CH_3$ |
| 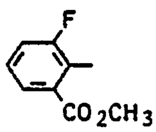 | 0 | $CH_3$ | CN | $CF_3$ | $CH_3$ |
| 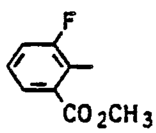 | 1 | $CH_3$ | CN | $CF_3$ | $CH_3$ |
| 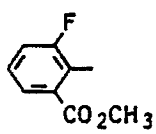 | 0 | $COCH_3$ | CN | $CF_3$ | $CH_3$ |
| 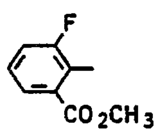 | 1 | $COCH_3$ | CN | $CF_3$ | $CH_3$ |
| 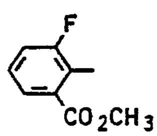 | 0 | H | H | $CF_3$ | $CF_3$ |
| 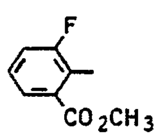 | 1 | H | H | $CF_3$ | $CF_3$ |
| 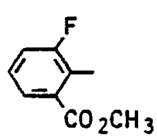 | 0 | $CH_3$ | H | $CF_3$ | $CF_3$ |
| 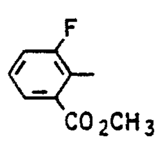 | 1 | $CH_3$ | H | $CF_3$ | $CF_3$ |

Tabelle I - Forts.

| A | n | $R^1$ | $R^2$ | $R^4$ | $R^{4''}$ |
|---|---|---|---|---|---|
| 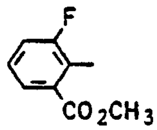 | 0 | $COCH_3$ | H | $CF_3$ | $CF_3$ |
| 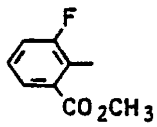 | 1 | $COCH_3$ | H | $CF_3$ | $CF_3$ |
| 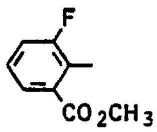 | 0 | H | Cl | $CF_3$ | $CF_3$ |
| 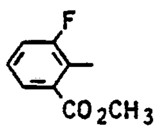 | 1 | H | Cl | $CF_3$ | $CF_3$ |
| 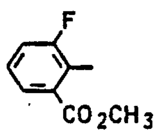 | 0 | $CH_3$ | Cl | $CF_3$ | $CF_3$ |
| 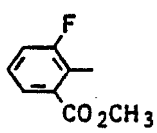 | 1 | $CH_3$ | Cl | $CF_3$ | $CF_3$ |
| 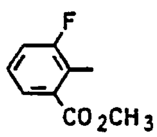 | 0 | $COCH_3$ | Cl | $CF_3$ | $CF_3$ |
| 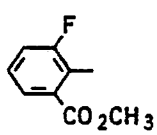 | 1 | $COCH_3$ | Cl | $CF_3$ | $CF_3$ |
| 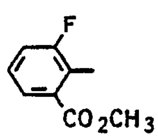 | 0 | H | CN | $CF_3$ | $CF_3$ |
| 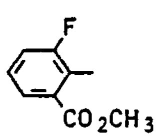 | 1 | H | CN | $CF_3$ | $CF_3$ |
| 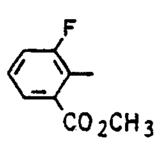 | 0 | $CH_3$ | CN | $CF_3$ | $CF_3$ |
| 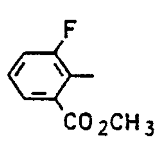 | 1 | $CH_3$ | CN | $CF_3$ | $CF_3$ |

Tabelle I - Forts.

| A | n | $R^1$ | $R^2$ | $R^4$ | $R^{4''}$ |
|---|---|---|---|---|---|
| 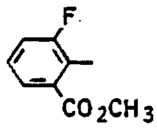 | 0 | $COCH_3$ | CN | $CF_3$ | $CF_3$ |
| 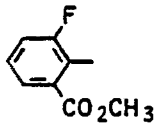 | 1 | $COCH_3$ | CN | $CF_3$ | $CF_3$ |
| 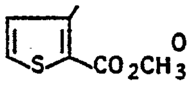 | 0 | H | H | $CH_3$ | $CH_3$ |
| 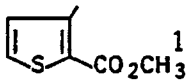 | 1 | H | H | $CH_3$ | $CH_3$ |
| 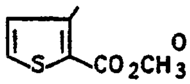 | 0 | $CH_3$ | H | $CH_3$ | $CH_3$ |
| 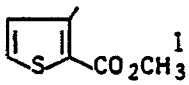 | 1 | $CH_3$ | H | $CH_3$ | $CH_3$ |
| 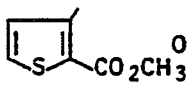 | 0 | $COCH_3$ | H | $CH_3$ | $CH_3$ |
| 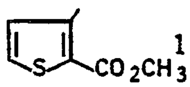 | 1 | $COCH_3$ | H | $CH_3$ | $CH_3$ |
| 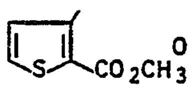 | 0 | H | Cl | $CH_3$ | $CH_3$ |
| 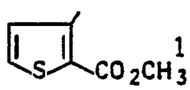 | 1 | H | Cl | $CH_3$ | $CH_3$ |
| 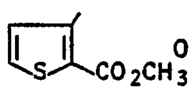 | 0 | $CH_3$ | Cl | $CH_3$ | $CH_3$ |
| 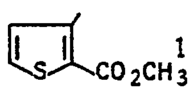 | 1 | $CH_3$ | Cl | $CH_3$ | $CH_3$ |
| 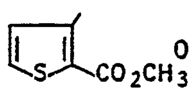 | 0 | $COCH_3$ | Cl | $CH_3$ | $CH_3$ |
| 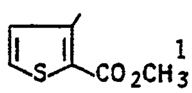 | 1 | $COCH_3$ | Cl | $CH_3$ | $CH_3$ |
| 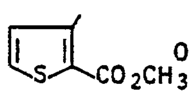 | 0 | H | CN | $CH_3$ | $CH_3$ |
| 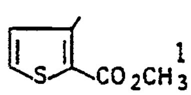 | 1 | H | CN | $CH_3$ | $CH_3$ |
| 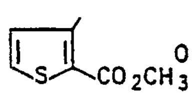 | 0 | $CH_3$ | CN | $CH_3$ | $CH_3$ |

Tabelle I - Forts.

| A | n | $R^1$ | $R^2$ | $R^4$ | $R^{4''}$ |
|---|---|---|---|---|---|
| 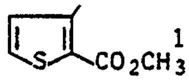 | 1 | $CH_3$ | CN | $CH_3$ | $CH_3$ |
| 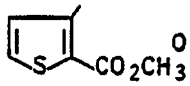 | 0 | $COCH_3$ | CN | $CH_3$ | $CH_3$ |
| 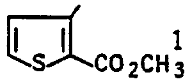 | 1 | $COCH_3$ | CN | $CH_3$ | $CH_3$ |
| 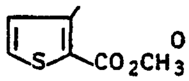 | 0 | H | H | $CH_3$ | Cl |
| 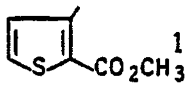 | 1 | H | H | $CH_3$ | Cl |
| 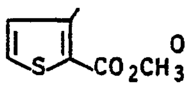 | 0 | $CH_3$ | H | $CH_3$ | Cl |
| 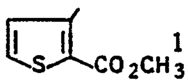 | 1 | $CH_3$ | H | $CH_3$ | Cl |
| 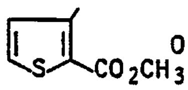 | 0 | $COCH_3$ | H | $CH_3$ | Cl |
| 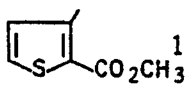 | 1 | $COCH_3$ | H | $CH_3$ | Cl |
| 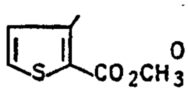 | 0 | H | Cl | $CH_3$ | Cl |
| 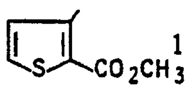 | 1 | H | Cl | $CH_3$ | Cl |
| 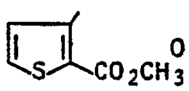 | 0 | $CH_3$ | Cl | $CH_3$ | Cl |
| 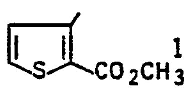 | 1 | $CH_3$ | Cl | $CH_3$ | Cl |
| 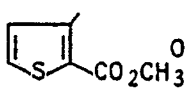 | 0 | $COCH_3$ | Cl | $CH_3$ | Cl |
| 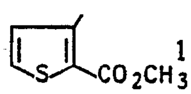 | 1 | $COCH_3$ | Cl | $CH_3$ | Cl |
| 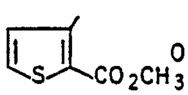 | 0 | H | CN | $CH_3$ | Cl |
| 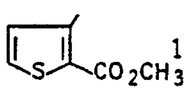 | 1 | H | CN | $CH_3$ | Cl |

Tabelle I - Forts.

| A | n | $R^1$ | $R^2$ | $R^4$ | $R^{4''}$ |
|---|---|---|---|---|---|
| 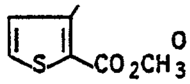 | 0 | $CH_3$ | CN | $CH_3$ | Cl |
| 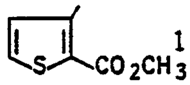 | 1 | $CH_3$ | CN | $CH_3$ | Cl |
| 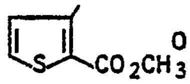 | 0 | $COCH_3$ | CN | $CH_3$ | Cl |
| 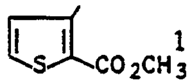 | 1 | $COCH_3$ | CN | $CH_3$ | Cl |
| 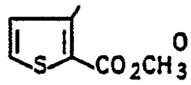 | 0 | H | H | $CH_3$ | $CF_3$ |
| 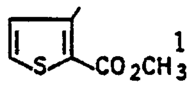 | 1 | H | H | $CH_3$ | $CF_3$ |
| 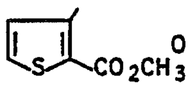 | 0 | $CH_3$ | H | $CH_3$ | $CF_3$ |
| 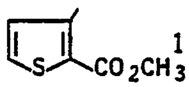 | 1 | $CH_3$ | H | $CH_3$ | $CF_3$ |
| 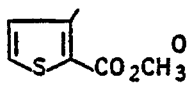 | 0 | $COCH_3$ | H | $CH_3$ | $CF_3$ |
| 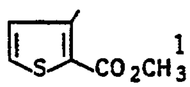 | 1 | $COCH_3$ | H | $CH_3$ | $CF_3$ |
| 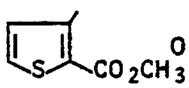 | 0 | H | Cl | $CH_3$ | $CF_3$ |
| 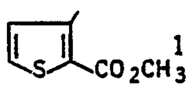 | 1 | H | Cl | $CH_3$ | $CF_3$ |
| 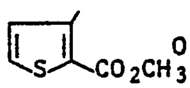 | 0 | $CH_3$ | Cl | $CH_3$ | $CF_3$ |
| 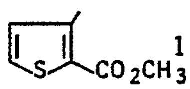 | 1 | $CH_3$ | Cl | $CH_3$ | $CF_3$ |
| 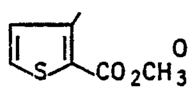 | 0 | $COCH_3$ | Cl | $CH_3$ | $CF_3$ |
| 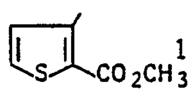 | 1 | $COCH_3$ | Cl | $CH_3$ | $CF_3$ |

Tabelle I - Forts.

| A | n | $R^1$ | $R^2$ | $R^4$ | $R^{4''}$ |
|---|---|---|---|---|---|
| 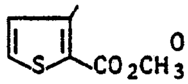 | 0 | H | CN | $CH_3$ | $CF_3$ |
| 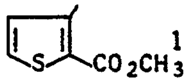 | 1 | H | CN | $CH_3$ | $CF_3$ |
| 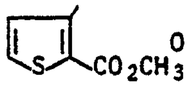 | 0 | $CH_3$ | CN | $CH_3$ | $CF_3$ |
| 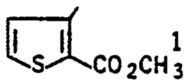 | 1 | $CH_3$ | CN | $CH_3$ | $CF_3$ |
| 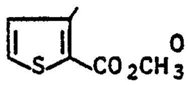 | 0 | $COCH_3$ | CN | $CH_3$ | $CF_3$ |
| 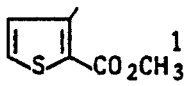 | 1 | $COCH_3$ | CN | $CH_3$ | $CF_3$ |
| 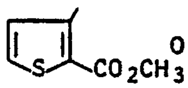 | 0 | H | H | $CH_3$ | $OCH_3$ |
| 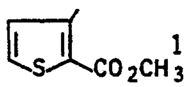 | 1 | H | H | $CH_3$ | $OCH_3$ |
| 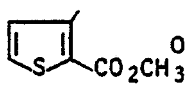 | 0 | $CH_3$ | H | $CH_3$ | $OCH_3$ |
| 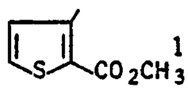 | 1 | $CH_3$ | H | $CH_3$ | $OCH_3$ |
| 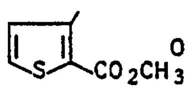 | 0 | $COCH_3$ | H | $CH_3$ | $OCH_3$ |
| 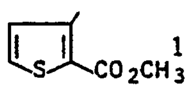 | 1 | $COCH_3$ | H | $CH_3$ | $OCH_3$ |
| 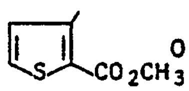 | 0 | H | Cl | $CH_3$ | $OCH_3$ |
| 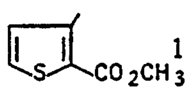 | 1 | H | Cl | $CH_3$ | $OCH_3$ |
| 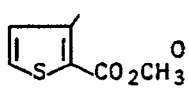 | 0 | $CH_3$ | Cl | $CH_3$ | $OCH_3$ |
| 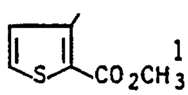 | 1 | $CH_3$ | Cl | $CH_3$ | $OCH_3$ |

Tabelle I - Forts.

| A | n | $R^1$ | $R^2$ | $R^4$ | $R^{4''}$ |
|---|---|---|---|---|---|
| 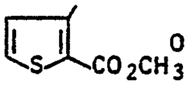 | 0 | $COCH_3$ | Cl | $CH_3$ | $OCH_3$ |
| 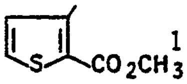 | 1 | $OCH_3$ | Cl | $CH_3$ | $OCH_3$ |
| 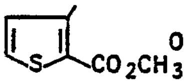 | 0 | H | CN | $CH_3$ | $OCH_3$ |
| 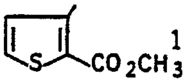 | 1 | H | CN | $CH_3$ | $OCH_3$ |
| 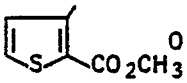 | 0 | $CH_3$ | CN | $CH_3$ | $OCH_3$ |
| 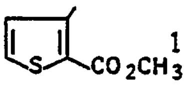 | 1 | $CH_3$ | CN | $CH_3$ | $OCH_3$ |
| 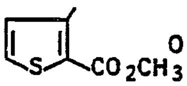 | 0 | $COCH_3$ | CN | $CH_3$ | $OCH_3$ |
| 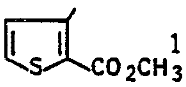 | 1 | $COCH_3$ | CN | $CH_3$ | $OCH_3$ |
| 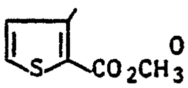 | 0 | H | H | $CF_3$ | $CH_3$ |
| 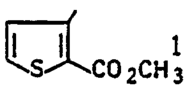 | 1 | H | H | $CF_3$ | $CH_3$ |
| 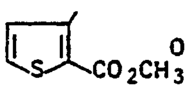 | 0 | $CH_3$ | H | $CF_3$ | $CH_3$ |
| 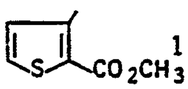 | 1 | $CH_3$ | H | $CF_3$ | $CH_3$ |
| 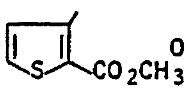 | 0 | $COCH_3$ | H | $CF_3$ | $CH_3$ |
| 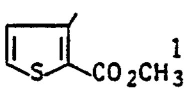 | 1 | $COCH_3$ | H | $CF_3$ | $CH_3$ |
| 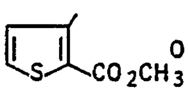 | 0 | H | Cl | $CF_3$ | $CH_3$ |
| 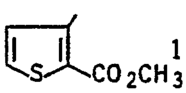 | 1 | H | Cl | $CF_3$ | $CH_3$ |

Tabelle I - Forts.

| A | n | $R^1$ | $R^2$ | $R^4$ | $R^{4''}$ |
|---|---|---|---|---|---|
| 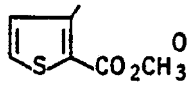 | 0 | $CH_3$ | Cl | $CF_3$ | $CH_3$ |
| 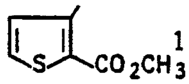 | 1 | $CH_3$ | Cl | $CF_3$ | $CH_3$ |
| 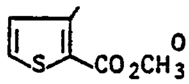 | 0 | $COCH_3$ | Cl | $CF_3$ | $CH_3$ |
| 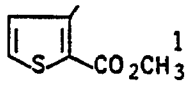 | 1 | $COCH_3$ | Cl | $CF_3$ | $CH_3$ |
| 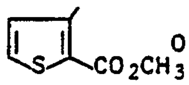 | 0 | H | CN | $CF_3$ | $CH_3$ |
| 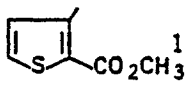 | 1 | H | CN | $CF_3$ | $CH_3$ |
| 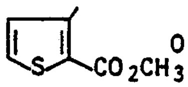 | 0 | $CH_3$ | CN | $CF_3$ | $CH_3$ |
| 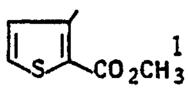 | 1 | $CH_3$ | CN | $CF_3$ | $CH_3$ |
| 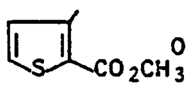 | 0 | $COCH_3$ | CN | $CF_3$ | $CH_3$ |
| 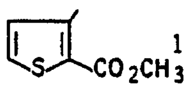 | 1 | $COCH_3$ | CN | $CF_3$ | $CH_3$ |
| 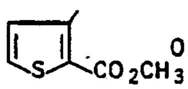 | 0 | H | H | $CF_3$ | $CF_3$ |
| 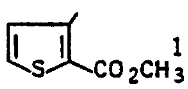 | 1 | H | H | $CF_3$ | $CF_3$ |
| 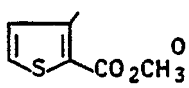 | 0 | $CH_3$ | H | $CF_3$ | $CF_3$ |
| 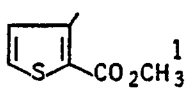 | 1 | $CH_3$ | H | $CF_3$ | $CF_3$ |
| 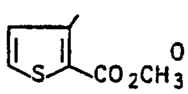 | 0 | $COCH_3$ | H | $CF_3$ | $CF_3$ |
| 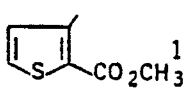 | 1 | $COCH_3$ | H | $CF_3$ | $CF_3$ |

Tabelle I - Forts.

| A | n | $R^1$ | $R^2$ | $R^4$ | $R^{4''}$ |
|---|---|---|---|---|---|
| 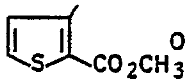 | 0 | H | Cl | $CF_3$ | $CF_3$ |
| 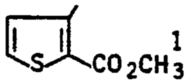 | 1 | H | Cl | $CF_3$ | $CF_3$ |
| 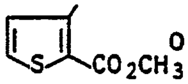 | 0 | $CH_3$ | Cl | $CF_3$ | $CF_3$ |
| 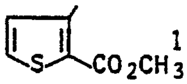 | 1 | $CH_3$ | Cl | $CF_3$ | $CF_3$ |
| 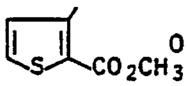 | 0 | $COCH_3$ | Cl | $CF_3$ | $CF_3$ |
| 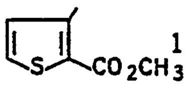 | 1 | $COCH_3$ | Cl | $CF_3$ | $CF_3$ |
| 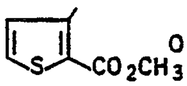 | 0 | H | CN | $CF_3$ | $CF_3$ |
| 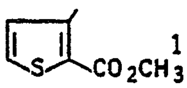 | 1 | H | CN | $CF_3$ | $CF_3$ |
| 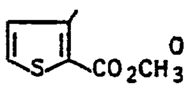 | 0 | $CH_3$ | CN | $CF_3$ | $CF_3$ |
| 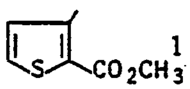 | 1 | $CH_3$ | CN | $CF_3$ | $CF_3$ |
| 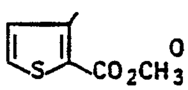 | 0 | $COCH_3$ | CN | $CF_3$ | $CF_3$ |
| 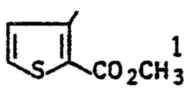 | 1 | $COCH_3$ | CN | $CF_3$ | $CF_3$ |
| 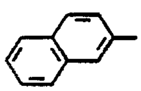 | 0 | H | H | $CH_3$ | $CH_3$ |
| 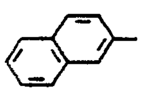 | 1 | H | H | $CH_3$ | $CH_3$ |
| 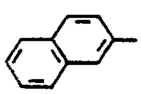 | 0 | $CH_3$ | H | $CH_3$ | $CH_3$ |
| 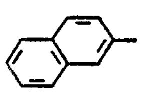 | 1 | $CH_3$ | H | $CH_3$ | $CH_3$ |

Tabelle I - Forts.

| A | n | R1 | R2 | R4 | R4'' |
|---|---|---|---|---|---|
| 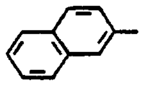 | 0 | $COCH_3$ | H | $CH_3$ | $CH_3$ |
| 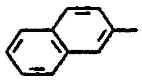 | 1 | $COCH_3$ | H | $CH_3$ | $CH_3$ |
| 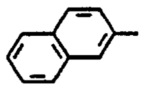 | 0 | H | Cl | $CH_3$ | $CH_3$ |
| 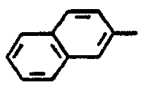 | 1 | H | Cl | $CH_3$ | $CH_3$ |
| 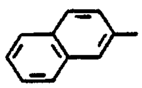 | 0 | $CH_3$ | Cl | $CH_3$ | $CH_3$ |
| 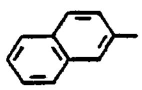 | 1 | $CH_3$ | Cl | $CH_3$ | $CH_3$ |
| 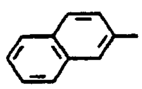 | 0 | $COCH_3$ | Cl | $CH_3$ | $CH_3$ |
| 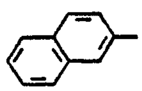 | 1 | $COCH_3$ | Cl | $CH_3$ | $CH_3$ |
| 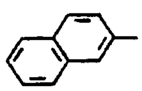 | 0 | H | CN | $CH_3$ | $CH_3$ |
| 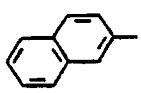 | 1 | H | CN | $CH_3$ | $CH_3$ |
| 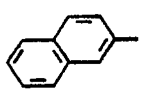 | 0 | $CH_3$ | CN | $CH_3$ | $CH_3$ |
| 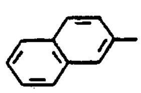 | 1 | $CH_3$ | CN | $CH_3$ | $CH_3$ |
| 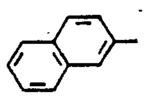 | 0 | $COCH_3$ | CN | $CH_3$ | $CH_3$ |
| 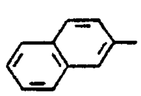 | 1 | $COCH_3$ | CN | $CH_3$ | $CH_3$ |

Tabelle I - Forts.

| A | n | $R^1$ | $R^2$ | $R^4$ | $R^{4''}$ |
|---|---|---|---|---|---|
| 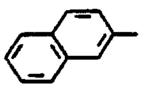 | 0 | H | H | $CH_3$ | Cl |
| 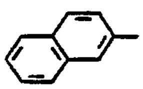 | 1 | H | H | $CH_3$ | Cl |
| 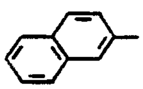 | 0 | $CH_3$ | H | $CH_3$ | Cl |
| 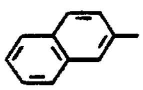 | 1 | $CH_3$ | H | $CH_3$ | Cl |
| 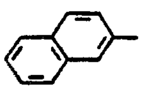 | 0 | $COCH_3$ | H | $CH_3$ | Cl |
| 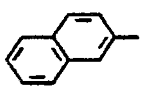 | 1 | $COCH_3$ | H | $CH_3$ | Cl |
| 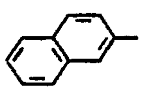 | 0 | H | Cl | $CH_3$ | Cl |
| 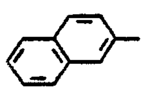 | 1 | H | Cl | $CH_3$ | Cl |
| 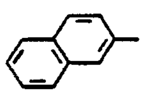 | 0 | $CH_3$ | Cl | $CH_3$ | Cl |
| 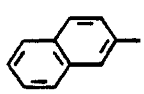 | 1 | $CH_3$ | Cl | $CH_3$ | Cl |
| 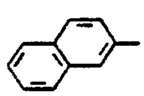 | 0 | $COCH_3$ | Cl | $CH_3$ | Cl |
| 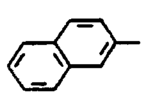 | 0 | $COCH_3$ | Cl | $CH_3$ | Cl |
| 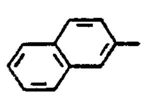 | 0 | H | CN | $CH_3$ | Cl |
| 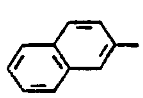 | 1 | H | CN | $CH_3$ | Cl |

Tabelle I - Forts.

| A | n | R1 | R2 | R4 | R4'' |
|---|---|---|---|---|---|
| 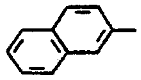 | 0 | $CH_3$ | CN | $CH_3$ | Cl |
| 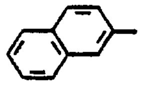 | 1 | $CH_3$ | CN | $CH_3$ | Cl |
| 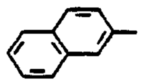 | 0 | $COCH_3$ | CN | $CH_3$ | Cl |
| 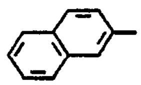 | 1 | $COCH_3$ | CN | $CH_3$ | Cl |
| 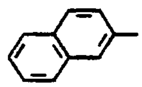 | 0 | H | H | $CH_3$ | $CF_3$ |
| 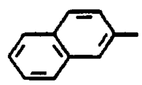 | 1 | H | H | $CH_3$ | $CF_3$ |
| 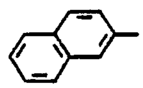 | 0 | $CH_3$ | H | $CH_3$ | $CF_3$ |
| 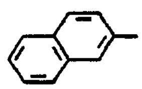 | 1 | $CH_3$ | H | $CH_3$ | $CF_3$ |
| 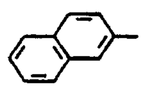 | 0 | $COCH_3$ | H | $CH_3$ | $CF_3$ |
| 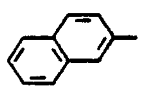 | 1 | $COCH_3$ | H | $CH_3$ | $CF_3$ |
| 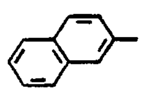 | 0 | H | Cl | $CH_3$ | $CF_3$ |
| 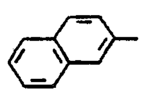 | 1 | H | Cl | $CH_3$ | $CF_3$ |
| 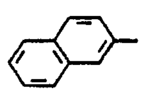 | 0 | $CH_3$ | Cl | $CH_3$ | $CF_3$ |
| 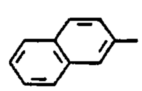 | 1 | $CH_3$ | Cl | $CH_3$ | $CF_3$ |

Tabelle I - Forts.

| A | n | R1 | R2 | R4 | R4'' |
|---|---|---|---|---|---|
| 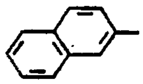 | 0 | $COCH_3$ | Cl | $CH_3$ | $CF_3$ |
| 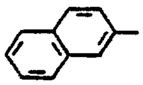 | 1 | $COCH_3$ | Cl | $CH_3$ | $CF_3$ |
| 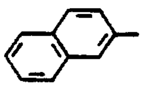 | 0 | H | CN | $CH_3$ | $CF_3$ |
| 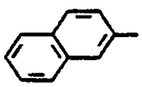 | 1 | H | CN | $CH_3$ | $CF_3$ |
| 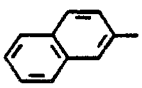 | 0 | $CH_3$ | CN | $CH_3$ | $CF_3$ |
| 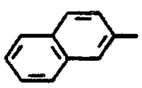 | 1 | $CH_3$ | CN | $CH_3$ | $CF_3$ |
| 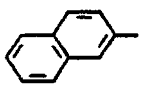 | 0 | $COCH_3$ | CN | $CH_3$ | $CF_3$ |
| 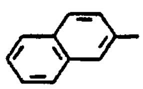 | 1 | $COCH_3$ | CN | $CH_3$ | $CF_3$ |
| 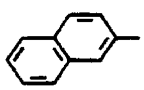 | 0 | H | H | $CH_3$ | $OCH_3$ |
| 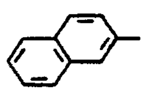 | 1 | H | H | $CH_3$ | $OCH_3$ |
| 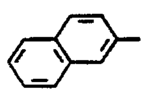 | 0 | $CH_3$ | H | $CH_3$ | $OCH_3$ |
| 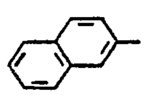 | 1 | $CH_3$ | H | $CH_3$ | $OCH_3$ |
| 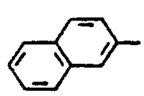 | 0 | $COCH_3$ | H | $CH_3$ | $OCH_3$ |
| 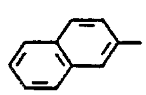 | 1 | $COCH_3$ | H | $CH_3$ | $OCH_3$ |

Tabelle I - Forts.

| A | n | $R^1$ | $R^2$ | $R^4$ | $R^{4''}$ |
|---|---|---|---|---|---|
| 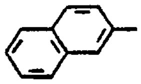 | 0 | H | Cl | $CH_3$ | $OCH_3$ |
| 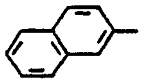 | 1 | H | Cl | $CH_3$ | $OCH_3$ |
| 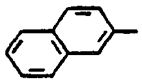 | 0 | $CH_3$ | Cl | $CH_3$ | $OCH_3$ |
| 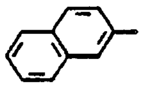 | 1 | $CH_3$ | Cl | $CH_3$ | $OCH_3$ |
| 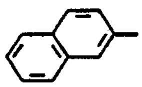 | 0 | $COCH_3$ | Cl | $CH_3$ | $OCH_3$ |
| 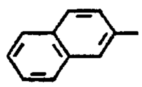 | 1 | $COCH_3$ | Cl | $CH_3$ | $OCH_3$ |
| 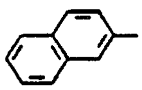 | 0 | H | CN | $CH_3$ | $OCH_3$ |
| 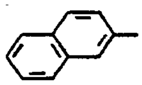 | 1 | H | CN | $CH_3$ | $OCH_3$ |
| 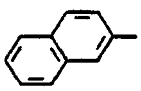 | 0 | $CH_3$ | CN | $CH_3$ | $OCH_3$ |
| 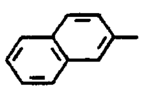 | 1 | $CH_3$ | CN | $CH_3$ | $OCH_3$ |
| 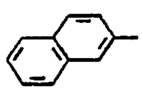 | 0 | $COCH_3$ | CN | $CH_3$ | $OCH_3$ |
| 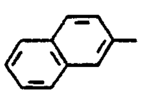 | 1 | $COCH_3$ | CN | $CH_3$ | $OCH_3$ |
| 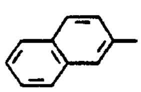 | 0 | H | H | $CF_3$ | $CH_3$ |
| 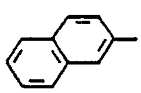 | 1 | H | H | $CF_3$ | $CH_3$ |

Tabelle I - Forts.

| A | n | $R^1$ | $R^2$ | $R^4$ | $R^{4''}$ |
|---|---|---|---|---|---|
| 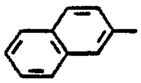 | 0 | $CH_3$ | H | $CF_3$ | $CH_3$ |
| 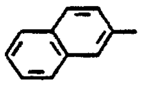 | 1 | $CH_3$ | H | $CF_3$ | $CH_3$ |
| 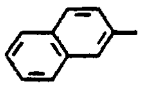 | 0 | $COCH_3$ | H | $CF_3$ | $CH_3$ |
| 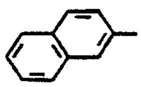 | 1 | $COCH_3$ | H | $CF_3$ | $CH_3$ |
| 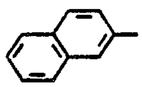 | 0 | H | Cl | $CF_3$ | $CH_3$ |
| 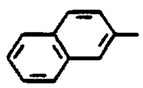 | 1 | H | Cl | $CF_3$ | $CH_3$ |
| 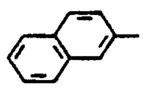 | 0 | $CH_3$ | Cl | $CF_3$ | $CH_3$ |
| 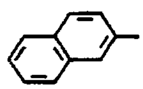 | 1 | $CH_3$ | Cl | $CF_3$ | $CH_3$ |
| 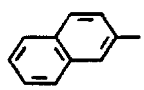 | 0 | $COCH_3$ | Cl | $CF_3$ | $CH_3$ |
| 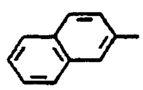 | 1 | $COCH_3$ | Cl | $CF_3$ | $CH_3$ |
| 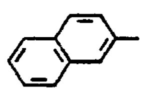 | 0 | H | CN | $CF_3$ | $CH_3$ |
| 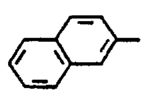 | 1 | H | CN | $CF_3$ | $CH_3$ |
| 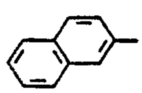 | 0 | $CH_3$ | CN | $CF_3$ | $CH_3$ |
| 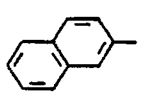 | 1 | $CH_3$ | CN | $CF_3$ | $CH_3$ |

Tabelle I - Forts.

| A | n | $R^1$ | $R^2$ | $R^4$ | $R^{4''}$ |
|---|---|---|---|---|---|
| 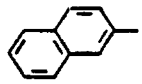 | 0 | $COCH_3$ | CN | $CF_3$ | $CH_3$ |
| 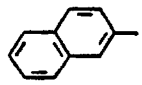 | 1 | $COCH_3$ | CN | $CF_3$ | $CH_3$ |
| 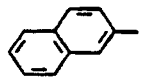 | 0 | H | H | $CF_3$ | $CF_3$ |
| 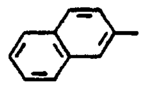 | 1 | H | H | $CF_3$ | $CF_3$ |
| 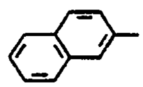 | 0 | $CH_3$ | H | $CF_3$ | $CF_3$ |
| 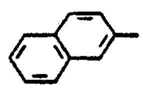 | 1 | $CH_3$ | H | $CF_3$ | $CF_3$ |
| 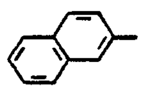 | 0 | $COCH_3$ | H | $CF_3$ | $CF_3$ |
| 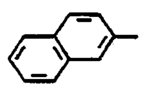 | 1 | $COCH_3$ | H | $CF_3$ | $CF_3$ |
| 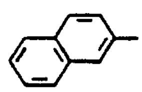 | 0 | H | Cl | $CF_3$ | $CF_3$ |
| 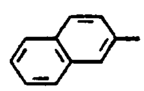 | 1 | H | Cl | $CF_3$ | $CF_3$ |
| 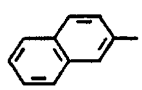 | 0 | $CH_3$ | Cl | $CF_3$ | $CF_3$ |
| 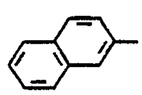 | 1 | $CH_3$ | Cl | $CF_3$ | $CF_3$ |

Tabelle I - Forts.

| A | n | $R^1$ | $R^2$ | $R^4$ | $R^{4''}$ |
|---|---|---|---|---|---|
| 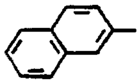 | 0 | $COCH_3$ | Cl | $CF_3$ | $CF_3$ |
| 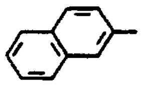 | 1 | $COCH_3$ | Cl | $CF_3$ | $CF_3$ |
| 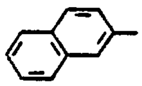 | 0 | H | CN | $CF_3$ | $CF_3$ |
| 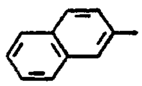 | 1 | H | CN | $CF_3$ | $CF_3$ |
| 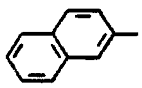 | 0 | $CH_3$ | CN | $CF_3$ | $CF_3$ |
| 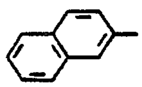 | 1 | $CH_3$ | CN | $CF_3$ | $CF_3$ |
| 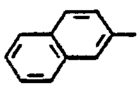 | 0 | $COCH_3$ | CN | $CF_3$ | $CF_3$ |
| 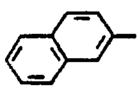 | 1 | $COCH_3$ | CN | $CF_3$ | $CF_3$ |

Tabelle II

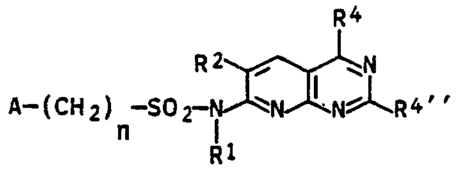

Ib

| A | n | $R^1$ | $R^2$ | $R^4$ | $R^{4''}$ |
|---|---|---|---|---|---|
| 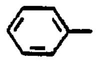 | 0 | H | H | $CH_3$ | $CH_3$ |
| 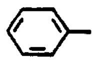 | 1 | H | H | $CH_3$ | $CH_3$ |
| 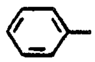 | 0 | $CH_3$ | H | $CH_3$ | $CH_3$ |
| 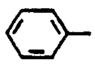 | 1 | $CH_3$ | H | $CH_3$ | $CH_3$ |
| 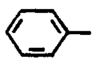 | 0 | $COCH_3$ | H | $CH_3$ | $CH_3$ |
| 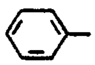 | 1 | $COCH_3$ | H | $CH_3$ | $CH_3$ |
| 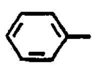 | 0 | H | Cl | $CH_3$ | $CH_3$ |
| 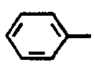 | 1 | H | Cl | $CH_3$ | $CH_3$ |
| 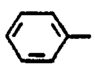 | 0 | $CH_3$ | Cl | $CH_3$ | $CH_3$ |
| 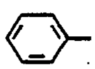 | 1 | $CH_3$ | Cl | $CH_3$ | $CH_3$ |
| 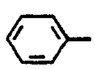 | 0 | $COCH_3$ | Cl | $CH_3$ | $CH_3$ |
| 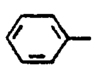 | 1 | $COCH_3$ | Cl | $CH_3$ | $CH_3$ |
| 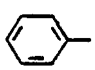 | 0 | H | CN | $CH_3$ | $CH_3$ |
| 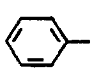 | 1 | H | CN | $CH_3$ | $CH_3$ |
| 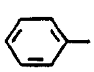 | 0 | $CH_3$ | CN | $CH_3$ | $CH_3$ |
| 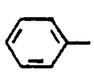 | 1 | $CH_3$ | CN | $CH_3$ | $CH_3$ |

Tabelle II - Forts.

| A | n | $R^1$ | $R^2$ | $R^4$ | $R^{4''}$ |
|---|---|---|---|---|---|
| 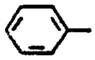 | 0 | $COCH_3$ | CN | $CH_3$ | $CH_3$ |
| 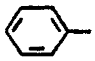 | 1 | $COCH_3$ | CN | $CH_3$ | $CH_3$ |
| 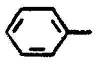 | 0 | H | H | $CH_3$ | Cl |
| 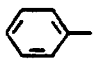 | 1 | H | H | $CH_3$ | Cl |
| 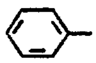 | 0 | $CH_3$ | H | $CH_3$ | Cl |
| 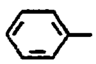 | 1 | $CH_3$ | H | $CH_3$ | Cl |
| 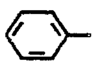 | 0 | $COCH_3$ | H | $CH_3$ | Cl |
| 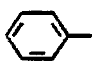 | 1 | $COCH_3$ | H | $CH_3$ | Cl |
| 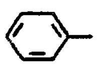 | 0 | H | Cl | $CH_3$ | Cl |
| 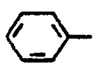 | 1 | H | Cl | $CH_3$ | Cl |
| 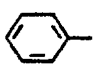 | 0 | $CH_3$ | Cl | $CH_3$ | Cl |
| 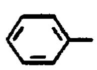 | 1 | $CH_3$ | Cl | $CH_3$ | Cl |
| 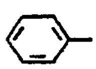 | 0 | $COCH_3$ | Cl | $CH_3$ | Cl |
| 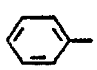 | 1 | $COCH_3$ | Cl | $CH_3$ | Cl |
| 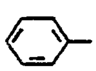 | 0 | H | CN | $CH_3$ | Cl |
| 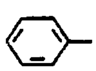 | 1 | H | CN | $CH_3$ | Cl |
| 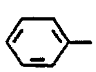 | 0 | $CH_3$ | CN | $CH_3$ | Cl |
| 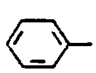 | 1 | $CH_3$ | CN | $CH_3$ | Cl |

Tabelle II - Forts.

| A | n | R1 | R2 | R4 | R4'' |
|---|---|---|---|---|---|
| 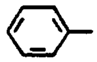 | 0 | $COCH_3$ | CN | $CH_3$ | Cl |
| 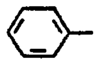 | 1 | $COCH_3$ | CN | $CH_3$ | Cl |
| 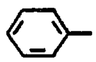 | 0 | H | H | $CH_3$ | $CF_3$ |
| 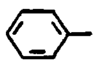 | 1 | H | H | $CH_3$ | $CF_3$ |
| 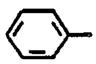 | 0 | $CH_3$ | H | $CH_3$ | $CF_3$ |
| 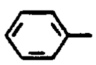 | 1 | $CH_3$ | H | $CH_3$ | $CF_3$ |
| 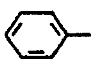 | 0 | $COCH_3$ | H | $CH_3$ | $CF_3$ |
| 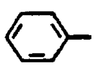 | 1 | $COCH_3$ | H | $CH_3$ | $CF_3$ |
| 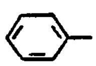 | 0 | H | Cl | $CH_3$ | $CF_3$ |
| 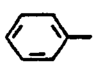 | 1 | H | Cl | $CH_3$ | $CF_3$ |
| 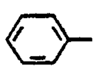 | 0 | $CH_3$ | Cl | $CH_3$ | $CF_3$ |
| 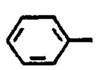 | 1 | $CH_3$ | Cl | $CH_3$ | $CF_3$ |
| 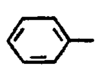 | 0 | $COCH_3$ | Cl | $CH_3$ | $CF_3$ |
| 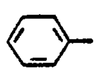 | 1 | $COCH_3$ | Cl | $CH_3$ | $CF_3$ |
| 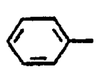 | 0 | H | CN | $CH_3$ | $CF_3$ |
| 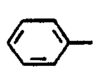 | 1 | H | CN | $CH_3$ | $CF_3$ |
| 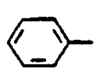 | 0 | $CH_3$ | CN | $CH_3$ | $CF_3$ |
| 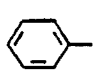 | 1 | $CH_3$ | CN | $CH_3$ | $CF_3$ |

Tabelle II - Forts.

| A | n | R1 | R2 | R4 | R4'' |
|---|---|---|---|---|---|
| 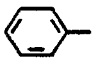 | 0 | $COCH_3$ | CN | $CH_3$ | $CF_3$ |
| 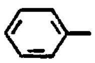 | 1 | $COCH_3$ | CN | $CH_3$ | $CF_3$ |
| 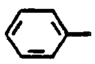 | 0 | H | H | $CH_3$ | $OCH_3$ |
| 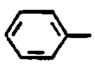 | 1 | H | H | $CH_3$ | $OCH_3$ |
| 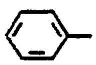 | 0 | $CH_3$ | H | $CH_3$ | $OCH_3$ |
| 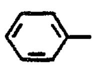 | 1 | $CH_3$ | H | $CH_3$ | $OCH_3$ |
| 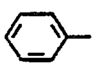 | 0 | $COCH_3$ | H | $CH_3$ | $OCH_3$ |
| 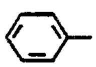 | 1 | $COCH_3$ | H | $CH_3$ | $OCH_3$ |
| 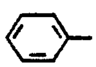 | 0 | H | Cl | $CH_3$ | $OCH_3$ |
| 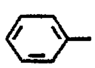 | 1 | H | Cl | $CH_3$ | $OCH_3$ |
| 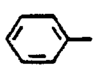 | 0 | $CH_3$ | Cl | $CH_3$ | $OCH_3$ |
| 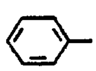 | 1 | $CH_3$ | Cl | $CH_3$ | $OCH_3$ |
| 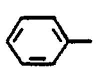 | 0 | $COCH_3$ | Cl | $CH_3$ | $OCH_3$ |
| 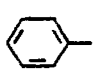 | 1 | $COCH_3$ | Cl | $CH_3$ | $OCH_3$ |
| 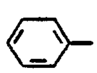 | 0 | H | CN | $CH_3$ | $OCH_3$ |
| 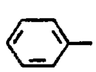 | 1 | H | CN | $CH_3$ | $OCH_3$ |
| 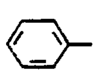 | 0 | $CH_3$ | CN | $CH_3$ | $OCH_3$ |
| 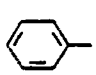 | 1 | $CH_3$ | CN | $CH_3$ | $OCH_3$ |

Tabelle II - Forts.

| A | n | $R^1$ | $R^2$ | $R^4$ | $R^{4''}$ |
|---|---|---|---|---|---|
| 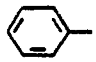 | 0 | $COCH_3$ | CN | $CH_3$ | $OCH_3$ |
| 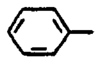 | 1 | $COCH_3$ | CN | $CH_3$ | $OCH_3$ |
| 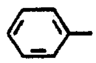 | 0 | H | H | $CF_3$ | $CH_3$ |
| 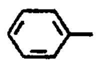 | 1 | H | H | $CF_3$ | $CH_3$ |
| 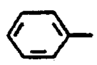 | 0 | $CH_3$ | H | $CF_3$ | $CH_3$ |
| 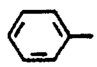 | 1 | $CH_3$ | H | $CF_3$ | $CH_3$ |
| 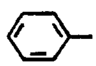 | 0 | $COCH_3$ | H | $CF_3$ | $CH_3$ |
| 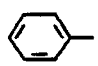 | 1 | $COCH_3$ | H | $CF_3$ | $CH_3$ |
| 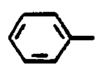 | 0 | H | Cl | $CF_3$ | $CH_3$ |
| 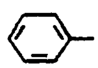 | 1 | H | Cl | $CF_3$ | $CH_3$ |
| 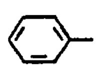 | 0 | $CH_3$ | Cl | $CF_3$ | $CH_3$ |
| 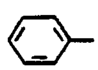 | 1 | $CH_3$ | Cl | $CF_3$ | $CH_3$ |
| 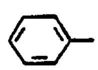 | 0 | $COCH_3$ | Cl | $CF_3$ | $CH_3$ |
| 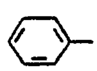 | 1 | $COCH_3$ | Cl | $CF_3$ | $CH_3$ |
| 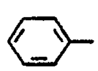 | 0 | H | CN | $CF_3$ | $CH_3$ |
| 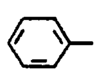 | 1 | H | CN | $CF_3$ | $CH_3$ |
| 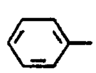 | 0 | $CH_3$ | CN | $CF_3$ | $CH_3$ |
| 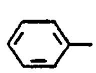 | 1 | $CH_3$ | CN | $CF_3$ | $CH_3$ |

Tabelle II - Forts.

| A | n | $R^1$ | $R^2$ | $R^4$ | $R^{4''}$ |
|---|---|---|---|---|---|
| 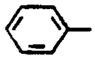 | 0 | $COCH_3$ | CN | $CF_3$ | $CH_3$ |
| 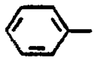 | 1 | $COCH_3$ | CN | $CF_3$ | $CH_3$ |
| 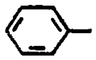 | 0 | H | H | $CF_3$ | $CF_3$ |
| 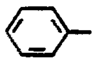 | 1 | H | H | $CF_3$ | $CF_3$ |
| 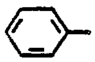 | 0 | $CH_3$ | H | $CF_3$ | $CF_3$ |
| 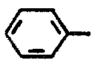 | 1 | $CH_3$ | H | $CF_3$ | $CF_3$ |
| 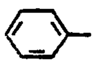 | 0 | $COCH_3$ | H | $CF_3$ | $CF_3$ |
| 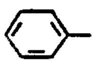 | 1 | $COCH_3$ | H | $CF_3$ | $CF_3$ |
| 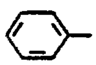 | 0 | H | Cl | $CF_3$ | $CF_3$ |
| 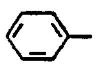 | 1 | H | Cl | $CF_3$ | $CF_3$ |
| 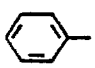 | 0 | $CH_3$ | Cl | $CF_3$ | $CF_3$ |
| 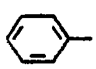 | 1 | $CH_3$ | Cl | $CF_3$ | $CF_3$ |
| 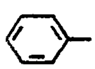 | 0 | $COCH_3$ | Cl | $CF_3$ | $CF_3$ |
| 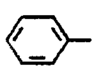 | 1 | $COCH_3$ | Cl | $CF_3$ | $CF_3$ |
| 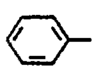 | 0 | H | CN | $CF_3$ | $CF_3$ |
| 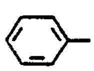 | 1 | H | CN | $CF_3$ | $CF_3$ |
| 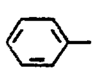 | 0 | $CH_3$ | CN | $CF_3$ | $CF_3$ |
| 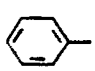 | 1 | $CH_3$ | CN | $CF_3$ | $CF_3$ |

Tabelle II - Forts.

| A | n | $R^1$ | $R^2$ | $R^4$ | $R^{4''}$ |
|---|---|---|---|---|---|
| 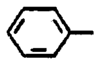 | 0 | $COCH_3$ | CN | $CF_3$ | $CF_3$ |
| 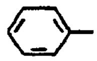 | 0 | $COCH_3$ | CN | $CF_3$ | $CF_3$ |
| 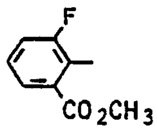 | 0 | H | H | $CH_3$ | $CH_3$ |
| 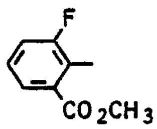 | 1 | H | H | $CH_3$ | $CH_3$ |
| 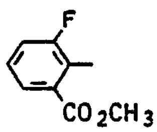 | 0 | $CH_3$ | H | $CH_3$ | $CH_3$ |
| 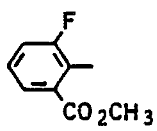 | 1 | $CH_3$ | H | $CH_3$ | $CH_3$ |
| 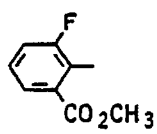 | 0 | $COCH_3$ | H | $CH_3$ | $CH_3$ |
| 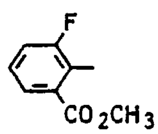 | 1 | $COCH_3$ | H | $CH_3$ | $CH_3$ |
| 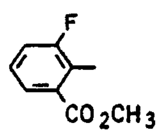 | 0 | H | Cl | $CH_3$ | $CH_3$ |
| 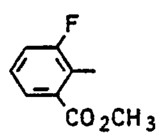 | 1 | H | Cl | $CH_3$ | $CH_3$ |
| 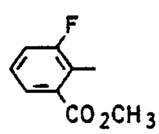 | 0 | $CH_3$ | Cl | $CH_3$ | $CH_3$ |
| 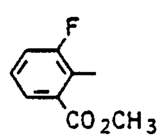 | 1 | $CH_3$ | Cl | $CH_3$ | $CH_3$ |

Tabelle II - Forts.

| A | n | $R^1$ | $R^2$ | $R^4$ | $R^{4''}$ |
|---|---|---|---|---|---|
| 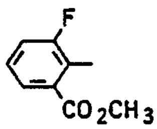 | 0 | $COCH_3$ | Cl | $CH_3$ | $CH_3$ |
| 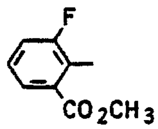 | 1 | $COCH_3$ | Cl | $CH_3$ | $CH_3$ |
| 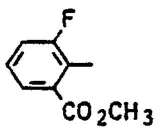 | 0 | H | CN | $CH_3$ | $CH_3$ |
| 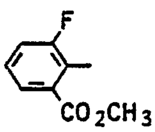 | 1 | H | CN | $CH_3$ | $CH_3$ |
| 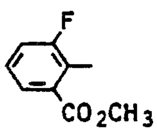 | 0 | $CH_3$ | CN | $CH_3$ | $CH_3$ |
| 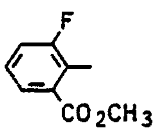 | 1 | $CH_3$ | CN | $CH_3$ | $CH_3$ |
| 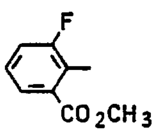 | 0 | $COCH_3$ | CN | $CH_3$ | $CH_3$ |
| 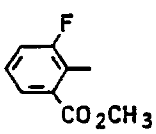 | 1 | $COCH_3$ | CN | $CH_3$ | $CH_3$ |
| 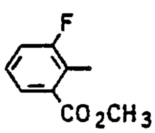 | 0 | H | H | $CH_3$ | Cl |
| 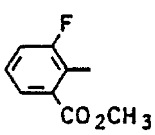 | 1 | H | H | $CH_3$ | Cl |
| 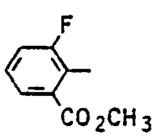 | 0 | $CH_3$ | H | $CH_3$ | Cl |
| 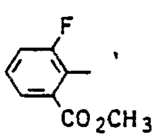 | 1 | $CH_3$ | H | $CH_3$ | Cl |

Tabelle II - Forts.

| A | n | $R^1$ | $R^2$ | $R^4$ | $R^{4''}$ |
|---|---|---|---|---|---|
| 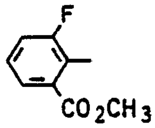 | 0 | $COCH_3$ | H | $CH_3$ | Cl |
| 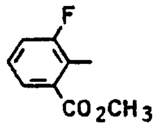 | 1 | $COCH_3$ | H | $CH_3$ | Cl |
| 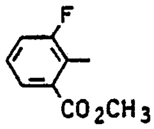 | 0 | H | Cl | $CH_3$ | Cl |
| 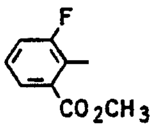 | 1 | H | Cl | $CH_3$ | Cl |
| 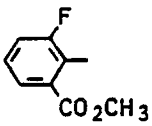 | 0 | $CH_3$ | Cl | $CH_3$ | Cl |
| 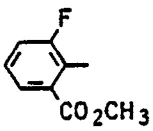 | 1 | $CH_3$ | Cl | $CH_3$ | Cl |
| 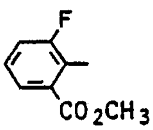 | 0 | $COCH_3$ | Cl | $CH_3$ | Cl |
| 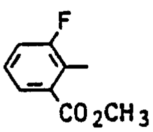 | 1 | $COCH_3$ | Cl | $CH_3$ | Cl |
| 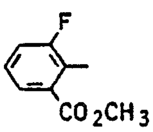 | 0 | H | CN | $CH_3$ | Cl |
| 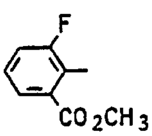 | 1 | H | CN | $CH_3$ | Cl |
| 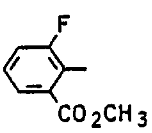 | 0 | $CH_3$ | CN | $CH_3$ | Cl |
| 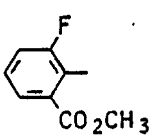 | 1 | $CH_3$ | CN | $CH_3$ | Cl |

Tabelle II - Forts.

| A | n | $R^1$ | $R^2$ | $R^4$ | $R^{4''}$ |
|---|---|---|---|---|---|
| 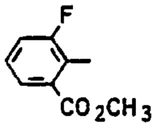 | 0 | $COCH_3$ | CN | $CH_3$ | Cl |
| 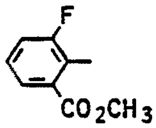 | 1 | $COCH_3$ | CN | $CH_3$ | Cl |
| 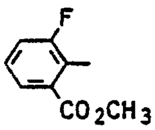 | 0 | H | H | $CH_3$ | $CF_3$ |
| 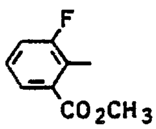 | 1 | H | H | $CH_3$ | $CF_3$ |
| 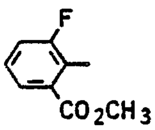 | 0 | $CH_3$ | H | $CH_3$ | $CF_3$ |
| 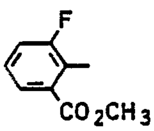 | 1 | $CH_3$ | H | $CH_3$ | $CF_3$ |
| 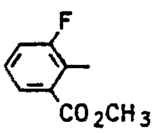 | 0 | $COCH_3$ | H | $CH_3$ | $CF_3$ |
| 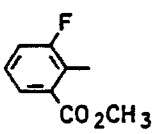 | 1 | $COCH_3$ | H | $CH_3$ | $CF_3$ |
| 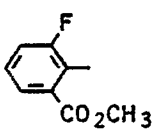 | 0 | H | Cl | $CH_3$ | $CF_3$ |
| 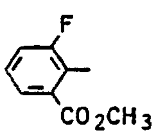 | 1 | H | Cl | $CH_3$ | $CF_3$ |
| 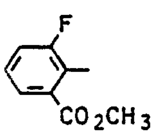 | 0 | $CH_3$ | Cl | $CH_3$ | $CF_3$ |
| 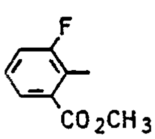 | 1 | $CH_3$ | Cl | $CH_3$ | $CF_3$ |

Tabelle II - Forts.

| A | n | $R^1$ | $R^2$ | $R^4$ | $R^{4''}$ |
|---|---|---|---|---|---|
| 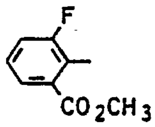 | 0 | $COCH_3$ | Cl | $CH_3$ | $CF_3$ |
| 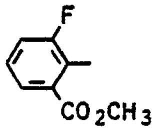 | 1 | $COCH_3$ | Cl | $CH_3$ | $CF_3$ |
| 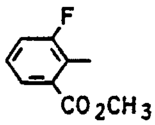 | 0 | H | CN | $CH_3$ | $CF_3$ |
| 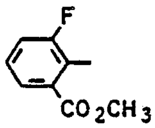 | 1 | H | CN | $CH_3$ | $CF_3$ |
| 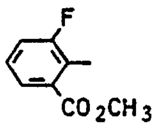 | 0 | $CH_3$ | CN | $CH_3$ | $CF_3$ |
| 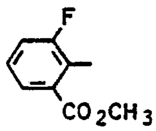 | 1 | $CH_3$ | CN | $CH_3$ | $CF_3$ |
| 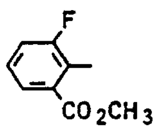 | 0 | $COCH_3$ | CN | $CH_3$ | $CF_3$ |
| 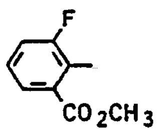 | 1 | $COCH_3$ | CN | $CH_3$ | $CF_3$ |
| 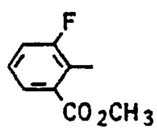 | 0 | H | H | $CH_3$ | $OCH_3$ |
| 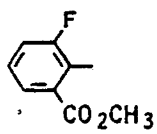 | 1 | H | H | $CH_3$ | $OCH_3$ |
| 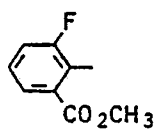 | 0 | $CH_3$ | H | $CH_3$ | $OCH_3$ |
| 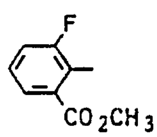 | 1 | $CH_3$ | H | $CH_3$ | $OCH_3$ |

Tabelle II - Forts.

| A | n | $R^1$ | $R^2$ | $R^4$ | $R^{4''}$ |
|---|---|---|---|---|---|
| 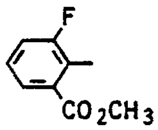 | 0 | $COCH_3$ | H | $CH_3$ | $OCH_3$ |
| 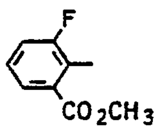 | 1 | $COCH_3$ | H | $CH_3$ | $OCH_3$ |
| 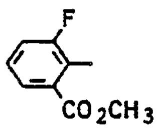 | 0 | H | Cl | $CH_3$ | $OCH_3$ |
| 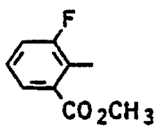 | 1 | H | Cl | $CH_3$ | $OCH_3$ |
| 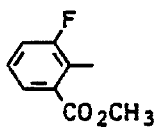 | 0 | $CH_3$ | Cl | $CH_3$ | $OCH_3$ |
| 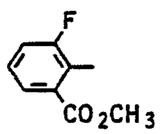 | 1 | $CH_3$ | Cl | $CH_3$ | $OCH_3$ |
| 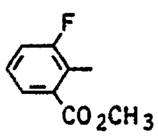 | 0 | $COCH_3$ | Cl | $CH_3$ | $OCH_3$ |
| 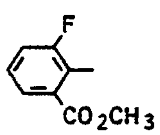 | 1 | $COCH_3$ | Cl | $CH_3$ | $OCH_3$ |
| 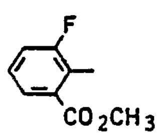 | 0 | H | CN | $CH_3$ | $OCH_3$ |
| 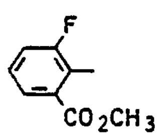 | 1 | H | CN | $CH_3$ | $OCH_3$ |
| 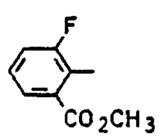 | 0 | $CH_3$ | CN | $CH_3$ | $OCH_3$ |
| 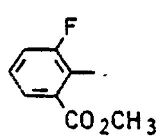 | 1 | $CH_3$ | CN | $CH_3$ | $OCH_3$ |

Tabelle II - Forts.

| A | n | $R^1$ | $R^2$ | $R^4$ | $R^{4''}$ |
|---|---|---|---|---|---|
| 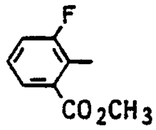 | 0 | $COCH_3$ | CN | $CH_3$ | $OCH_3$ |
| 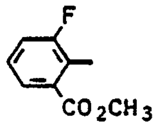 | 1 | $COCH_3$ | CN | $CH_3$ | $OCH_3$ |
| 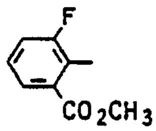 | 0 | H | H | $CF_3$ | $CH_3$ |
| 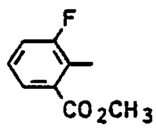 | 1 | H | H | $CF_3$ | $CH_3$ |
| 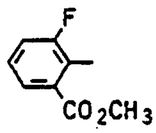 | 0 | $CH_3$ | H | $CF_3$ | $CH_3$ |
| 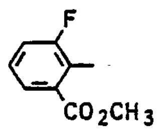 | 1 | $CH_3$ | H | $CF_3$ | $CH_3$ |
| 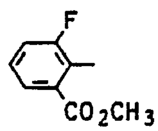 | 0 | $COCH_3$ | H | $CF_3$ | $CH_3$ |
| 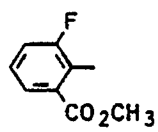 | 1 | $COCH_3$ | H | $CF_3$ | $CH_3$ |
| 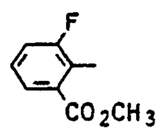 | 0 | H | Cl | $CF_3$ | $CH_3$ |
| 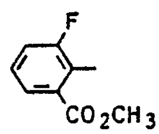 | 1 | H | Cl | $CF_3$ | $CH_3$ |
| 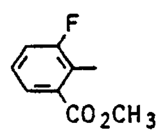 | 0 | $CH_3$ | Cl | $CF_3$ | $CH_3$ |
| 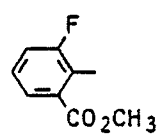 | 1 | $CH_3$ | Cl | $CF_3$ | $CH_3$ |

Tabelle II - Forts.

| A | n | $R^1$ | $R^2$ | $R^4$ | $R^{4''}$ |
|---|---|---|---|---|---|
| 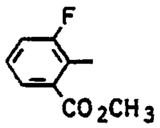 | 0 | $COCH_3$ | Cl | $CF_3$ | $CH_3$ |
| 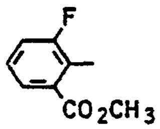 | 1 | $COCH_3$ | Cl | $CF_3$ | $CH_3$ |
| 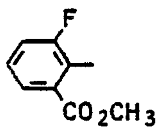 | 0 | H | CN | $CF_3$ | $CH_3$ |
| 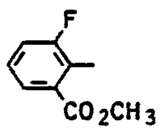 | 1 | H | CN | $CF_3$ | $CH_3$ |
| 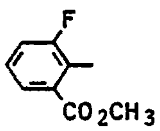 | 0 | $CH_3$ | CN | $CF_3$ | $CH_3$ |
| 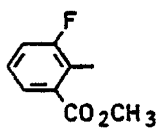 | 1 | $CH_3$ | CN | $CF_3$ | $CH_3$ |
| 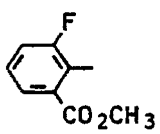 | 0 | $COCH_3$ | CN | $CF_3$ | $CH_3$ |
| 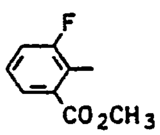 | 1 | $COCH_3$ | CN | $CF_3$ | $CH_3$ |
| 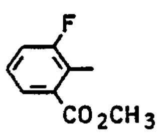 | 0 | H | H | $CF_3$ | $CF_3$ |
| 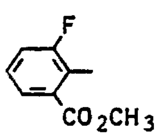 | 1 | H | H | $CF_3$ | $CF_3$ |
| 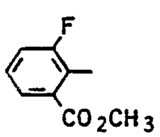 | 0 | $CH_3$ | H | $CF_3$ | $CF_3$ |
| 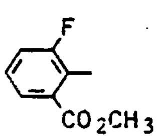 | 1 | $CH_3$ | H | $CF_3$ | $CF_3$ |

Tabelle II - Forts.

| A | n | R1 | R2 | R4 | R4'' |
|---|---|---|---|---|---|
| 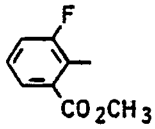 | 0 | $COCH_3$ | H | $CF_3$ | $CF_3$ |
| 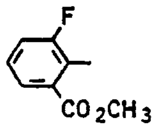 | 1 | $COCH_3$ | H | $CF_3$ | $CF_3$ |
| 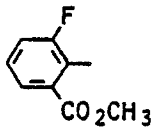 | 0 | H | Cl | $CF_3$ | $CF_3$ |
| 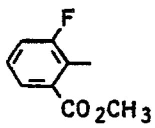 | 1 | H | Cl | $CF_3$ | $CF_3$ |
| 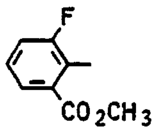 | 0 | $CH_3$ | Cl | $CF_3$ | $CF_3$ |
| 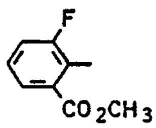 | 1 | $CH_3$ | Cl | $CF_3$ | $CF_3$ |
| 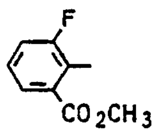 | 0 | $COCH_3$ | Cl | $CF_3$ | $CF_3$ |
| 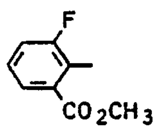 | 1 | $COCH_3$ | Cl | $CF_3$ | $CF_3$ |
| 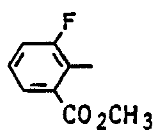 | 0 | H | CN | $CF_3$ | $CF_3$ |
| 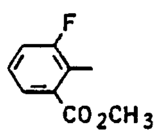 | 1 | H | CN | $CF_3$ | $CF_3$ |
| 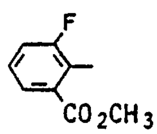 | 0 | $CH_3$ | CN | $CF_3$ | $CF_3$ |
| 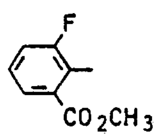 | 1 | $CH_3$ | CN | $CF_3$ | $CF_3$ |

Tabelle II - Forts.

| A | n | $R^1$ | $R^2$ | $R^4$ | $R^{4''}$ |
|---|---|---|---|---|---|
| 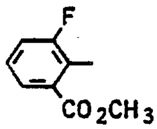 | 0 | $COCH_3$ | CN | $CF_3$ | $CF_3$ |
| 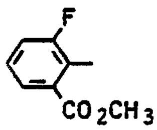 | 1 | $COCH_3$ | CN | $CF_3$ | $CF_3$ |
| 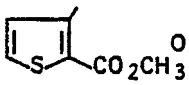 | 0 | H | H | $CH_3$ | $CH_3$ |
| 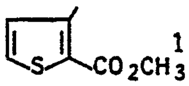 | 1 | H | H | $CH_3$ | $CH_3$ |
| 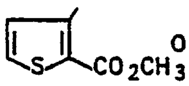 | 0 | $CH_3$ | H | $CH_3$ | $CH_3$ |
| 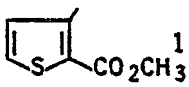 | 1 | $CH_3$ | H | $CH_3$ | $CH_3$ |
| 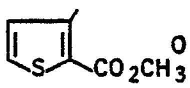 | 0 | $COCH_3$ | H | $CH_3$ | $CH_3$ |
| 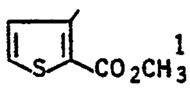 | 1 | $COCH_3$ | H | $CH_3$ | $CH_3$ |
| 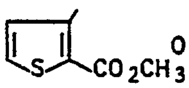 | 0 | H | Cl | $CH_3$ | $CH_3$ |
| 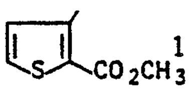 | 1 | H | Cl | $CH_3$ | $CH_3$ |
| 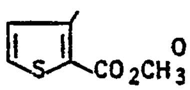 | 0 | $CH_3$ | Cl | $CH_3$ | $CH_3$ |
| 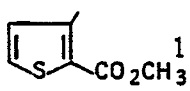 | 1 | $CH_3$ | Cl | $CH_3$ | $CH_3$ |
| 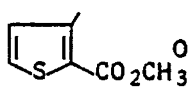 | 0 | $COCH_3$ | Cl | $CH_3$ | $CH_3$ |
| 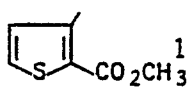 | 1 | $COCH_3$ | Cl | $CH_3$ | $CH_3$ |
| 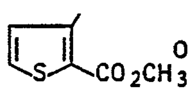 | 0 | H | CN | $CH_3$ | $CH_3$ |
| 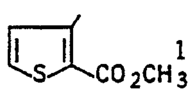 | 1 | H | CN | $CH_3$ | $CH_3$ |
| 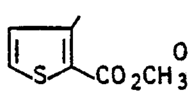 | 0 | $CH_3$ | CN | $CH_3$ | $CH_3$ |

Tabelle II - Forts.

| A | n | $R^1$ | $R^2$ | $R^4$ | $R^{4''}$ |
|---|---|---|---|---|---|
| 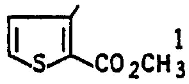 | 1 | $CH_3$ | CN | $CH_3$ | $CH_3$ |
| 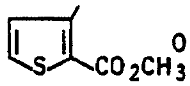 | 0 | $COCH_3$ | CN | $CH_3$ | $CH_3$ |
| 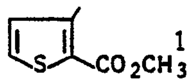 | 1 | $COCH_3$ | CN | $CH_3$ | $CH_3$ |
| 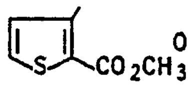 | 0 | H | H | $CH_3$ | Cl |
| 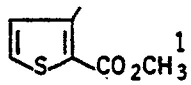 | 1 | H | H | $CH_3$ | Cl |
| 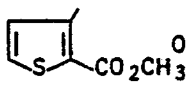 | 0 | $CH_3$ | H | $CH_3$ | Cl |
| 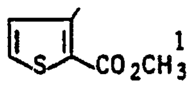 | 1 | $CH_3$ | H | $CH_3$ | Cl |
| 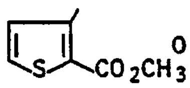 | 0 | $COCH_3$ | H | $CH_3$ | Cl |
| 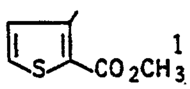 | 1 | $COCH_3$ | H | $CH_3$ | Cl |
| 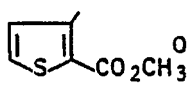 | 0 | H | Cl | $CH_3$ | Cl |
| 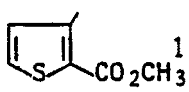 | 1 | H | Cl. | $CH_3$ | Cl |
| 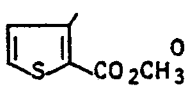 | 0 | $CH_3$ | Cl | $CH_3$ | Cl |
| 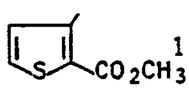 | 1 | $CH_3$ | Cl | $CH_3$ | Cl |
| 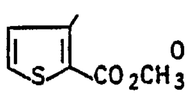 | 0 | $COCH_3$ | Cl | $CH_3$ | Cl |
| 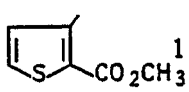 | 1 | $COCH_3$ | Cl | $CH_3$ | Cl |
| 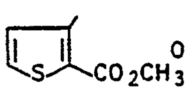 | 0 | H | CN | $CH_3$ | Cl |
| 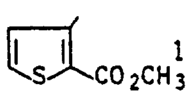 | 1 | H | CN | $CH_3$ | Cl |

Tabelle II - Forts.

| A | n | $R^1$ | $R^2$ | $R^4$ | $R^{4''}$ |
|---|---|---|---|---|---|
| 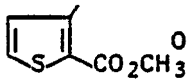 | 0 | $CH_3$ | CN | $CH_3$ | Cl |
| 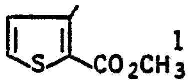 | 1 | $CH_3$ | CN | $CH_3$ | Cl |
| 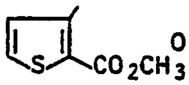 | 0 | $COCH_3$ | CN | $CH_3$ | Cl |
| 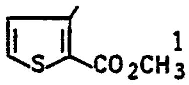 | 1 | $COCH_3$ | CN | $CH_3$ | Cl |
| 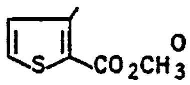 | 0 | H | H | $CH_3$ | $CF_3$ |
| 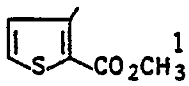 | 1 | H | H | $CH_3$ | $CF_3$ |
| 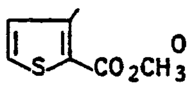 | 0 | $CH_3$ | H | $CH_3$ | $CF_3$ |
| 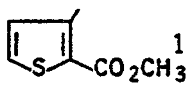 | 1 | $CH_3$ | H | $CH_3$ | $CF_3$ |
| 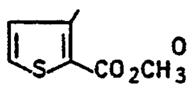 | 0 | $COCH_3$ | H | $CH_3$ | $CF_3$ |
| 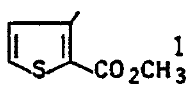 | 1 | $COCH_3$ | H | $CH_3$ | $CF_3$ |
| 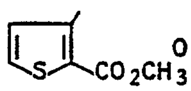 | 0 | H | Cl | $CH_3$ | $CF_3$ |
| 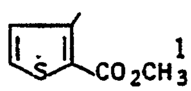 | 1 | H | Cl | $CH_3$ | $CF_3$ |
| 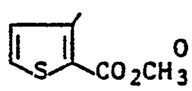 | 0 | $CH_3$ | Cl | $CH_3$ | $CF_3$ |
| 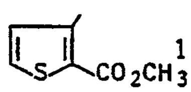 | 1 | $CH_3$ | Cl | $CH_3$ | $CF_3$ |
| 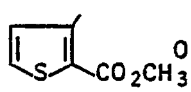 | 0 | $COCH_3$ | Cl | $CH_3$ | $CF_3$ |
| 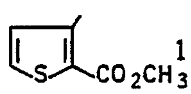 | 1 | $COCH_3$ | Cl | $CH_3$ | $CF_3$ |

Tabelle II - Forts.

| A | n | $R^1$ | $R^2$ | $R^4$ | $R^{4''}$ |
| --- | --- | --- | --- | --- | --- |
| 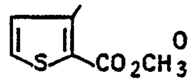 | 0 | H | CN | $CH_3$ | $CF_3$ |
| 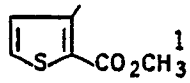 | 1 | H | CN | $CH_3$ | $CF_3$ |
| 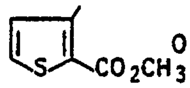 | 0 | $CH_3$ | CN | $CH_3$ | $CF_3$ |
| 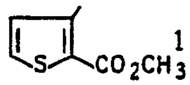 | 1 | $CH_3$ | CN | $CH_3$ | $CF_3$ |
| 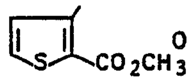 | 0 | $COCH_3$ | CN | $CH_3$ | $CF_3$ |
| 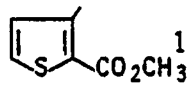 | 1 | $COCH_3$ | CN | $CH_3$ | $CF_3$ |
| 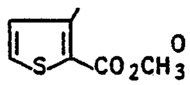 | 0 | H | H | $CH_3$ | $OCH_3$ |
| 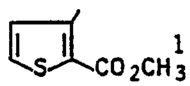 | 1 | H | H | $CH_3$ | $OCH_3$ |
| 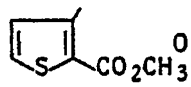 | 0 | $CH_3$ | H | $CH_3$ | $OCH_3$ |
| 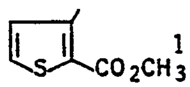 | 1 | $CH_3$ | H | $CH_3$ | $OCH_3$ |
| 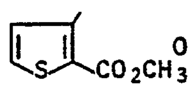 | 0 | $COCH_3$ | H | $CH_3$ | $OCH_3$ |
| 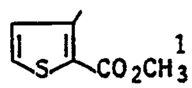 | 1 | $COCH_3$ | H | $CH_3$ | $OCH_3$ |
| 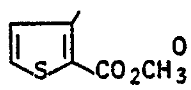 | 0 | H | Cl | $CH_3$ | $OCH_3$ |
| 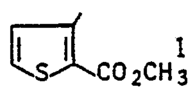 | 1 | H | Cl | $CH_3$ | $OCH_3$ |
| 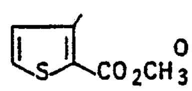 | 0 | $CH_3$ | Cl | $CH_3$ | $OCH_3$ |
| 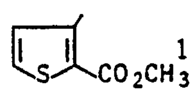 | 1 | $CH_3$ | Cl | $CH_3$ | $OCH_3$ |

Tabelle II - Forts.

| A | n | $R^1$ | $R^2$ | $R^4$ | $R^{4''}$ |
|---|---|---|---|---|---|
| 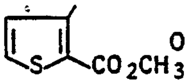 | 0 | $COCH_3$ | Cl | $CH_3$ | $OCH_3$ |
| 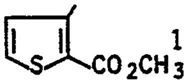 | 1 | $OCH_3$ | Cl | $CH_3$ | $OCH_3$ |
| 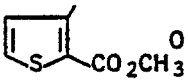 | 0 | H | CN | $CH_3$ | $OCH_3$ |
| 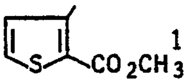 | 1 | H | CN | $CH_3$ | $OCH_3$ |
| 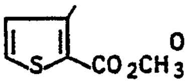 | 0 | $CH_3$ | CN | $CH_3$ | $OCH_3$ |
| 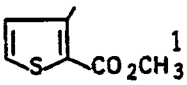 | 1 | $CH_3$ | CN | $CH_3$ | $OCH_3$ |
| 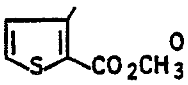 | 0 | $COCH_3$ | CN | $CH_3$ | $OCH_3$ |
| 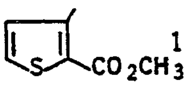 | 1 | $COCH_3$ | CN | $CH_3$ | $OCH_3$ |
| 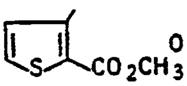 | 0 | H | H | $CF_3$ | $CH_3$ |
| 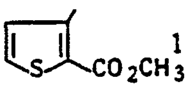 | 1 | H | H | $CF_3$ | $CH_3$ |
| 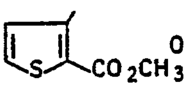 | 0 | $CH_3$ | H | $CF_3$ | $CH_3$ |
| 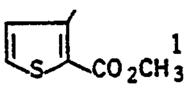 | 1 | $CH_3$ | H | $CF_3$ | $CH_3$ |
| 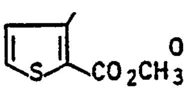 | 0 | $COCH_3$ | H | $CF_3$ | $CH_3$ |
| 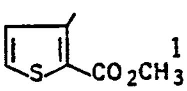 | 1 | $COCH_3$ | H | $CF_3$ | $CH_3$ |
| 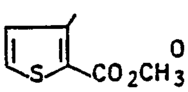 | 0 | H | Cl | $CF_3$ | $CH_3$ |
| 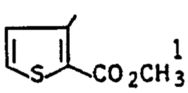 | 1 | H | Cl | $CF_3$ | $CH_3$ |

Tabelle II - Forts.

| A | n | $R^1$ | $R^2$ | $R^4$ | $R^{4''}$ |
|---|---|---|---|---|---|
| 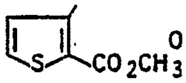 | 0 | $CH_3$ | Cl | $CF_3$ | $CH_3$ |
| 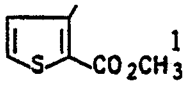 | 1 | $CH_3$ | Cl | $CF_3$ | $CH_3$ |
| 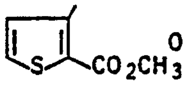 | 0 | $COCH_3$ | Cl | $CF_3$ | $CH_3$ |
| 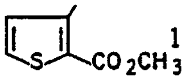 | 1 | $COCH_3$ | Cl | $CF_3$ | $CH_3$ |
| 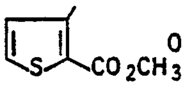 | 0 | H | CN | $CF_3$ | $CH_3$ |
| 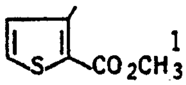 | 1 | H | CN | $CF_3$ | $CH_3$ |
| 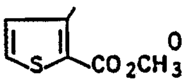 | 0 | $CH_3$ | CN | $CF_3$ | $CH_3$ |
| 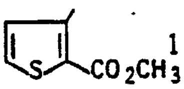 | 1 | $CH_3$ | CN | $CF_3$ | $CH_3$ |
| 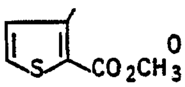 | 0 | $COCH_3$ | CN | $CF_3$ | $CH_3$ |
| 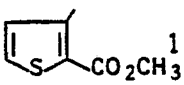 | 1 | $COCH_3$ | CN | $CF_3$ | $CH_3$ |
| 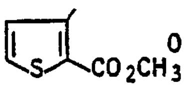 | 0 | H | H | $CF_3$ | $CF_3$ |
| 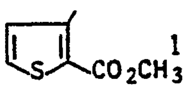 | 1 | H | H | $CF_3$ | $CF_3$ |
| 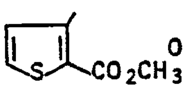 | 0 | $CH_3$ | H | $CF_3$ | $CF_3$ |
| 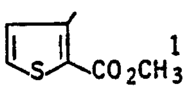 | 1 | $CH_3$ | H | $CF_3$ | $CF_3$ |
| 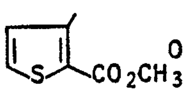 | 0 | $COCH_3$ | H | $CF_3$ | $CF_3$ |
| 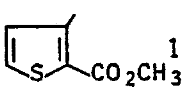 | 1 | $COCH_3$ | H | $CF_3$ | $CF_3$ |

Tabelle II - Forts.

| A | n | $R^1$ | $R^2$ | $R^4$ | $R^{4''}$ |
|---|---|---|---|---|---|
| 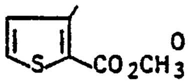 | 0 | H | Cl | $CF_3$ | $CF_3$ |
| 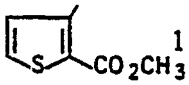 | 1 | H | Cl | $CF_3$ | $CF_3$ |
| 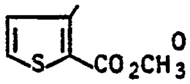 | 0 | $CH_3$ | Cl | $CF_3$ | $CF_3$ |
| 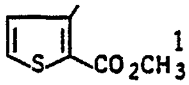 | 1 | $CH_3$ | Cl | $CF_3$ | $CF_3$ |
| 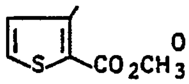 | 0 | $COCH_3$ | Cl | $CF_3$ | $CF_3$ |
| 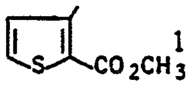 | 1 | $COCH_3$ | Cl | $CF_3$ | $CF_3$ |
| 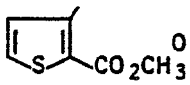 | 0 | H | CN | $CF_3$ | $CF_3$ |
| 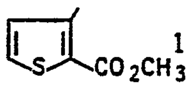 | 1 | H | CN | $CF_3$ | $CF_3$ |
| 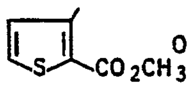 | 0 | $CH_3$ | CN | $CF_3$ | $CF_3$ |
| 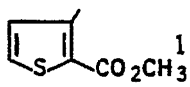 | 1 | $CH_3$ | CN | $CF_3$ | $CF_3$ |
| 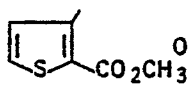 | 0 | $COCH_3$ | CN | $CF_3$ | $CF_3$ |
| 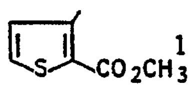 | 1 | $COCH_3$ | CN | $CF_3$ | $CF_3$ |
| 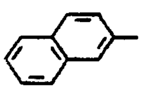 | 0 | H | H | $CH_3$ | $CH_3$ |
| 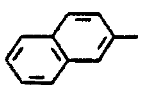 | 1 | H | H | $CH_3$ | $CH_3$ |
| 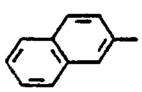 | 0 | $CH_3$ | H | $CH_3$ | $CH_3$ |
| 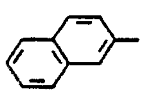 | 1 | $CH_3$ | H | $CH_3$ | $CH_3$ |

Tabelle II - Forts.

| A | n | $R^1$ | $R^2$ | $R^4$ | $R^{4''}$ |
|---|---|---|---|---|---|
| 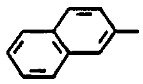 | 0 | $COCH_3$ | H | $CH_3$ | $CH_3$ |
| 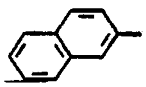 | 1 | $COCH_3$ | H | $CH_3$ | $CH_3$ |
| 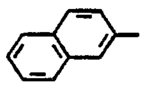 | 0 | H | Cl | $CH_3$ | $CH_3$ |
| 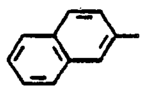 | 1 | H | Cl | $CH_3$ | $CH_3$ |
| 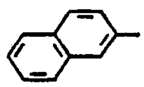 | 0 | $CH_3$ | Cl | $CH_3$ | $CH_3$ |
| 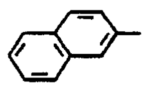 | 1 | $CH_3$ | Cl | $CH_3$ | $CH_3$ |
| 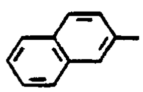 | 0 | $COCH_3$ | Cl | $CH_3$ | $CH_3$ |
| 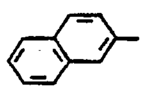 | 1 | $COCH_3$ | Cl | $CH_3$ | $CH_3$ |
| 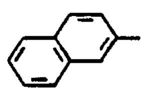 | 0 | H | CN | $CH_3$ | $CH_3$ |
| 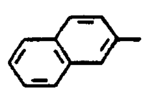 | 1 | H | CN | $CH_3$ | $CH_3$ |
| 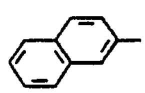 | 0 | $CH_3$ | CN | $CH_3$ | $CH_3$ |
| 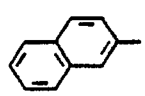 | 1 | $CH_3$ | CN | $CH_3$ | $CH_3$ |
| 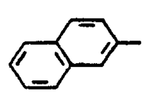 | 0 | $COCH_3$ | CN | $CH_3$ | $CH_3$ |
| 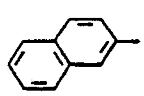 | 1 | $COCH_3$ | CN | $CH_3$ | $CH_3$ |

Tabelle II - Forts.

| A | n | $R^1$ | $R^2$ | $R^4$ | $R^{4''}$ |
|---|---|---|---|---|---|
| 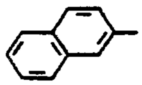 | 0 | H | H | $CH_3$ | Cl |
| 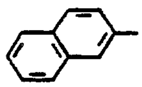 | 1 | H | H | $CH_3$ | Cl |
| 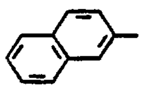 | 0 | $CH_3$ | H | $CH_3$ | Cl |
| 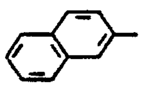 | 1 | $CH_3$ | H | $CH_3$ | Cl |
| 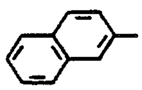 | 0 | $COCH_3$ | H | $CH_3$ | Cl |
| 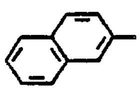 | 1 | $COCH_3$ | H | $CH_3$ | Cl |
| 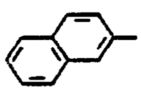 | 0 | H | Cl | $CH_3$ | Cl |
| 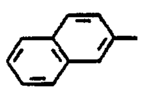 | 1 | H | Cl | $CH_3$ | Cl |
| 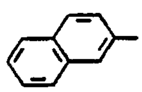 | 0 | $CH_3$ | Cl | $CH_3$ | Cl |
| 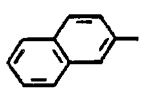 | 1 | $CH_3$ | Cl | $CH_3$ | Cl |
| 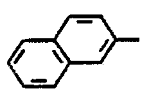 | 0 | $COCH_3$ | Cl | $CH_3$ | Cl |
| 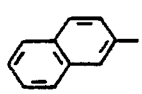 | 0 | $COCH_3$ | Cl | $CH_3$ | Cl |
| 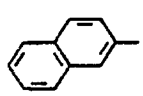 | 0 | H | CN | $CH_3$ | Cl |
| 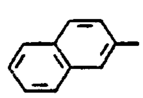 | 1 | H | CN | $CH_3$ | Cl |

Tabelle II - Forts.

| A | n | $R^1$ | $R^2$ | $R^4$ | $R^{4''}$ |
|---|---|---|---|---|---|
| 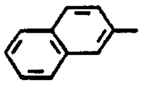 | 0 | $CH_3$ | CN | $CH_3$ | Cl |
| 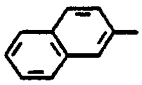 | 1 | $CH_3$ | CN | $CH_3$ | Cl |
| 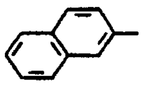 | 0 | $COCH_3$ | CN | $CH_3$ | Cl |
| 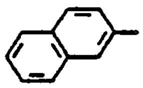 | 1 | $COCH_3$ | CN | $CH_3$ | Cl |
| 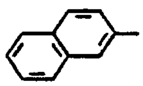 | 0 | H | H | $CH_3$ | $CF_3$ |
| 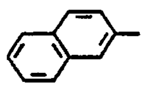 | 1 | H | H | $CH_3$ | $CF_3$ |
| 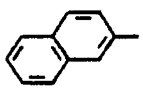 | 0 | $CH_3$ | H | $CH_3$ | $CF_3$ |
| 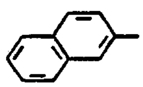 | 1 | $CH_3$ | H | $CH_3$ | $CF_3$ |
| 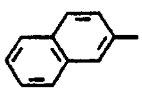 | 0 | $COCH_3$ | H | $CH_3$ | $CF_3$ |
| 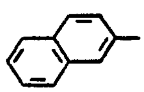 | 1 | $COCH_3$ | H | $CH_3$ | $CF_3$ |
| 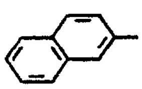 | 0 | H | Cl | $CH_3$ | $CF_3$ |
| 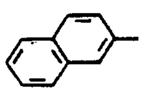 | 1 | H | Cl | $CH_3$ | $CF_3$ |
| 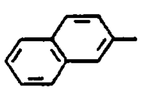 | 0 | $CH_3$ | Cl | $CH_3$ | $CF_3$ |
| 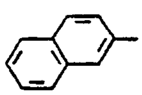 | 1 | $CH_3$ | Cl | $CH_3$ | $CF_3$ |

Tabelle II - Forts.

| A | n | $R^1$ | $R^2$ | $R^4$ | $R^{4''}$ |
|---|---|---|---|---|---|
| 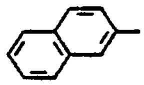 | 0 | $COCH_3$ | Cl | $CH_3$ | $CF_3$ |
| 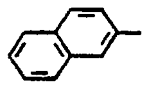 | 1 | $COCH_3$ | Cl | $CH_3$ | $CF_3$ |
| 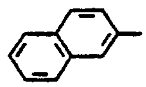 | 0 | H | CN | $CH_3$ | $CF_3$ |
| 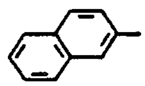 | 1 | H | CN | $CH_3$ | $CF_3$ |
| 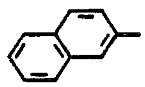 | 0 | $CH_3$ | CN | $CH_3$ | $CF_3$ |
| 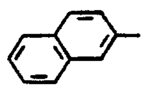 | 1 | $CH_3$ | CN | $CH_3$ | $CF_3$ |
| 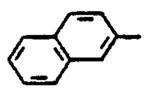 | 0 | $COCH_3$ | CN | $CH_3$ | $CF_3$ |
| 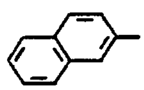 | 1 | $COCH_3$ | CN | $CH_3$ | $CF_3$ |
| 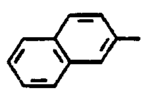 | 0 | H | H | $CH_3$ | $OCH_3$ |
| 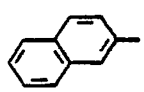 | 1 | H | H | $CH_3$ | $OCH_3$ |
| 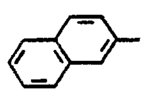 | 0 | $CH_3$ | H | $CH_3$ | $OCH_3$ |
| 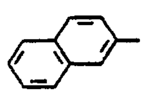 | 1 | $CH_3$ | H | $CH_3$ | $OCH_3$ |
| 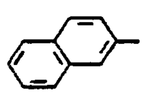 | 0 | $COCH_3$ | H | $CH_3$ | $OCH_3$ |
| 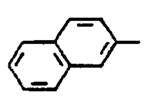 | 1 | $COCH_3$ | H | $CH_3$ | $OCH_3$ |

Tabelle II - Forts.

| A | n | R1 | R2 | R4 | R4'' |
|---|---|---|---|---|---|
| 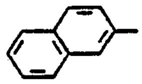 | 0 | H | Cl | $CH_3$ | $OCH_3$ |
| 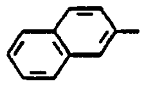 | 1 | H | Cl | $CH_3$ | $OCH_3$ |
| 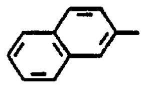 | 0 | $CH_3$ | Cl | $CH_3$ | $OCH_3$ |
| 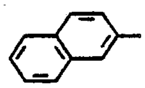 | 1 | $CH_3$ | Cl | $CH_3$ | $OCH_3$ |
| 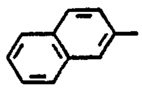 | 0 | $COCH_3$ | Cl | $CH_3$ | $OCH_3$ |
| 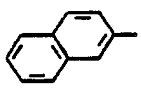 | 1 | $COCH_3$ | Cl | $CH_3$ | $OCH_3$ |
| 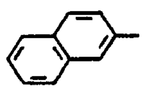 | 0 | H | CN | $CH_3$ | $OCH_3$ |
| 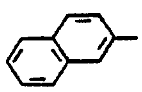 | 1 | H | CN | $CH_3$ | $OCH_3$ |
| 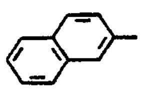 | 0 | $CH_3$ | CN | $CH_3$ | $OCH_3$ |
| 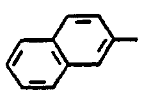 | 1 | $CH_3$ | CN | $CH_3$ | $OCH_3$ |
| 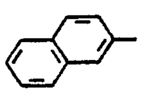 | 0 | $COCH_3$ | CN | $CH_3$ | $OCH_3$ |
| 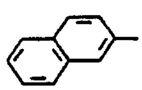 | 1 | $COCH_3$ | CN | $CH_3$ | $OCH_3$ |
| 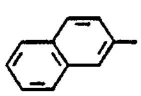 | 0 | H | H | $CF_3$ | $CH_3$ |
| 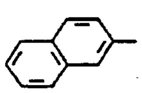 | 1 | H | H | $CF_3$ | $CH_3$ |

Tabelle II - Forts.

| A | n | $R^1$ | $R^2$ | $R^4$ | $R^{4''}$ |
|---|---|---|---|---|---|
| 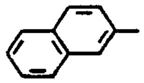 | 0 | $CH_3$ | H | $CF_3$ | $CH_3$ |
| 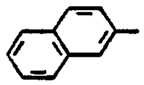 | 1 | $CH_3$ | H | $CF_3$ | $CH_3$ |
| 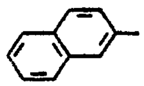 | 0 | $COCH_3$ | H | $CF_3$ | $CH_3$ |
| 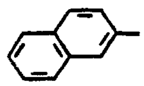 | 1 | $COCH_3$ | H | $CF_3$ | $CH_3$ |
| 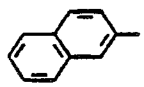 | 0 | H | Cl | $CF_3$ | $CH_3$ |
| 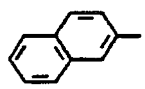 | 1 | H | Cl | $CF_3$ | $CH_3$ |
| 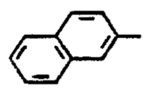 | 0 | $CH_3$ | Cl | $CF_3$ | $CH_3$ |
| 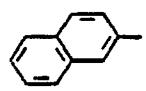 | 1 | $CH_3$ | Cl | $CF_3$ | $CH_3$ |
| 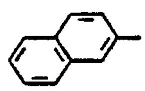 | 0 | $COCH_3$ | Cl | $CF_3$ | $CH_3$ |
| 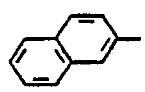 | 1 | $COCH_3$ | Cl | $CF_3$ | $CH_3$ |
| 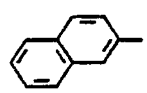 | 0 | H | CN | $CF_3$ | $CH_3$ |
| 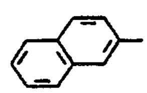 | 1 | H | CN | $CF_3$ | $CH_3$ |
| 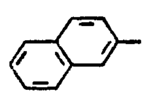 | 0 | $CH_3$ | CN | $CF_3$ | $CH_3$ |
| 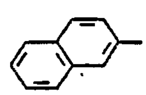 | 1 | $CH_3$ | CN | $CF_3$ | $CH_3$ |

Tabelle II - Forts.

| A | n | $R^1$ | $R^2$ | $R^4$ | $R^{4''}$ |
|---|---|---|---|---|---|
| 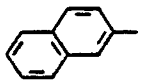 | 0 | $COCH_3$ | CN | $CF_3$ | $CH_3$ |
| 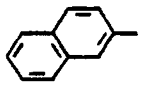 | 1 | $COCH_3$ | CN | $CF_3$ | $CH_3$ |
| 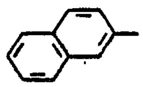 | 0 | H | H | $CF_3$ | $CF_3$ |
| 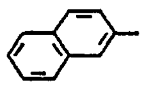 | 1 | H | H | $CF_3$ | $CF_3$ |
| 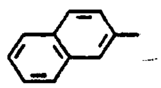 | 0 | $CH_3$ | H | $CF_3$ | $CF_3$ |
| 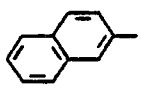 | 1 | $CH_3$ | H | $CF_3$ | $CF_3$ |
| 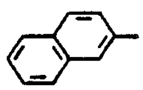 | 0 | $COCH_3$ | H | $CF_3$ | $CF_3$ |
| 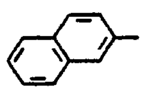 | 1 | $COCH_3$ | H | $CF_3$ | $CF_3$ |
| 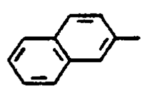 | 0 | H | Cl | $CF_3$ | $CF_3$ |
| 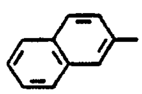 | 1 | H | Cl | $CF_3$ | $CF_3$ |
| 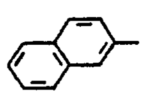 | 0 | $CH_3$ | Cl | $CF_3$ | $CF_3$ |
| 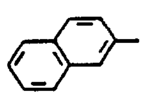 | 1 | $CH_3$ | Cl | $CF_3$ | $CF_3$ |

Tabelle II - Forts.

| A | n | $R^1$ | $R^2$ | $R^4$ | $R^{4''}$ |
|---|---|---|---|---|---|
| 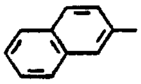 | 0 | $COCH_3$ | Cl | $CF_3$ | $CF_3$ |
| 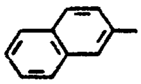 | 1 | $COCH_3$ | Cl | $CF_3$ | $CF_3$ |
| 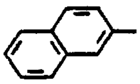 | 0 | H | CN | $CF_3$ | $CF_3$ |
| 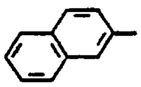 | 1 | H | CN | $CF_3$ | $CF_3$ |
| 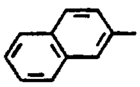 | 0 | $CH_3$ | CN | $CF_3$ | $CF_3$ |
| 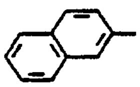 | 1 | $CH_3$ | CN | $CF_3$ | $CF_3$ |
| 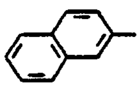 | 0 | $COCH_3$ | CN | $CF_3$ | $CF_3$ |
| 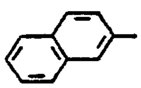 | 1 | $COCH_3$ | CN | $CF_3$ | $CF_3$ |

Tabelle III

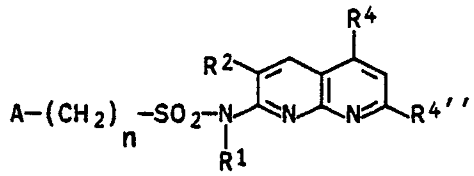

Ia

| A | n | $R^1$ | $R^2$ | $R^4$ | $R^{4''}$ |
|---|---|---|---|---|---|
| 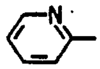 | 0 | H | H | $CH_3$ | $OCH_3$ |
| 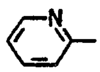 | 1 | H | H | $CH_3$ | $OCH_3$ |
| 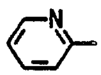 | 0 | $CH_3$ | H | $CH_3$ | $OCH_3$ |
| 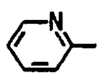 | 1 | $CH_3$ | H | $CH_3$ | $OCH_3$ |
| 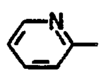 | 0 | H | CN | $CH_3$ | $OCH_3$ |
| 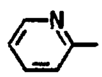 | 1 | H | CN | $CH_3$ | $OCH_3$ |
| 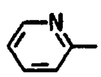 | 0 | $CH_3$ | CN | $CH_3$ | $OCH_3$ |
| 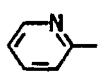 | 1 | $CH_3$ | CN | $CH_3$ | $OCH_3$ |
| 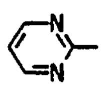 | 0 | H | H | $CF_3$ | $OCH_3$ |
| 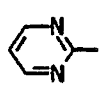 | 1 | H | H | $CF_3$ | $OCH_3$ |
| 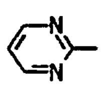 | 0 | $CH_3$ | H | $CF_3$ | $OCH_3$ |
| 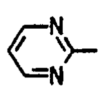 | 1 | $CH_3$ | H | $CF_3$ | $OCH_3$ |
| 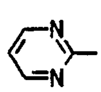 | 0 | H | CN | $CF_3$ | $OCH_3$ |
| 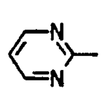 | 1 | H | CN | $CF_3$ | $OCH_3$ |

Tabelle III - Forts.

| A | n | $R^1$ | $R^2$ | $R^4$ | $R^{4''}$ |
|---|---|---|---|---|---|
| 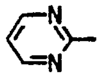 | 0 | $CH_3$ | CN | $CF_3$ | $OCH_3$ |
| 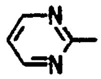 | 1 | $CH_3$ | CN | $CF_3$ | $OCH_3$ |
| 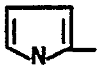 | 0 | H | H | $CH_3$ | $CF_3$ |
| 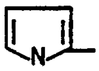 | 1 | H | H | $CH_3$ | $CF_3$ |
| 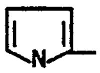 | 0 | $CH_3$ | H | $CH_3$ | $CF_3$ |
| 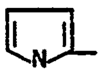 | 1 | $CH_3$ | H | $CH_3$ | $CF_3$ |
| 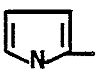 | 0 | H | CN | $CH_3$ | $CF_3$ |
| 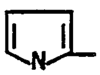 | 1 | H | CN | $CH_3$ | $CF_3$ |
| 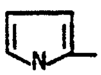 | 0 | $CH_3$ | CN | $CH_3$ | $CF_3$ |
| 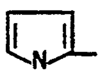 | 1 | $CH_3$ | CN | $CH_3$ | $CF_3$ |
| 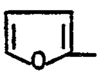 | 0 | H | H | $CH_3$ | $OCH_3$ |
| 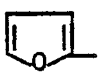 | 1 | H | H | $CH_3$ | $OCH_3$ |
| 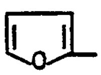 | 0 | $CH_3$ | H | $CH_3$ | $OCH_3$ |
| 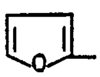 | 1 | $CH_3$ | H | $CH_3$ | $OCH_3$ |
| 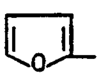 | 0 | H | CN | $CH_3$ | $OCH_3$ |
| 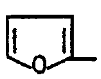 | 1 | H | CN | $CH_3$ | $OCH_3$ |

Tabelle III - Forts.

| A | n | $R^1$ | $R^2$ | $R^4$ | $R^{4''}$ |
|---|---|---|---|---|---|
| 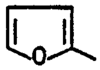 | 0 | $CH_3$ | CN | $CH_3$ | $OCH_3$ |
| 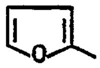 | 1 | $CH_3$ | CN | $CH_3$ | $OCH_3$ |
| 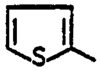 | 0 | H | H | $CF_3$ | $OCH_3$ |
| 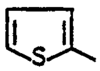 | 1 | H | H | $CF_3$ | $OCH_3$ |
| 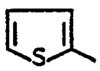 | 0 | $CH_3$ | H | $CF_3$ | $OCH_3$ |
| 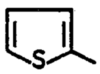 | 1 | $CH_3$ | H | $CF_3$ | $OCH_3$ |
| 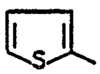 | 0 | H | CN | $CF_3$ | $OCH_3$ |
| 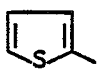 | 1 | H | CN | $CF_3$ | $OCH_3$ |
| 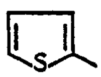 | 0 | $CH_3$ | CN | $CF_3$ | $OCH_3$ |
| 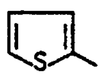 | 1 | $CH_3$ | CN | $CF_3$ | $OCH_3$ |
| 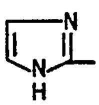 | 0 | H | H | $CH_3$ | $CF_3$ |
| 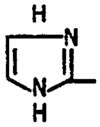 | 1 | H | H | $CH_3$ | $CF_3$ |
| 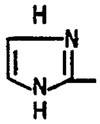 | 0 | $CH_3$ | H | $CH_3$ | $CF_3$ |
| 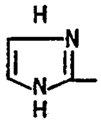 | 1 | $CH_3$ | H | $CH_3$ | $CF_3$ |
| 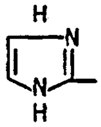 | 0 | H | CN | $CH_3$ | $CF_3$ |
| 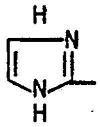 | 1 | H | CN | $CH_3$ | $CF_3$ |

Tabelle III - Forts.

| A | n | $R^1$ | $R^2$ | $R^4$ | $R^{4''}$ |
|---|---|---|---|---|---|
| 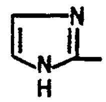 | 0 | $CH_3$ | CN | $CH_3$ | $CF_3$ |
| 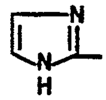 | 1 | $CH_3$ | CN | $CH_3$ | $CF_3$ |
| 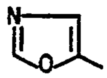 | 0 | H | H | $CH_3$ | $OCH_3$ |
| 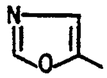 | 1 | H | H | $CH_3$ | $OCH_3$ |
| 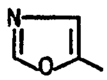 | 0 | $CH_3$ | H | $CH_3$ | $OCH_3$ |
| 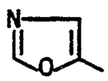 | 1 | $CH_3$ | H | $CH_3$ | $OCH_3$ |
| 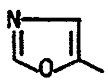 | 0 | H | CN | $CH_3$ | $OCH_3$ |
| 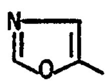 | 1 | H | CN | $CH_3$ | $OCH_3$ |
| 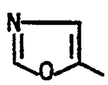 | 0 | $CH_3$ | CN | $CH_3$ | $OCH_3$ |
| 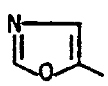 | 1 | $CH_3$ | CN | $CH_3$ | $OCH_3$ |
| 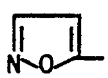 | 0 | H | H | $CF_3$ | $OCH_3$ |
| 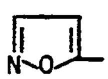 | 1 | H | H | $CF_3$ | $OCH_3$ |
| 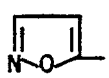 | 0 | $CH_3$ | H | $CF_3$ | $OCH_3$ |
| 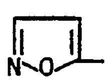 | 1 | $CH_3$ | H | $CF_3$ | $OCH_3$ |
| 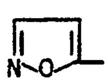 | 0 | H | CN | $CF_3$ | $OCH_3$ |
| 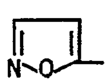 | 1 | H | CN | $CF_3$ | $OCH_3$ |

Tabelle III - Forts.

| A | n | $R^1$ | $R^2$ | $R^4$ | $R^{4''}$ |
|---|---|---|---|---|---|
| 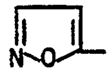 | 0 | $CH_3$ | CN | $CF_3$ | $OCH_3$ |
| 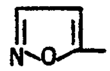 | 0 | $CH_3$ | CN | $CF_3$ | $OCH_3$ |
| 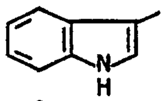 | 0 | H | H | $CH_3$ | $CF_3$ |
| 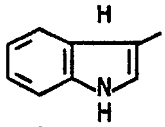 | 1 | H | H | $CH_3$ | $CF_3$ |
| 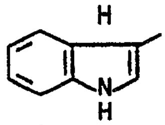 | 0 | $CH_3$ | H | $CH_3$ | $CF_3$ |
| 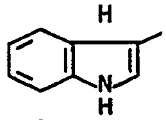 | 1 | $CH_3$ | H | $CH_3$ | $CF_3$ |
| 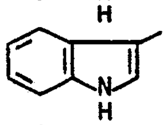 | 0 | H | CN | $CH_3$ | $CF_3$ |
| 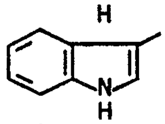 | 1 | H | CN | $CH_3$ | $CF_3$ |
| 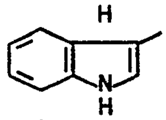 | 0 | $CH_3$ | CN | $CH_3$ | $CF_3$ |
| 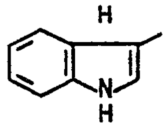 | 1 | $CH_3$ | CN | $CH_3$ | $CF_3$ |
| 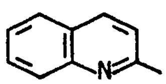 | 0 | H | H | $CH_3$ | $OCH_3$ |
| 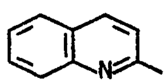 | 1 | H | H | $CH_3$ | $OCH_3$ |
| 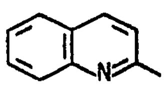 | 0 | $CH_3$ | H | $CH_3$ | $OCH_3$ |
| 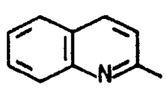 | 1 | $CH_3$ | H | $CH_3$ | $OCH_3$ |
| 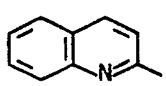 | 0 | H | CN | $CH_3$ | $OCH_3$ |
| 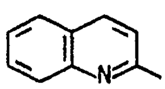 | 1 | H | CN | $CH_3$ | $OCH_3$ |

Tabelle III - Forts.

| A | n | $R^1$ | $R^2$ | $R^4$ | $R^{4''}$ |
|---|---|---|---|---|---|
| 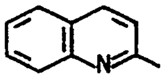 | 0 | $CH_3$ | CN | $CH_3$ | $OCH_3$ |
| 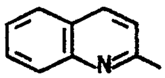 | 1 | $CH_3$ | CN | $CH_3$ | $OCH_3$ |
| 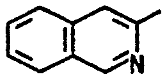 | 0 | H | H | $CF_3$ | $OCH_3$ |
| 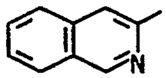 | 1 | H | H | $CF_3$ | $OCH_3$ |
| 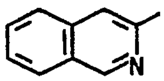 | 0 | $CH_3$ | H | $CF_3$ | $OCH_3$ |
| 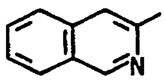 | 1 | $CH_3$ | H | $CF_3$ | $OCH_3$ |
| 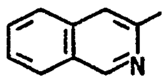 | 0 | H | CN | $CF_3$ | $OCH_3$ |
| 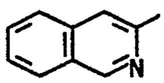 | 1 | H | CN | $CF_3$ | $OCH_3$ |
| 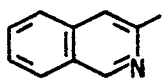 | 0 | $CH_3$ | CN | $CF_3$ | $OCH_3$ |
| 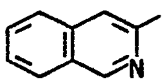 | 0 | $CH_3$ | CN | $CF_3$ | $OCH_3$ |
| 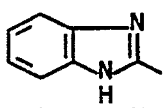 | 0 | H | H | $CH_3$ | $CF_3$ |
| 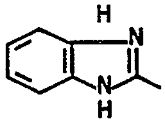 | 1 | H | H | $CH_3$ | $CF_3$ |
| 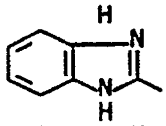 | 0 | $CH_3$ | H | $CH_3$ | $CF_3$ |
| 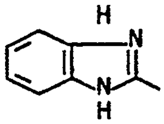 | 1 | $CH_3$ | H | $CH_3$ | $CF_3$ |
| 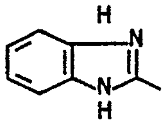 | 0 | H | CN | $CH_3$ | $CF_3$ |
| 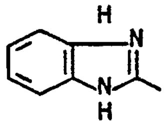 | 1 | H | CN | $CH_3$ | $CF_3$ |

Tabelle III - Forts.

| A | n | $R^1$ | $R^2$ | $R^4$ | $R^{4''}$ |
|---|---|---|---|---|---|
| 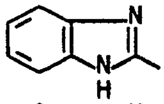 | 0 | $CH_3$ | CN | $CH_3$ | $CF_3$ |
| 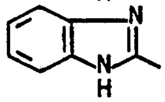 | 1 | $CH_3$ | CN | $CH_3$ | $CF_3$ |

Tabelle IV

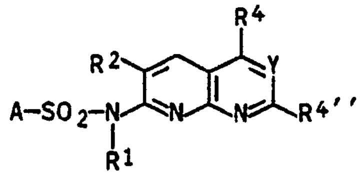

| A | $R^1$ | $R^2$ | $R^4$ | $R^{4''}$ | Y |
|---|---|---|---|---|---|
| 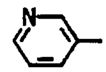 | H | H | $CH_3$ | $OCH_3$ | N |
| 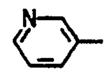 | $CH_3$ | H | $CH_3$ | $OCH_3$ | N |
| 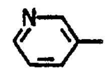 | H | CN | $CH_3$ | $OCH_3$ | C-Cl |
| 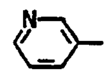 | $CH_3$ | CN | $CH_3$ | $OCH_3$ | C-Cl |
| 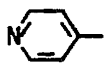 | H | H | $CH_3$ | $CF_3$ | N |
| 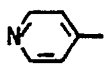 | $CH_3$ | H | $CH_3$ | $CF_3$ | N |
| 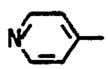 | H | CN | $CH_3$ | $CF_3$ | C-Cl |
| 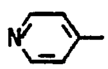 | $CH_3$ | CN | $CH_3$ | $CF_3$ | C-Cl |
| 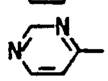 | H | H | $CF_3$ | $CH_3$ | N |
| 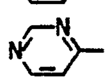 | $CH_3$ | H | $CF_3$ | $CH_3$ | N |
| 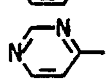 | H | CN | $CF_3$ | $CH_3$ | C-Cl |
| 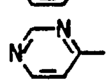 | $CH_3$ | CN | $CF_3$ | $CH_3$ | C-Cl |
| 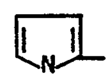 | H | H | $CH_3$ | Cl | N |
| 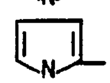 | $CH_3$ | H | $CH_3$ | Cl | N |
| 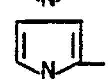 | H | CN | $CH_3$ | Cl | C-Cl |
| 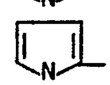 | $CH_3$ | CN | $CH_3$ | Cl | C-Cl |
| 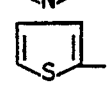 | H | H | $CF_3$ | $CF_3$ | N |
| 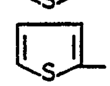 | $CH_3$ | H | $CF_3$ | $CF_3$ | N |
| 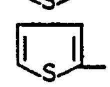 | H | CN | $CF_3$ | $CF_3$ | C-Cl |
| 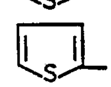 | $CH_3$ | CN | $CF_3$ | $CF_3$ | C-Cl |

Tabelle IV - Forts.

| A | $R^1$ | $R^2$ | $R^4$ | $R^{4''}$ | Y |
|---|---|---|---|---|---|
| 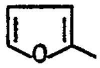 | H | H | $CH_3$ | $CH_3$ | N |
| 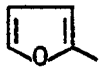 | $CH_3$ | H | $CH_3$ | $CH_3$ | N |
| 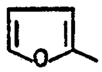 | H | CN | $CH_3$ | $CH_3$ | C-Cl |
| 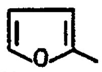 | $CH_3$ | CN | $CH_3$ | $CH_3$ | C-Cl |
| 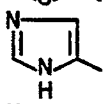 | H | H | $CH_3$ | $CH_3$ | N |
| 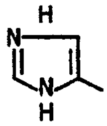 | $CH_3$ | H | $CH_3$ | $OCH_3$ | N |
| 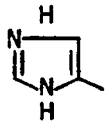 | H | CN | $CH_3$ | $OCH_3$ | C-Cl |
| 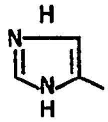 | $CH_3$ | CN | $CH_3$ | $OCH_3$ | C-Cl |
| 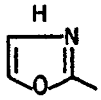 | H | H | $CH_3$ | $CF_3$ | N |
| 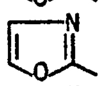 | $CH_3$ | H | $CH_3$ | $CF_3$ | N |
| 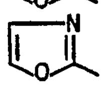 | H | CN | $CH_3$ | $CF_3$ | C-Cl |
| 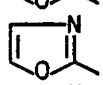 | $CH_3$ | CN | $CH_3$ | $CF_3$ | C-Cl |
| 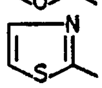 | H | H | $CF_3$ | $CH_3$ | N |
| 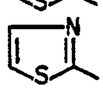 | $CH_3$ | H | $CF_3$ | $CH_3$ | N |
| 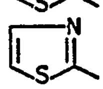 | H | CN | $CF_3$ | $CH_3$ | C-Cl |
| 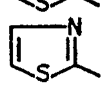 | $CH_3$ | CN | $CF_3$ | $CH_3$ | C-Cl |
| 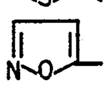 | H | H | $CH_3$ | Cl | N |
| 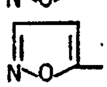 | $CH_3$ | H | $CH_3$ | Cl | N |
| 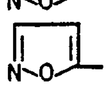 | H | CN | $CH_3$ | Cl | C-Cl |
| 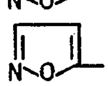 | $CH_3$ | CN | $CH_3$ | Cl | C-Cl |
| 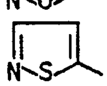 | H | H | $CF_3$ | $CF_3$ | N |

Tabelle IV - Forts.

| A | $R^1$ | $R^2$ | $R^4$ | $R^{4''}$ | Y |
|---|---|---|---|---|---|
| 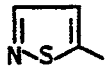 | $CH_3$ | H | $CF_3$ | $CF_3$ | N |
| 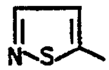 | H | CN | $CF_3$ | $CF_3$ | C-Cl |
| 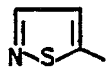 | $CH_3$ | CN | $CF_3$ | $CF_3$ | C-Cl |
| 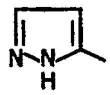 | H | H | $CH_3$ | $CH_3$ | N |
| 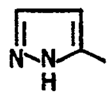 | $CH_3$ | H | $CH_3$ | $CH_3$ | N |
| 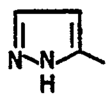 | H | CN | $CH_3$ | $CH_3$ | C-Cl |
| 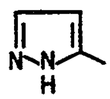 | $CH_3$ | CN | $CH_3$ | $CH_3$ | C-Cl |
| 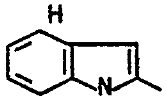 | H | H | $CH_3$ | $OCH_3$ | N |
| 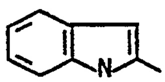 | $CH_3$ | H | $CH_3$ | $OCH_3$ | N |
| 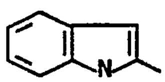 | H | CN | $CH_3$ | $OCH_3$ | C-Cl |
| 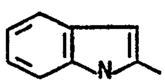 | $CH_3$ | CN | $CH_3$ | $OCH_3$ | C-Cl |
| 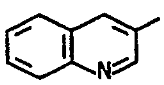 | H | H | $CH_3$ | $CF_3$ | N |
| 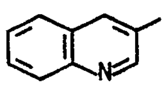 | $CH_3$ | H | $CH_3$ | $CF_3$ | N |
| 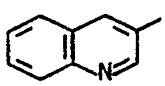 | H | CN | $CH_3$ | $CF_3$ | C-Cl |
| 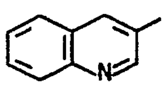 | $CH_3$ | CN | $CH_3$ | $CF_3$ | C-Cl |
| 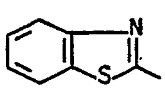 | H | H | $CF_3$ | $CH_3$ | N |
| 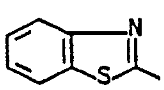 | $CH_3$ | H | $CF_3$ | $CH_3$ | N |

Tabelle IV - Forts.

| A | $R^1$ | $R^2$ | $R^4$ | $R^{4''}$ | Y |
|---|---|---|---|---|---|
| 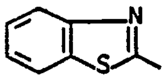 | H | CN | $CF_3$ | $CH_3$ | C-Cl |
| 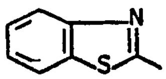 | $CH_3$ | CN | $CF_3$ | $CH_3$ | C-Cl |
| 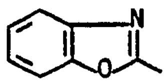 | H | H | $CH_3$ | Cl | N |
| 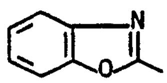 | $CH_3$ | H | $CH_3$ | Cl | N |
| 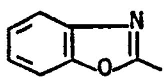 | H | CN | $CH_3$ | Cl | C-Cl |
| 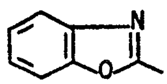 | $CH_3$ | CN | $CH_3$ | Cl | C-Cl |
| 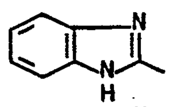 | H | H | $CF_3$ | $CF_3$ | N |
| 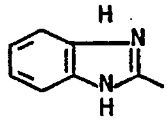 | $CH_3$ | H | $CF_3$ | $CF_3$ | N |
| 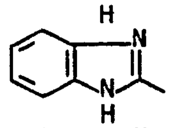 | H | CN | $CF_3$ | $CF_3$ | C-Cl |
| 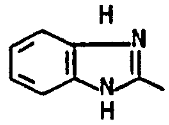 | $CH_3$ | CN | $CF_3$ | $CF_3$ | C-Cl |
| 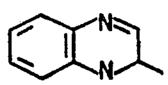 | H | H | $CH_3$ | $CH_3$ | N |
| 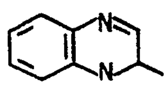 | $CH_3$ | H | $CH_3$ | $CH_3$ | N |
| 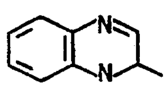 | H | CN | $CH_3$ | $CH_3$ | C-Cl |
| 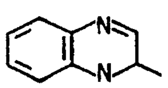 | $CH_3$ | CN | $CH_3$ | $CH_3$ | C-Cl |

Die substituierten Sulfonamide I bzw. die sie enthaltenden herbiziden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungformen richten sich nach den Verwendungszwecken; sie sollten in jedem Fall möglichst die feinste Verteilung der erfindungsgemäßen Wirkstoffe gewährleisten.

Die Verbindungen I eignen sich allgemein zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon,

Chlorbenzol, Isophoron oder stark polare Lösungsmittel, wie N,N-Dimethylformamid, Dimethylsulfoxid, N-Methylpyrrolidon oder Wasser.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin-und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Formulierungen enthalten im allgemeinen zwischen 0,1 und 95 Gew.%, vorzugsweise zwischen 0,5 und 90 Gew.%, Wirkstoff.

Beispiele für Formulierungen sind:

I. Man vermischt 90 Gewichtsteile der Verbindung Nr. 1.047 mit 10 Gewichtsteilen N-Methyl-α-pyrrolidon und erhält eine Lösung, die zur Anwendung in Form kleinster Tropfen geeignet ist.

II. 20 Gewichtsteile der Verbindung Nr. 1.011 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.

III. 20 Gewichtsteile der Verbindung Nr. 3.008 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

IV. 20 Gewichtsteile des Wirkstoffs Nr. 3.012 werden in einer Mischung gelöst, die aus 25 Gewichtsteilen Cyclohexanon, 65 Gewichtsteilen einer Mineralölfraktion vom Siedepunkt 210 bis 280 °C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

V. 20 Gewichtsteile des Wirkstoffs Nr. 2.023 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.% des Wirkstoffs enthält.

VI. 3 Gewichtsteile des Wirkstoffs Nr. 1.092 werden mit 97 Gewichtsteilen feinteiligem Kaolin vermischt. Man erhält auf diese Weise ein Stäubemittel, das 3 Gew.% des Wirkstoffs enthält.

VII. 30 Gewichtsteile des Wirkstoffs Nr. 3.039 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Gewichtsteile des Wirkstoffs Nr. 1.037 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.

Die Applikation kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Bekämpfungsziel, Jahreszeit, Zielpflanzen und Wachstumsstadium 0,001 bis 5,0 kg/ha, vorzugsweise 0,01 bis 1,0 kg/ha.

In Anbetracht der Vielseitigkeit der Applikationsmethoden können die erfindungsgemäßen Verbindungen bzw. sie enthaltende Mittel noch in einer weiteren Zahl von Kulturplfanzen zur Beseitigung unerwünschter Pflanzen eingesetzt werden. In Betracht kommen beispielsweise folgende Kulturen:

| Botanischer Name | Deutscher Name |
|---|---|
| Ananas comosus | Ananas |
| Arachis hypogaea | Erdnuß |
| Asparagus officinalis | Spargel |
| Brassica napus var. napus | Raps |
| Brassica napus var. napobrassica | Kohlrübe |
| Brassica napus var. rapa | Weiße Rübe |
| Brassica rapa var. silvestris | Rüben |
| Camellia sinensis | Teestrauch |
| Carthamus tinctorius | Saflor - Färberdistel |
| Carya illinoinensis | Pekannußbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Cynodon dactylon | Bermudagras |
| Elaeis guineensis | Ölpalme |
| Fragaria vesca | Erdbeere |
| Glycine max | Sojabohne |
| Gossypium hirsutum (Gossypium arboreum, Gossypium herbaceum, Gossypium vitifolium) | Baumwolle |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Lactuca sativa | Kopfsalat |
| Linum usitatissimum | Faserlein |
| Lycopersicon lycopersicum | Tomate |
| Malus spp. | Apfel |

| Botanischer Name | Deutscher Name |
|---|---|
| Manihot esculenta | Maniok |
| Medicago sativa | Luzerne |
| Mentha piperita | Pfefferminze |
| Musa spp. | Obst- und Mehlbanane |
| Nicotiana tabacum (N. rustica) | Tabak |
| Olea europaea | Ölbaum |
| Oryza sativa | Reis |
| Panicum miliaceum | Rispenhirse |
| Phaseolus lunatus | Mondbohne |
| Phaseolus mungo | Erdbohne |
| Phaseolus vulgaris | Buschbohnen |
| Pennisetum glaucum | Perl- oder Rohrkolbenhirse |
| Petroselinum crispum spp. tuberosum | Wurzelpetersilie |
| Picea abies | Rotfichte |
| Abies alba | Weißtanne |
| Pinus spp. | Kiefer |
| Pisum sativum | Gartenerbse |
| Prunus avium | Süßkirsche |
| Prunus domestica | Pflaume |
| Prunus dulcis | Mandelbaum |
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |
| Sesamum indicum | Sesam |
| Solanum tuberosum | Kartoffel |
| Sorghum bicolor (s. vulgare) | Mohrenhirse |
| Sorghum dochna | Zuckerhirse |
| Spinacia oleracea | Spinat |
| Theobroma cacao | Kakaobaum |
| Trifolium pratense | Rotklee |
| Triticum aestivum | Weizen |
| Triticum durum | Hartweizen |
| Vaccinium corymbosum | Kulturheidelbeere |
| Vaccinium vitis-idaea | Preißelbeere |
| Vicia faba | Pferdebohnen |
| Vigna sinensis (V. unguiculata) | Kuhbohne |
| Vitis vinifera | Weinrebe |
| Zea mays | Mais |

Zur Verbreiterung des Wirkungsspektrums und zur Erzielung synergistischer Effekte kö nnen die Sulfonamide der Formel I mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3, 1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Imidazolinone, Sulfonylharnstoffe, Cyclohexan-1,3-dionderivate, Chinolincarbonsäurederivate, Phenyloxy- bzw. Heteroaryloxyphenylpropionsäuren sowie deren Salze, Ester und Amide und andere in Betracht.

Außerdem kann es von Nutzen sein, die neuen Verbindungen der Formel I allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

Die in den nachstehenden Synthesebeispielen wiedergegebenen Arbeitsvorschriften wurden unter entsprechender Abwandlung der Ausgangsverbindungen zur Herstellung weiterer Verbindungen I benutzt. Die so erhaltenen Verbindungen sind in den Tabellen 1, 1a, 2 und 3 mit physikalischen Angaben aufgeführt.

Beispiel 1

2-Chlor-N-(5,7-dimethyl-1,8-naphthyridin-2-yl)benzolsulfonamid

Zu einer Suspension von 4,1 g (23,6 mmol) an kommerziell erhältlichem 2-Amino-5,7-dimethyl-1,8-naphthyridin in 100 ml trockenem Pyridin wurden bei einer Innentemperatur von 40 bis 50 °C 7,5 g (35,5 mmol) 2-Chlorbenzolsulfonsäurechlorid unter Rühren langsam zugetropft.

Anschließend wurde das Reaktionsgemisch eine Stunde lang bei 75 °C gerührt und danach weitere 1,5 Stunden am Rückfluß gekocht. Nach dem Abkühlen wurde bis zur Trockne eingeengt und der Rückstand einer Säulenchromatographie an Kieselgel zuerst mit Methylenchlorid und dann mit Essigester als Laufmittel unterzogen. Das Rohprodukt wurde durch Umkristallisation aus Methanol weiter gereinigt. Man erhielt 0,75 g (9 %) des gewünschten Produktes, das $^1$H-NMR- und IR-spektroskopisch charakterisiert wurde und einen Schmelzpunkt von 200 bis 202 °C hat.

$^1$H-NMR-Spektrum (250 MHz, $CDCl_3$):
δ [ppm] = 2,55 (s; 3H), 2,60 (s; 3H), 6,92 (d, J = 9,5 HZ; 1H), 7,05 (s; 1H), 7,35-7,50 (m; 3H), 8,03 (d, J = 9,5 Hz; 1H), 8,28 (d, J = 7 Hz; 1H), 12,0 (br, s; 1H).

Beispiel 2

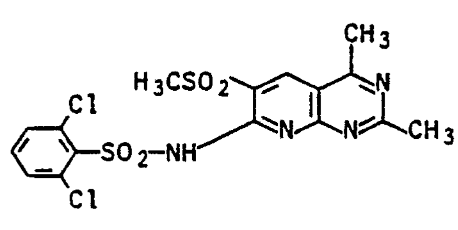

a) Eine Suspension von 38,4 g (0,25 mol) 4-Amino-2,6-dienethylpyrimidin-5-carbaldehyd in 600 ml Methanol wurde bei 25 °C nacheinander mit 30,3 g (0,25 mol) Methylsulfonylacetonitril und 45,8 g (0,25 mol) einer 30 %igen Natriummethylatlösung in Methanol versetzt. Unter leichter Erwärmung bildete sich zunächst eine klare Lösung, aus der sich dann ein voluminöser Niederschlag abschied. Nach 2 Stunden Rühren bei Siedetemperatur wurde auf 0 °C abgekühlt, der Niederschlag isoliert, mit Methanol gewaschen und getrocknet. Man erhielt so 47 % an 7-Amino-2,4-dimethyl-6-methylsulfonylpyrido[2,3-d]-pyrimidin.

$^1$H-NMR (250 MHz, $d^6$ DMSO, vs, TMS):
δ [ppm]: 8,70 (s, 1H), 7,43 (br, 2H), 3,34 (s, 3H), 2,71 (s, 3H), 2,61 (s, 3H).

b) Eine Suspension von 1,05 g NaH (44 mmol) in 80 ml THF wurde bei Raumtemperatur mit 5,0 g (20 mmol) des Amins a versetzt, und bis zum Ende der Gasentwicklung auf Rückflußtemperatur erhitzt.

Nach dem Abkühlen auf ca. 40° C wurde mit 5,4 g (22 mmol) 2,6-Dichlorbenzolsulfonsäurechlorid in 20 ml THF versetzt und 5 h bei Rückflußtemperatur gerührt. Der gebildete Niederschlag wurde abgesaugt und in einem Gemisch von Methylenchlorid und 3 n Salzsäure aufgenommen. Die organische Phase wurde isoliert, mit Wasser gewaschen, getrocknet und bei vermindertem Druck getrocknet. Man erhielt so 81 % des gewünschten Sulfonamids vom Fp. > 230° C (Wirkstoffbeispiel 3.008).

Tabelle 1

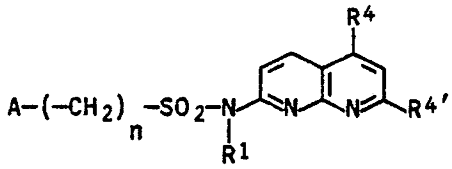

| Verbindung | A | n | $R^1$ | $R^4$ | $R^{4'}$ | Fp [°C] |
|---|---|---|---|---|---|---|
| 1.001 | 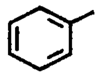 | 0 | H | $CH_3$ | $CH_3$ | 182-184 |
| 1.002 | 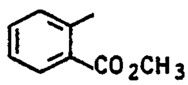 | 0 | H | $CH_3$ | $CH_3$ | 178-180 |
| 1.003 | 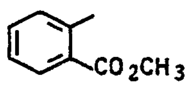 | 0 | H | $CH_3$ | Cl | 174-179 |
| 1.004 | 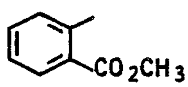 | 0 | H | $CH_3$ | $CF_3$ | 161-164 |
| 1.004 · $N(n-C_4H_9)_3$ | 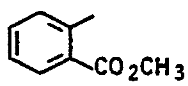 | 0 | H | $CH_3$ | $CF_3$ | 63-68 |
| 1.005 | 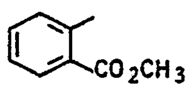 | 0 | H | $CF_3$ | $CH_3$ | 153-156 |
| 1.006 | 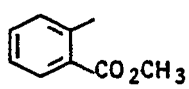 | 0 | H | $CH_3$ | $OCH_3$ | 179-183 |
| 1.006 · HCl | 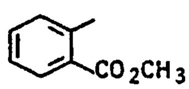 | 0 | H | $CH_3$ | $OCH_3$ | 169-171 |
| 1.007 | 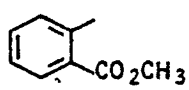 | 0 | K | $CF_3$ | $CF_3$ | 265-268 |
| 1.008 | 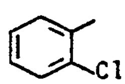 | 0 | H | $CH_3$ | $CH_3$ | 200-202 |
| 1.009 | 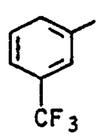 | 0 | H | $CH_3$ | $CH_3$ | 128-132 |
| 1.010 | 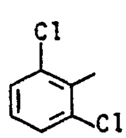 | 0 | H | $CH_3$ | $CH_3$ | 230-233 |

Tabelle 1 - Forts.

| Verbindung | A | n | $R^1$ | $R^4$ | $R^{4'}$ | Fp [°C] |
|---|---|---|---|---|---|---|
| 1.011 | 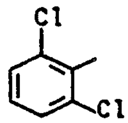 | 0 | H | $CH_3$ | Cl | 225-229 |
| 1.011 · $N(n\text{-}C_4H_9)_3$ | 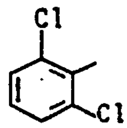 | 0 | H | $CH_3$ | Cl | 96-98 |
| 1.012 | 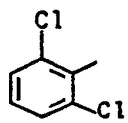 | 0 | H | $CH_3$ | $CF_3$ | 181-184 |
| 1.013 | 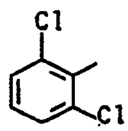 | 0 | H | $CF_3$ | $CH_3$ | 232-235 |
| 1.014 | 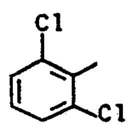 | 0 | H | $CH_3$ | $OCH_3$ | 215-221 |
| 1.015 | 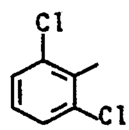 | 0 | H | $CF_3$ | $CF_3$ | 215-219 |
| 1.016 | 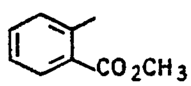 | 0 | Na | $CH_3$ | $OCH_3$ | 208-214 |
| 1.017 | 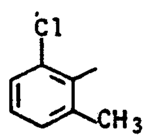 | 0 | H | $CH_3$ | $CH_3$ | 229-232 |
| 1.017 · HCl | 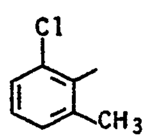 | 0 | H | $CH_3$ | $CH_3$ | 241-249 |
| 1.018 | 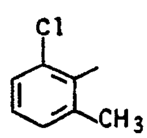 | 0 | H | $CH_3$ | Cl | 200-203 |
| 1.019 | 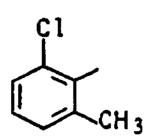 | 0 | H | $CF_3$ | $CH_3$ | 186-189 |

Tabelle 1 - Forts.

| Verbindung | A | n | $R^1$ | $R^4$ | $R^{4'}$ | Fp [°C] |
|---|---|---|---|---|---|---|
| 1.020 | 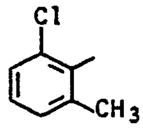 | 0 | H | $CH_3$ | $CF_3$ | 187-189 |
| 1.021 | 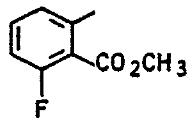 | 0 | H | $CH_3$ | $CH_3$ | 210-215 |
| 1.022 | 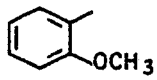 | 0 | H | $CH_3$ | $CH_3$ | 218-222 |
| 1.023 | 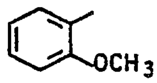 | 0 | Na | $CH_3$ | $CH_3$ | 236-239 |
| 1.024 | 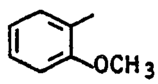 | 0 | H | $CH_3$ | Cl | 232-238 |
| 1.025 | 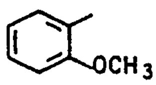 | 0 | H | $CH_3$ | $OCH_3$ | 192-196 |
| 1.026 | 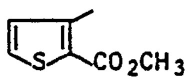 | 0 | H | $CH_3$ | $CH_3$ | 207-211 |
| 1.026 · HCl | 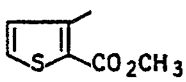 | 0 | H | $CH_3$ | $CH_3$ | 204-209 |
| 1.027 | 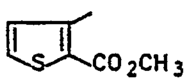 | 0 | H | $CH_3$ | Cl | 218-220 |
| 1.028 | 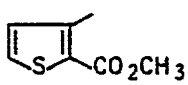 | 0 | H | $CF_3$ | $CH_3$ | 192-195 |
| 1.029 | 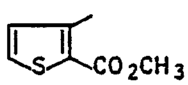 | 0 | H | $CH_3$ | $CF_3$ | 198-200 |
| 1.030 | 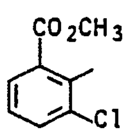 | 0 | H | $CH_3$ | $CH_3$ | 244-228 |
| 1.031 | 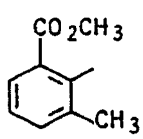 | 0 | H | $CH_3$ | $CH_3$ | 188-192 |

Tabelle 1 - Forts.

| Verbindung | A | n | $R^1$ | $R^4$ | $R^{4'}$ | Fp [°C] |
|---|---|---|---|---|---|---|
| 1.032 | 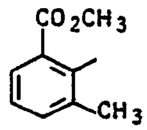 | 0 | H | $CH_3$ | Cl | 169-170 |
| 1.033 | 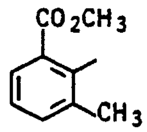 | 0 | H | $CH_3$ | $OCH_3$ | 172-176 |
| 1.034 | 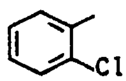 | 1 | H | $CH_3$ | $CH_3$ | 204-207 |
| 1.035 | 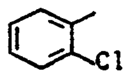 | 1 | H | $CH_3$ | Cl | 215-218 |
| 1.036 | 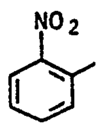 | 0 | H | $CH_3$ | $CH_3$ | 165 (Zers.) |
| 1.037 | 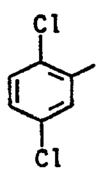 | 0 | H | $CH_3$ | $CH_3$ | 229-233 |
| 1.038 | 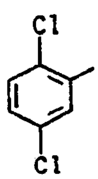 | 0 | H | $CH_3$ | Cl | 224-227 |
| 1.039 | 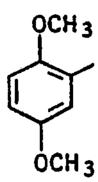 | 0 | H | $CH_3$ | $CH_3$ | 179-183 |
| 1.040 | 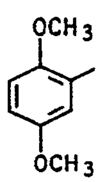 | 0 | Na | $CH_3$ | $CH_3$ | 193 (Zers.) |
| 1.041 | 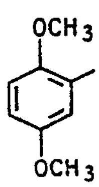 | 0 | H | $CH_3$ | Cl | 200-205 |

Tabelle 1 - Forts.

| Verbindung | A | n | $R^1$ | $R^4$ | $R^{4'}$ | Fp [°C] |
|---|---|---|---|---|---|---|
| 1.042 | 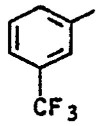 | 0 | Na | $CH_3$ | $CH_3$ | 172-180 |
| 1.043 | 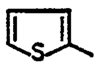 | 0 | H | $CH_3$ | $CH_3$ | 199-202 |
| 1.044 | 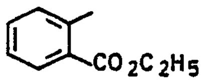 | 1 | H | $CH_3$ | $CH_3$ | 175-179 |
| 1.045 | 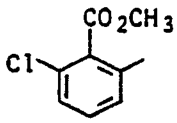 | 0 | H | $CH_3$ | $CH_3$ | 207-210 |
| 1.046 | 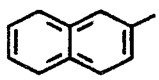 | 0 | H | $CH_3$ | $CH_3$ | 187-190 |
| 1.047 | 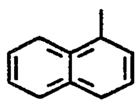 | 0 | H | $CH_3$ | $CH_3$ | 224-226 |
| 1.048 | 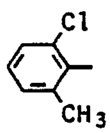 | 0 | H | $CF_3$ | $CF_3$ | 165-168 |
| 1.049 | 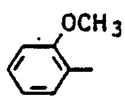 | 0 | H | $CF_3$ | $CF_3$ | 172-182 |
| 1.050 | 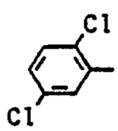 | 0 | H | $CF_3$ | $CF_3$ | 172-177 |
| 1.051 | 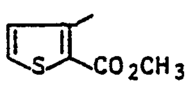 | 0 | H | $CF_3$ | $CF_3$ | 151-157 |
| 1.052 | 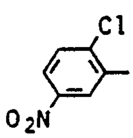 | 0 | H | $CH_3$ | $CH_3$ | 259-263 |
| 1.053 | 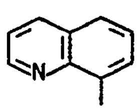 | 0 | H | $CH_3$ | $CH_3$ | 249-255 |
| 1.054 | 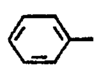 | 0 | H | $CH_3$ | Cl | 173-175 |

Tabelle 1 - Forts.

| Verbindung | A | n | $R^1$ | $R^4$ | $R^{4'}$ | Fp [°C] |
|---|---|---|---|---|---|---|
| 1.055 | 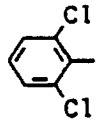 | 0 | H | $CH_3$ | $N(CH_3)_2$ | 286-288 |
| 1.056 | 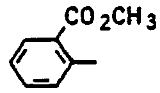 | 0 | H | $CH_3$ | $N(CH_3)_2$ | 240-243 |
| 1.057 | 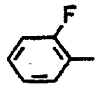 | 0 | H | $CH_3$ | $CH_3$ | 209-210 |
| 1.058 | 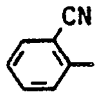 | 0 | H | $CH_3$ | $CH_3$ | 259-261 |
| 1.059 | 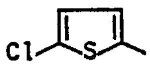 | 0 | H | $CH_3$ | $CH_3$ | 161-162 |
| 1.060 | 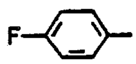 | 0 | H | $CH_3$ | $CH_3$ | 200-202 |
| 1.061 | 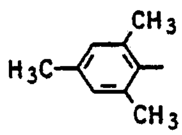 | 0 | H | $CH_3$ | $CH_3$ | 184-188 |
| 1.062 | 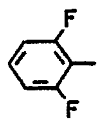 | 0 | H | $CH_3$ | $CH_3$ | 210-215 |
| 1.063 | 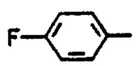 | 0 | H | $CH_3$ | Cl | 160-170 |
| 1.064 | 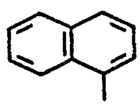 | 0 | H | $CH_3$ | Cl | 194-200 |
| 1.065 | 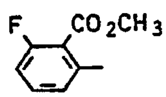 | 0 | H | $CH_3$ | Cl | 185-186 |
| 1.066 | 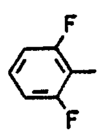 | 0 | H | $CH_3$ | Cl | 200-203 |
| 1.067 | 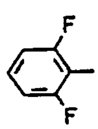 | 0 | H | $CH_3$ | $OCH_3$ | 220-225 |

Tabelle 1 - Forts.

| Verbindung | A | n | $R^1$ | $R^4$ | $R^{4'}$ | Fp [°C] |
|---|---|---|---|---|---|---|
| 1.068 | 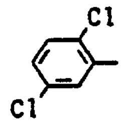 | 0 | H | $CH_3$ | $OCH_3$ | 217-221 |
| 1.069 | 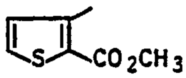 | 0 | H | $CH_3$ | $OCH_3$ | 240-241 |
| 1.070 | 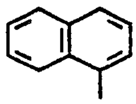 | 0 | H | $CH_3$ | $OCH_3$ | 240 |
| 1.071 | 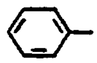 | 0 | H | $CH_3$ | $OCH_3$ | 215-126 |
| 1.072 | 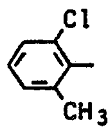 | 0 | H | $CH_3$ | $OCH_3$ | 238-240 |
| 1.073 | 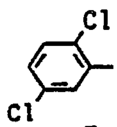 | 0 | H | $CH_3$ | $N(CH_3)_2$ | 252-255 |
| 1.074 | 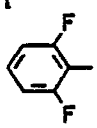 | 0 | H | $CH_3$ | $N(CH_3)_2$ | > 280 |
| 1.075 | 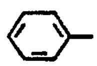 | 0 | H | $CH_3$ | $SCH_3$ | 166-168 |
| 1.076 | 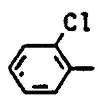 | 0 | H | $CH_3$ | $SCH_3$ | 226-268 |
| 1.077 | 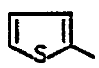 | 0 | H | $CH_3$ | $SCH_3$ | 261-268 |
| 1.078 | 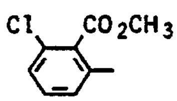 | 0 | H | $CH_3$ | $SCH_3$ | 208-216 |
| 1.079 | 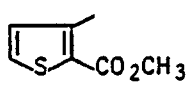 | 0 | H | $CH_3$ | $SCH_3$ | 204-206 |
| 1.080 | 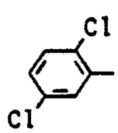 | 0 | H | $CH_3$ | $SCH_3$ | 226-230 |

Tabelle 1a

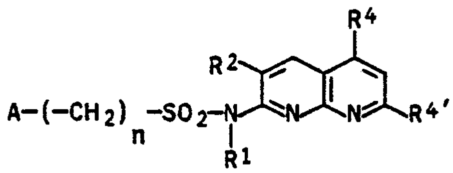

| Nr. | A | n | $R^1$ | $R^2$ | $R^4$ | $R^{4'}$ | Fp [°C] |
|---|---|---|---|---|---|---|---|
| 1.081 | 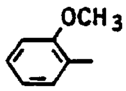 | 0 | H | H | $CH_3$ | $SCH_3$ | 210-215 |
| 1.082 | 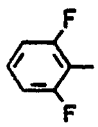 | 0 | H | H | $CH_3$ | $SCH_3$ | 230-233 |
| 1.083 | 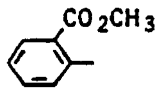 | 0 | H | H | $CH_3$ | $SCH_3$ | 231-235 |
| 1.084 | 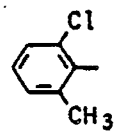 | 0 | H | H | $CH_3$ | $SCH_3$ | 225-227 |
| 1.085 | 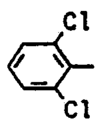 | 0 | H | H | $CH_3$ | $SCH_3$ | 225-228 |
| 1.086 | 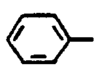 | 0 | H | CN | H | H | 200-208 |
| 1.087 | 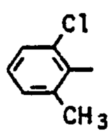 | 0 | H | CN | H | H | 182-186 |
| 1.088 | 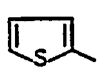 | 0 | H | H | 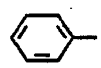 | $CH_3$ | > 300 |
| 1.089 | 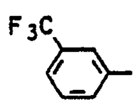 | 0 | H | H | 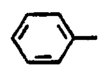 | $CH_3$ | 184-188 |
| 1.090 | 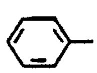 | 0 | H | H | 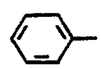 | $CH_3$ | 269-270 |
| 1.091 | 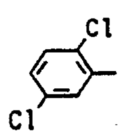 | 0 | H | H | 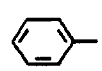 | $CH_3$ | 265-266 |

Tabelle 1a - Forts.

| Nr. | A | n | $R^1$ | $R^2$ | $R^4$ | $R^{4'}$ | Fp [°C] |
|---|---|---|---|---|---|---|---|
| 1.092 | 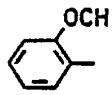 | 0 | H | H | 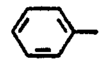 | $CH_3$ | 283-284 |
| 1.093 | 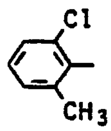 | 0 | H | H | 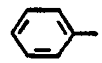 | $CH_3$ | 278-280 |
| 1.094 | 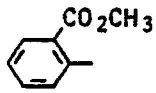 | 0 | H | H | 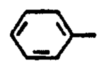 | $CH_3$ | 221-225 |
| 1.095 | 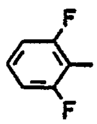 | 0 | H | H | 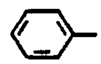 | $CH_3$ | 263 |
| 1.096 | 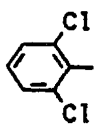 | 0 | H | H | 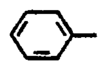 | $CH_3$ | 265-267 |
| 1.097 | 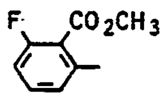 | 0 | H | H | $CH_3$ | 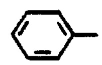 | 210-214 |
| 1.098 | 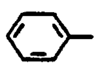 | 0 | H | H | $CH_3$ | 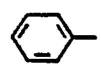 | 239-243 |
| 1.099 | 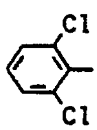 | 0 | H | H | $CH_3$ | 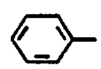 | 248-251 |
| 1.100 | 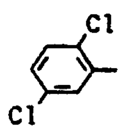 | 0 | H | H | $CH_3$ | 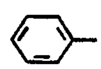 | 250-256 |
| 1.101 | 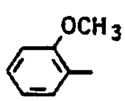 | 0 | H | H | $CH_3$ | 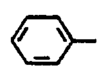 | 273-275 |
| 1.102 | 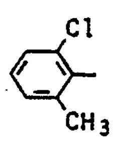 | 0 | H | H | $CH_3$ | 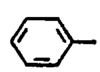 | 282-283 |
| 1.103 | 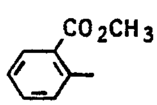 | 0 | H | H | $CH_3$ | 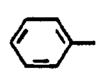 | 220-228 |

Tabelle 1a - Forts.

| Nr. | A | n | $R^1$ | $R^2$ | $R^4$ | $R^{4'}$ | Fp [°C] |
| --- | --- | --- | --- | --- | --- | --- | --- |
| 1.104 | 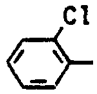 | 0 | H | H | $CH_3$ | 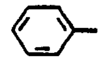 | 276-277 |
| 1.105 | 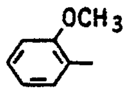 | 0 | H | H | $CH_3$ | 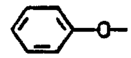 | 272-273 |
| 1.106 | 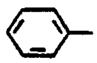 | 0 | H | H | $CH_3$ | 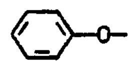 | 71-79 |
| 1.107 | 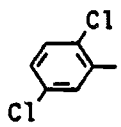 | 0 | H | H | $CH_3$ | 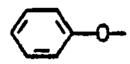 | 149-155 |
| 1.108 | 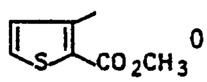 | 0 | H | H | $CH_3$ | 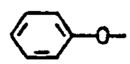 | 179-181 |
| 1.109 | 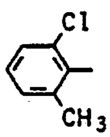 | 0 | H | H | $CH_3$ | 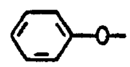 | 233-236 |
| 1.110 | 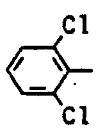 | 0 | H | H | $CH_3$ | 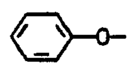 | 259-263 |
| 1.111 | 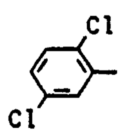 | 0 | H | H | H | $CH_3$ | 182-190 |
| 1.112 | 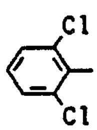 | 0 | H | H | H | $CH_3$ | 229-233 |
| 1.113 | 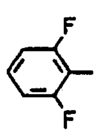 | 0 | H | H | Cl | Cl | 193-203 |
| 1.114 | 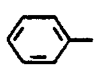 | 0 | H | H | $OCH_3$ | $OCH_3$ | 240-242 |
| 1.115 | 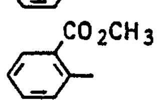 | 0 | H | H | H | $CH_3$ | 194-196 |
| 1.116 | 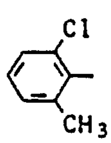 | 0 | 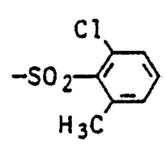 | H | H | $CH_3$ | 218-225 |

Tabelle 1a - Forts.

| Nr. | A | n | $R^1$ | $R^2$ | $R^4$ | $R^{4'}$ | Fp [°C] |
|---|---|---|---|---|---|---|---|
| 1.117 | 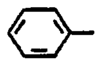 | 0 | $-SO_2-$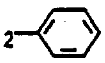 | H | H | $CH_3$ | 199-201 |
| 1.118 | 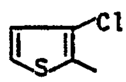 | 0 | H | H | $CH_3$ | $CH_3$ | 185-190 |
| 1.119 | 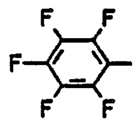 | 0 | H | H | $CH_3$ | $CH_3$ | 195 (Zers) |
| 1.120 | 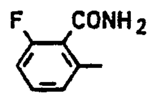 | 0 | H | H | $CH_3$ | $CH_3$ | 275 (Zers) |
| 1.121 | 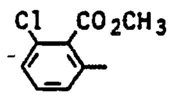 | 0 | H | H | 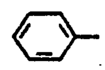 | $CH_3$ | 260-263 |
| 1.122 | 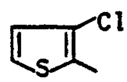 | 0 | H | H | $CH_3$ | Cl | 280-285 |
| 1.123 | 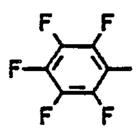 | 0 | H | H | $CH_3$ | Cl | 151-157 |
| 1.124 | 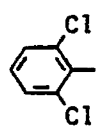 | 0 | $CH_3$ | H | $CH_3$ | Cl | 171-180 |
| 1.125 | 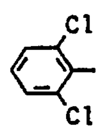 | 0 | H | CN | H | H | 214-218 |
| 1.126 | 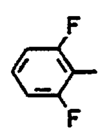 | 0 | H | CN | H | H | 220-224 |
| 1.127 | 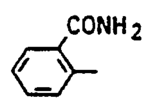 | 0 | H | H | $CH_3$ | 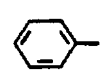 | 145 (Zers) |
| 1.128 | 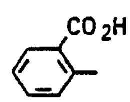 | 0 | H | H | $CH_3$ | 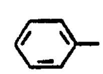 | 138 (Zers) |
| 1.129 | 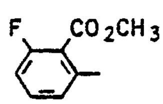 | 0 | K | H | $CH_3$ | 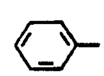 | 260-267 |

Tabelle 1a - Forts.

| Nr. | A | n | $R^1$ | $R^2$ | $R^4$ | $R^{4'}$ | Fp [°C] |
|---|---|---|---|---|---|---|---|
| 1.130 | 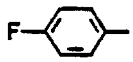 | 0 | 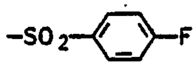 | H | $CH_3$ | $CH_3$ | 263-264 |
| 1.131 | 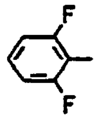 | 0 | 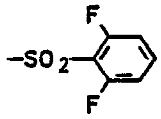 | H | $CH_3$ | $OCH_3$ | 203-206 |
| 1.132 | 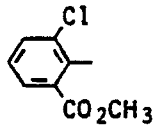 | 0 | 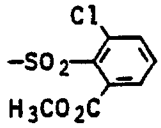 | H | $CH_3$ | $SCH_3$ | 248-250 |
| 1.133 | 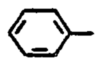 | 0 | 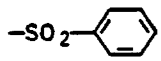 | H | $CH_3$ | $SCH_3$ | 243-250 |
| 1.134 | 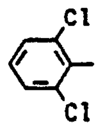 | 0 | 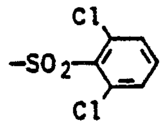 | H | OH | OH | 211-213 |
| 1.135 | 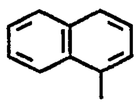 | 0 | H | H | 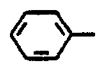 | $CH_3$ | > 280 |
| 1.136 | 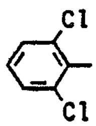 | 0 | H | H | $CH_3$ | H | 170-175 |
| 1.137 | 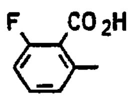 | 0 | H | H | $CH_3$ | 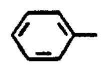 | 208-210 |
| 1.138 | 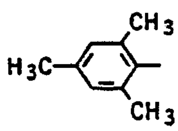 | 0 | H | H | $CH_3$ | Cl | 233-236 |
| 1.139 | 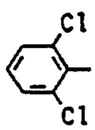 | 0 | 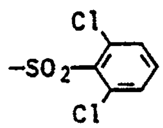 | H | H | Cl | 244-249 |
| 1.140 | 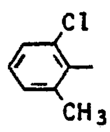 | 0 | $CH_3$ | H | $OCH_3$ | $OCH_3$ | 222 |
| 1.141 | 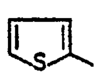 | 0 | 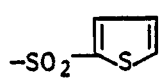 | H | $CH_3$ | $OCH_3$ | 222-230 |

Tabelle 1a - Forts.

| Nr. | A | n | $R^1$ | $R^2$ | $R^4$ | $R^{4'}$ | Fp [°C] |
|---|---|---|---|---|---|---|---|
| 1.142 | 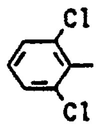 | 0 | H | H | H | OH | > 280 |
| 1.143 | 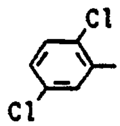 | 0 | H | H | $OCH_3$ | $OCH_3$ | 265-268 |
| 1.144 | 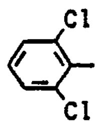 | 0 | H | H | $CH_3$ | Br | 245-250 |
| 1.145 | 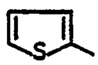 | 0 | $-SO_2-$ 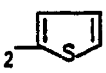 | H | $CH_3$ | Cl | 215-217 |
| 1.146 | 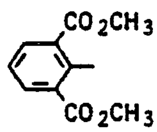 | 0 | H | H | $CH_3$ | $CH_3$ | 182 |
| 1.147 | 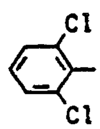 | 0 | H | H | $CH_3$ | $NH-NH_2$ | > 300 |

Tabelle 2

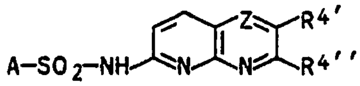

| Nr. | A | Z | R4′ | R4′′ | Fp [°C] |
|---|---|---|---|---|---|
| 2.001 | 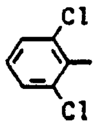 | 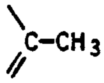 | $CH_3$ | $CH_3$ | 210-211 |
| 2.002 | 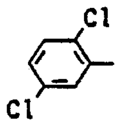 | 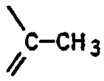 | $CH_3$ | $CH_3$ | 202-204 |
| 2.003 | 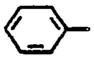 | 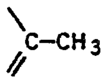 | $CH_3$ | $CH_3$ | 188-190 |
| 2.004 | 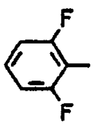 | 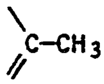 | $CH_3$ | $CH_3$ | 181-182 |
| 2.005 | 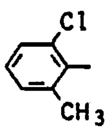 | 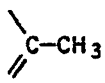 | $CH_3$ | $CH_3$ | 204-205 |
| 2.006 | 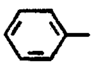 | 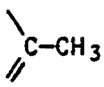 | $CH_3$ | Cl | 198-199 |
| 2.007 | 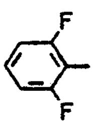 | 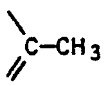 | $CH_3$ | Cl | 215-217 |
| 2.008 | 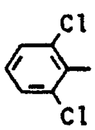 | 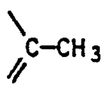 | $CH_3$ | Cl | 199-201 |
| 2.009 | 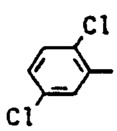 | 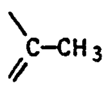 | $CH_3$ | Cl | 222-224 |
| 2.010 | 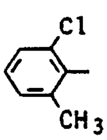 | 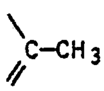 | $CH_3$ | Cl | 218-220 |
| 2.011 | 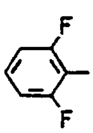 | 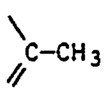 | $CH(CH_3)_2$ | Cl | 205-208 |

Tabelle 2 - Forts.

| Nr. | A | Z | R4' | R4'' | Fp [°C] |
|---|---|---|---|---|---|
| 2.012 | 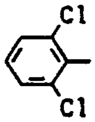 | 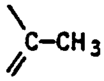 | $CH(CH_3)_2$ | Cl | 198-201 |
| 2.013 | 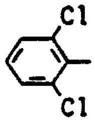 | 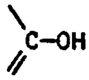 | $CH_3$ | OH | > 250 |
| 2.014 | 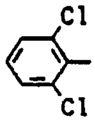 | 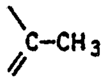 | Br | $CH_3$ | 232-235 |
| 2.015 | 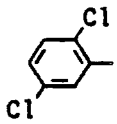 | 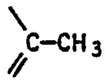 | Br | $CH_3$ | 221-223 |
| 2.016 | 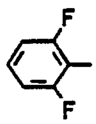 | 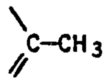 | Br | $CH_3$ | 188-190 |
| 2.017 | 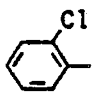 | 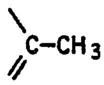 | Br | $CH_3$ | 243 |
| 2.018 | 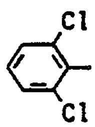 | 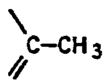 | Cl | $CH_3$ | 121-122 |
| 2.019 | 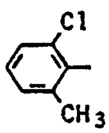 | 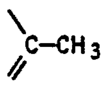 | Cl | $CH_3$ | 111-113 |
| 2.020 | 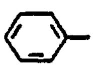 | 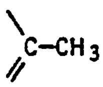 | Cl | $CH_3$ | 220-222 |
| 2.021 | 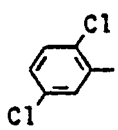 | 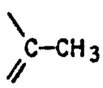 | Cl | $CH_3$ | 238-240 |
| 2.022 | 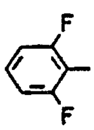 | 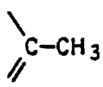 | Cl | $CH_3$ | 196-199 |

Tabelle 2 - Forts.

| Nr. | A | Z | $R^{4'}$ | $R^{4''}$ | Fp [°C] |
|---|---|---|---|---|---|
| 2.023 | 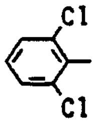 | 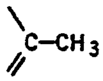 | $CH_3$ | $OCH_3$ | 235-236 |
| 2.024 | 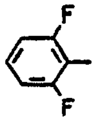 | 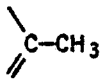 | $CH_3$ | $OCH_3$ | 224-226 |
| 2.025 | 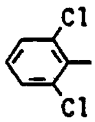 | 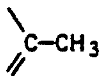 | $CH_3$ | 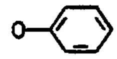 | 240 |
| 2.026 | 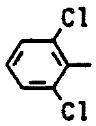 | 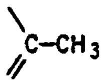 | $CH_3$ | 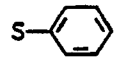 | 219-220 |
| 2.027 | 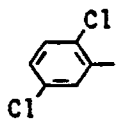 | 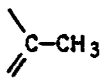 | $CH_3$ | 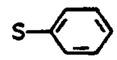 | 224-225 |
| 2.028 | 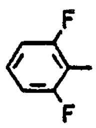 | 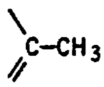 | $CH_3$ | 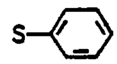 | 213-215 |
| 2.029 | 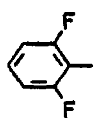 | 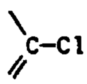 | $CH_3$ | Cl | 190 |
| 2.030 | 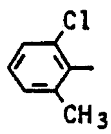 | 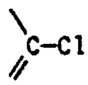 | $CH_3$ | Cl | 210 |
| 2.031 | 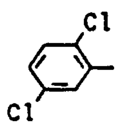 | 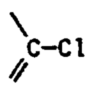 | n-$C_4H_9$ | Cl | 166-168 |
| 2.032 | 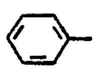 | 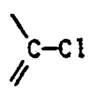 | n-$C_4H_9$ | Cl | 70-72 |
| 2.033 | 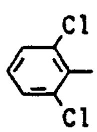 | 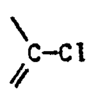 | n-$C_4H_9$ | Cl | 207-209 |

Tabelle 2 - Forts.

| Nr. | A | Z | R4′ | R4′′ | Fp [°C] |
|---|---|---|---|---|---|
| 2.034 | 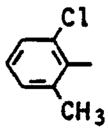 | 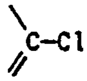 | $n-C_4H_9$ | Cl | 185-186 |
| 2.035 | 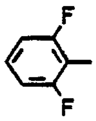 | 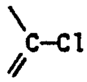 | $n-C_4H_9$ | Cl | 132-134 |
| 2.036 | 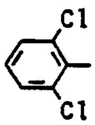 | 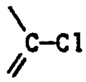 | $CH(CH_3)_2$ | Cl | 207-209 |
| 2.037 | 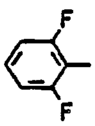 | 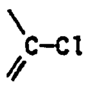 | $CH(CH_3)_2$ | Cl | > 70 (Zers.) |
| 2.038 | 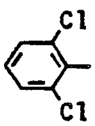 | 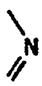 | $CH_3$ | $CH_3$ | > 260 |

Tabelle 3

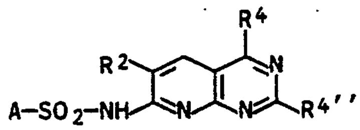

| Nr. | A | $R^2$ | $R^4$ | $R^{4''}$ | Fp [°C] |
|---|---|---|---|---|---|
| 3.001 | 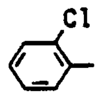 | CN | $CH_3$ | $CH_3$ | > 230 |
| 3.002 | 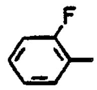 | CN | $CH_3$ | $CH_3$ | 210-212 |
| 3.003 | 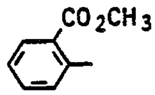 | CN | $CH_3$ | $CH_3$ | 220-222 |
| 3.004 | 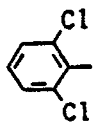 | CN | $CH_3$ | $CH_3$ | > 230 |
| 3.005 | 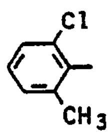 | CN | $CH_3$ | $CH_3$ | 233-235 |
| 3.006 | 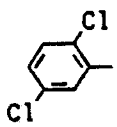 | CN | $CH_3$ | $CH_3$ | > 230 |
| 3.007 | 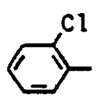 | $SO_2CH_3$ | $CH_3$ | $CH_3$ | > 230 |
| 3.008 | 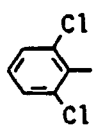 | $SO_2CH_3$ | $CH_3$ | $CH_3$ | > 230 |
| 3.009 | 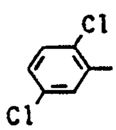 | $SO_2CH_3$ | $CH_3$ | $CH_3$ | > 230 |
| 3.010 | 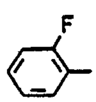 | $SO_2CH_3$ | $CH_3$ | $CH_3$ | 107-109 |
| 3.011 | 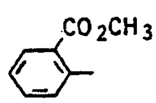 | $SO_2CH_3$ | $CH_3$ | $CH_3$ | 220-222 |

Tabelle 3 - Forts.

| Nr. | A | $R^2$ | $R^4$ | $R^{4''}$ | Fp [°C] |
|---|---|---|---|---|---|
| 3.012 | 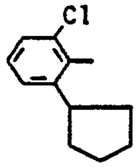 | $SO_2CH_3$ | $CH_3$ | $CH_3$ | > 215 |
| 3.013 | 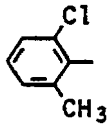 | H | $CH_3$ | $CH_3$ | > 215 |
| 3.014 | 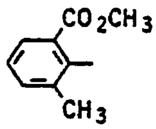 | H | $CH_3$ | $CH_3$ | 171-173 |
| 3.015 | 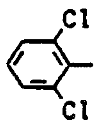 | H | $CH_3$ | $CH_3$ | > 230 |
| 3.016 | 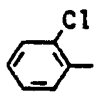 | CN | $OCH_3$ | $OCH_3$ | 199-201 |
| 3.017 | 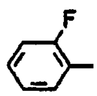 | CN | $OCH_3$ | $OCH_3$ | 153-156 |
| 3.018 | 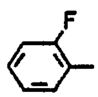 | 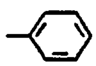 | $OCH_2CH_3$ | $OCH_2CH_3$ | 198 |
| 3.019 | 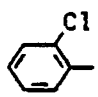 | 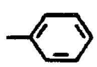 | $OCH_2CH_3$ | $OCH_2CH_3$ | 169-173 |
| 3.020 | 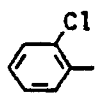 | 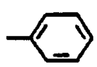 | $OCH_3$ | $OCH_3$ | 226-228 |
| 3.021 | 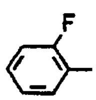 | 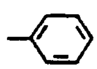 | $OCH_3$ | $OCH_3$ | 198-199 |
| 3.022 | 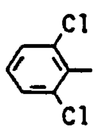 | 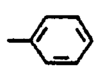 | $OCH_3$ | $OCH_3$ | > 230 |
| 3.023 | 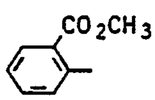 | 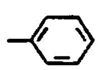 | $OCH_3$ | $OCH_3$ | 108-111 |

Tabelle 3 - Forts.

| Nr. | A | $R^2$ | $R^4$ | $R^{4''}$ | Fp [°C] |
|---|---|---|---|---|---|
| 3.024 | 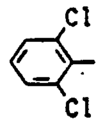 | CN | $OCH_3$ | $OCH_3$ | 218-220 |
| 3.025 | 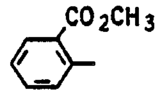 | CN | $OCH_3$ | $OCH_3$ | 211-213 |
| 3.026 | 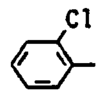 | $SO_2CH_3$ | $OCH_3$ | $OCH_3$ | > 215 |
| 3.027 | 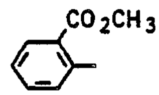 | $SO_2CH_3$ | $OCH_3$ | $OCH_3$ | > 230 |
| 3.028 | 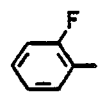 | $SO_2CH_3$ | $OCH_3$ | $OCH_3$ | 155-157 |
| 3.029 | 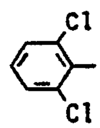 | $SO_2CH_3$ | $OCH_3$ | $OCH_3$ | 213-214 |
| 3.030 | 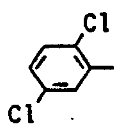 | $SO_2CH_3$ | $OCH_3$ | $OCH_3$ | 172-175 |
| 3.031 | 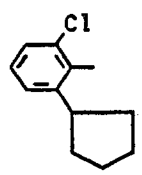 | $SO_2CH_3$ | $OCH_3$ | $OCH_3$ | 228-229 |
| 3.032 | 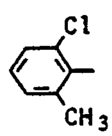 | $SO_2CH_3$ | $OCH_3$ | $OCH_3$ | 226-227 |
| 3.033 | 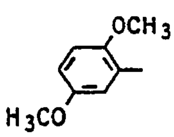 | $SO_2CH_3$ | $OCH_3$ | $OCH_3$ | 128 |
| 3.034 | 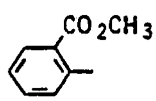 | CN | $CH_3$ | $OCH_3$ | 200 |

Tabelle 3 - Forts.

| Nr. | A | $R^2$ | $R^4$ | $R^{4''}$ | Fp [°C] |
|---|---|---|---|---|---|
| 3.035 | 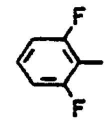 | CN | $CH_3$ | $OCH_3$ | 90-95 |
| 3.036 | 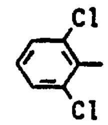 | CN | $CH_3$ | $OCH_3$ | 225-227 |
| 3.037 | 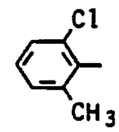 | CN | $CH_3$ | $OCH_3$ | 213-215 |
| 3.038 | 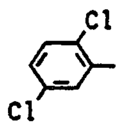 | CN | $CH_3$ | $OCH_3$ | 224-227 |
| 3.039 | 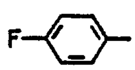 | CN | $CH_3$ | $OCH_3$ | 205-208 |
| 3.040 | 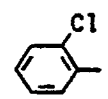 | CN | $CH_3$ | $OCH_3$ | 209 |

Anwendungsbeispiele

Die herbizide Wirkung der Sulfonamide der Formel I auf das Wachstum der Testpflanzen wird durch folgende Gewächshausversuche gezeigt.

Zur Anzucht der Testpflanzen dienten Plastikblumentöpfe mit 300 $cm^3$ Inhalt und lehmigem Sand mit etwa 3,0 % Humus als Substrat. Die Samen der Testpflanzen wurden nach Arten getrennt eingesät.

Zum Zweck der Nachauflaufbehandlung wurden entweder direkt gesäte oder in den gleichen Gefäßen aufgewachsene Pflanzen ausgewählt oder sie wurden erst als Keimpflanzen getrennt angezogen und einige Tage vor der Behandlung in die Versuchsgefäße verpflanzt.

Je nach Wuchsform wurden die Testpflanzen bei einer Wuchshöhe von 3 - 15 cm dann mit den in Wasser als Verteilungsmittel suspendierten oder emulgierten Wirkstoffen, die durch fein verteilende Düsen gespritzt wurden, behandelt. Die Aufwandmenge für die Nachauflaufbehandlung betrug 2,0 bzw. 3,0 kg/ha aktive Substanz.

Die Versuchsgefäße wurden im Gewächshaus aufgestellt, wobei für wärmeliebende Arten wärmere Bereiche (20 bis 35° C) und für solche gemäßigter Klimate 10 bis 20° C bevorzugt wurden. Die Versuchsperiode erstreckte sich über 2 bis 4 Wochen. Während dieser Zeit wurden die Pflanzen gepflegt und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

In den Gewächshausversuchen wurden Pflanzen der folgenden Arten verwendet:

| Pflanzenliste | |
|---|---|
| Lateinischer Name | Deutscher Name |
| Amaranthus retroflexus | Zurückgekrümmter Fuchsschwanz |
| Centaurea cyanus | Kornblume |
| Chenopodium album | Weißer Gänsefuß |
| Cyperus iria | - |
| Ipomoea spp. | Prunkwindearten |

Mit 2,0 bzw. 3,0 kg/ha a.S. im Nachauflaufverfahren eingesetzt, ließen sich mit den Beispielen 1.066 und 1.043 unerwünschte Pflanzen sehr gut bekämpfen.

## Ansprüche

1. Sulfonamide der allgemeinen Formel I,

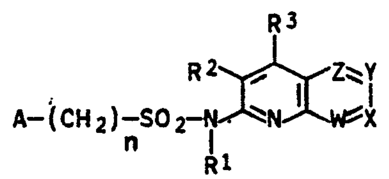

I

in der die Substituenten und Indices folgende Bedeutung haben:
$R^1$ Wasserstoff, Cyano,
eine $C_1$-$C_8$-Alkylgruppe, welche durch einen der folgenden Reste substituiert sein kann: $C_1$-$C_2$-Alkoxy, $C_1$-$C_2$-Alkylthio, Aryl, Aryloxy, Arylthio, Heteroaryl, Heteroaryloxy oder Heteroarylthio,
eine $C_2$-$C_5$-Alkenylgruppe,
eine $C_2$-$C_4$-Alkinylgruppe,
ein Rest $COR^4$, worin $R^4$ $C_1$-$C_4$-Alkyl, $C_1$-$C_4$-Alkoxy, $C_1$-$C_4$-Alkylthio, Aryl, Aryloxy, Arylthio, Aryl-$C_1$-$C_4$-alkoxy, Heteroaryl, Heteroaryloxy, Heteroarylthio oder Heteroaryl-$C_1$-$C_4$-alkoxy bedeutet,
ein Rest $CONR^5R^6$, worin $R^5$ und $R^6$ unabhängig voneiander folgende Bedeutung haben: Wasserstoff, $C_1$-$C_4$-Alkyl, $C_3$-$C_6$-Cycloalkyl, $C_2$-$C_5$-Alkenyl, Aryl, Heteroaryl, Aryl-$C_1$-$C_4$-alkyl, Heteroaryl-$C_1$-$C_4$-alkyl, $C_1$-$C_4$-Alkylcarbonyl oder gemeinsam eine $C_2$-$C_6$-Alkylenkette,
oder ein Rest $SO_mR^4$, worin m den Wert 1 oder 2 hat und $R^4$ die vorstehend gegebene Bedeutung besitzt;
$R^2$, $R^3$ unabhängig voneinander Nitro, Hydroxy, Carboxy, Mercapto, Halogen,
$C_1$-$C_4$-Alkyl, welches einfach durch Hydroxy, Mercapto, Amino, Aryloxy oder Heteroaryloxy und/oder ein- bis dreifach durch Halogen substituiert sein kann,
$C_3$-$C_6$-Cycloalkyl, welches ein- bis dreifach durch Halogen, Hydroxy, Mercapto und/oder $C_1$-$C_4$-Alkyl substituiert sein kann,
$C_3$-$C_6$-Cycloalkoxy,
$C_3$-$C_6$-Cycloalkylthio,
$C_2$-$C_5$-Halogenalkenyl,
$C_2$-$C_4$-Halogenalkinyl,
$C_1$-$C_4$-Alkoxy, welches ein- bis dreifach durch Halogen und/oder einfach durch Aryl oder Heteroaryl substituiert sein kann,
$C_1$-$C_4$-Alkylthio, welches ein- bis dreifach durch Halogen und/oder einfach durch Aryl oder Heteroaryl substituiert sein kann,
$C_2$-$C_5$-Alkenyloxy,
$C_2$-$C_4$-Alkinyloxy,
ein Rest $-NR^5R^6$ worin $R^5$ und $R^6$ die vorstehend gegebene Bedeutung besitzen oder
eine der unter $R^1$ genannten Gruppen,
W,X,Y,Z unabhängig voneinander Stickstoff oder
eine Gruppe

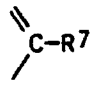

worin $R^7$ Hydrazino oder eine der unter $R^2$ genannten Gruppen bedeutet, wobei X, Y, Z und W nicht gleichzeitig Stickstoff bedeuten,
n den Wert 0 oder 1 und
A eine Aryl- oder Heteroarylgruppe, wobei diese Gruppen ein bis fünf Halogenatome und/oder ein bis drei der folgenden Substituenten tragen können:
$-SO_2R^8$, worin $R^8$ Hydroxy, $C_1$-$C_4$-Alkoxy, Aryl-$C_1$-$C_4$-alkoxy, Aryloxy, Heteroaryloxy, Heteroaryl-$C_1$-$C_4$-alkoxy oder $-NR^5R^6$ bedeutet, wobei $R^5$ und $R^6$ die vorstehend gegebene Bedeutung besitzen und/oder die unter $R^2$ genannten Gruppen
sowie deren Salze und N-Oxide, ausgenommen die Verbindungen der Formel I, in der A die Bedeutung 4-Aminophenyl hat, n = 0 ist, $R^1$, $R^2$ und $R^3$ die Bedeutung Wasserstoff, W die Bedeutung Stickstoff, X, Y und Z die Bedeutung Kohlenstoff hat und $R^4$ an X und Z Methyl und im übrigen Wasserstoff ist, sowie deren 4-Aminophenyl-Umsetzungsprodukte.

2. Sulfonamide der allgemeinen Formel Ia,

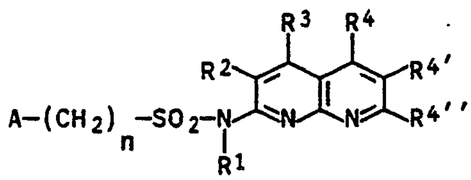
Ia

in der die Substituenten und Indices folgende Bedeutung haben:
$R^1$ Wasserstoff, eine $C_1$-$C_4$ Alkylcarbonylgruppe, welche im Alkylteil ein bis drei der folgenden Reste tragen kann: Halogen, $C_1$-$C_4$-Alkoxy und/oder $C_1$-$C_4$-Alkylthio,
oder eine $C_1$-$C_4$-Alkylgruppe, welche ein bis drei der folgenden Reste tragen kann: Halogen, $C_1$-$C_4$-Alkoxy und/oder $C_1$-$C_4$-Alkylthio,
$R^2$ Wasserstoff, eine Cyanogruppe oder ein Halogenatom,
$R^3$ Wasserstoff, eine $C_1$-$C_4$-Alkylgruppe oder ein Halogenatom,
$R^4$, $R^{4'}$ und $R^{4''}$ unabhängig voneinander Wasserstoff, Methyl, Ethyl, Trifluormethyl, Difluormethyl, Methoxy, Ethoxy, Dimethylamino, Diethylamino, Methylthio, Ethylthio, Phenyl, Phenoxy, Hydrazino, oder Halogenatom,
n 0 oder 1 und
A ein Phenylrest, ein Naphthylrest, ein Thienylrest oder ein Chinolinylrest, wobei diese aromatischen Reste ein bis drei der folgenden Gruppen tragen können: $C_1$-$C_8$-Alkyl, $C_1$-$C_4$-Alkoxy, $C_1$-$C_2$-Alkoxy-$C_1$-$C_4$-alkyl, $C_1$-$C_4$-Alkoxycarbonyl und/oder bis zu fünf Halogenatome
sowie deren Salze und N-Oxide.

3. Sulfonamide der allgemeinen Formel Ib,

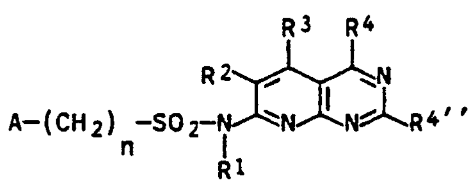
Ib

in der die Substituenten und Indices folgende Bedeutung haben:
$R^1$ Wasserstoff, eine $C_1$-$C_4$-Alkylcarbonylgruppe, welche im Alkylteil ein bis drei der folgenden Reste tragen kann: Halogen, $C_1$-$C_4$-Alkoxy und/oder $C_1$-$C_4$-Alkylthio
oder eine $C_1$-$C_4$-Alkylgruppe, welche ein bis drei der folgenden Reste tragen kann: Halogen, $C_1$-$C_4$-Alkoxy und/oder $C_1$-$C_4$-Alkylthio,
$R^2$ Wasserstoff, eine Cyanogruppe, ein Halogenatom, eine $C_1$-$C_4$-Alkoxycarbonylgruppe, eine $C_1$-$C_4$-Alkylsulfonylgruppe oder ein Phenylrest,
$R^3$ Wasserstoff, eine $C_1$-$C_4$-Alkylgruppe oder ein Halogenatom,

$R^4$, $R^{4'}$ und $R^{4''}$ unabhängig voneinander Wasserstoff, Methyl, Ethyl, Trilfuormethyl, Difluormethyl, Methoxy, Ethoxy, Dimethylamino, Diethylamino, Methylthio, Ethylthio, Phenyl, Phenoxy, Hydrazino oder Halogenatom,
n 0 oder 1 und
A ein Phenylrest, ein Naphthylrest, ein Thienylrest oder ein Chinolinylrest, wobei diese aromatischen Reste ein bis drei der folgenden Gruppen tragen können: Halogen, $C_1$-$C_8$-Alkyl, $C_1$-$C_4$-Alkoxy, $C_1$-$C_2$-Alkoxy-$C_1$-$C_4$-alkyl oder $C_1$-$C_4$-Alkoxycarbonyl
sowie deren Salze und N-Oxide.

4. Sulfonamide der allgemeinen Formel Ic,

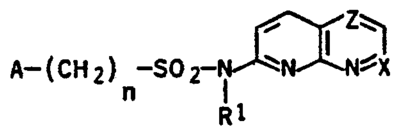

Ic

in der
A, X, Z, n und $R^1$ die in Anspruch 1 angegebene Bedeutung haben sowie deren Salze und N-Oxide.

5. Sulfonamide der allgemeinen Formel Id,

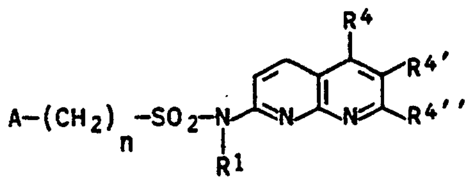

Id

in der A, n, $R^1$ und $R^4$, $R^{4'}$ und $R^{4''}$ die in Anspruch 1 angegebene Bedeutung haben, sowie deren Salze und N-Oxide.

6. Verfahren zur Herstellung von Sulfonamiden der allgemeinen Formel I, gemäß Anspruch 1, dadurch gekennzeichnet, daß man ein entsprechendes heterocyclisches Amin der allgemeinen Formel II,

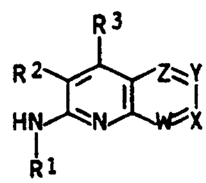

II

mit einem entsprechenden Sulfochlorid der allgemeinen Formel III,
$A\text{-}(\text{-}CH_2)_n\text{-}SO_2Cl$ III
in Gegenwart einer Base umsetzt.

7. Verfahren zur Herstellung von Sulfonamiden der allgemeinen Formel I, gemäß Anspruch 1, in der $R^1$ nicht Wasserstoff bedeutet, dadurch gekennzeichnet, daß man ein Sulfonamid der allgemeinen Formel I, gemäß Anspruch 1, mit der Bedeutung von $R^1$ = H in Gegenwart einer Base mit einem elektrophilen Reagens der allgemeinen Formel IV,
$R^1$-B IV
in der $R^1$ die unter Anspruch 1 angegebene Bedeutung mit Ausnahme von Wasserstoff hat, und B eine geeignete Abgangsgruppe wie z.B. Halogen, insbesondere Cl, ist, umsetzt.

8. Herbizides Mittel, enthaltend ein Sulfonamid der allgemeinen Formel I, gemäß Anspruch 1, dessen Salz oder N-Oxid sowie übliche Formulierungshilfsmittel.

9. Verfahren zur Bekämpfung unerwünschten Pflanzenwuchses, dadurch gekennzeichnet, daß man ein Sulfonamid der Formel I gemäß Anspruch 1, dessen Salz oder N-Oxid auf die Pflanzen und/oder ihren Lebensraum einwirken läßt.

10. Mittel zur Beeinflussung des Pflanzenwuchses, enthaltend ein Sulfonamid der Formel I gemäß Anspruch 1, dessen Salz oder N-Oxid sowie übliche Formulierungshilfsmittel.

11. Verfahren zur Beeinflussung des Pflanzenwachstums, dadurch gekennzeichnet, daß man ein Sulfonamid der Formel I gemäß Anspruch 1, dessen Salz oder N-Oxid auf die Pflanzen und/oder ihren Lebensraum einwirken läßt.